# EUROPEAN PATENT APPLICATION

(11) **EP 4 488 259 A1**
(43) Date of publication of application: **08.01.2025**
(21) Application number: 23778531.6
(22) Date of filing: 03.04.2023
(51) Int. Cl.: C07C 219/16, C07C 229/12, C07C 219/06, A61K 9/127, A61K 47/18, A61P 31/20

(54) **CATIONIC LIPID, LIPOSOME, LIPID NANOPARTICLE, AND USE**

(30) Priority: 02.04.2022 CN 202210349570
(71) Applicant: Carcell Biopharma Ltd., Shanghai 200131 (CN)
(72) Inventor: ZHANG, Yi, Shanghai 200010 (CN)
(74) Representative: Cabinet Becker et Associés
(86) International application number: PCT/CN2023/085894
(87) International publication number: WO 2023/186167

(57) **Abstract**

Disclosed is a cationic lipid having the following general formula (I), wherein A, B, C, and D are each as defined in the description. Also disclosed are a liposome and a lipid nanoparticle containing the cationic lipid, and use of the cationic lipid in the preparation of the liposome and the lipid nanoparticle.

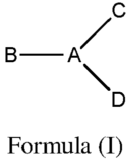

## Description

This application claims the priority of Chinese Patent Application No. 202210349570.6 filed on April 2, 2022, the disclosure of which is hereby incorporated by reference in its entirety as a part of the present application.

### INVENTION FIELD

The present disclosure generally relates to the field of gene therapy drug preparations, and more specifically to a cationic lipid for forming liposomes or lipid nanoparticles. The present disclosure also relates to liposomes containing the cationic lipid and uses of the cationic lipid.

### BACKGROUND

In recent years, with the continuously deepening understanding of the pathogenesis of diseases at the cellular level, gene therapy has gradually become a research hotspot issue. Various genetic engineering drugs have been continuously developed and successively commercialized.

In the gene therapy, gene transfer vectors are the key to an exogeneous gene entering recipient cells. Among them, cationic liposomes and lipid nanoparticles (LNPs) are gaining increasing attention due to their good targeting, few side effects, good stability, and high transfection efficiency. Cationic liposomes and LNPs are usually compounded by a cationic lipid, a neutral auxiliary lipid, and other materials under appropriate conditions, and their transfection efficiency is related to the structure of the cationic lipid used.

### SUMMARY

At least an embodiment of the present disclosure provides a cationic lipid of Formula (I): 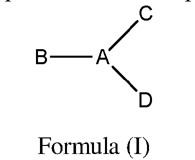
wherein A is selected from methine and nitrilo, wherein the methine is optionally unsubstituted or substituted with R^{a};
wherein: when A is methine, B is R¹-L¹-Y¹-L²-, wherein
   R¹ is R²R³N-, in which R² and R³ are each independently C₁₋₆ alkyl,
   or R¹ is a 5- or 6-membered heterocyclyl containing 1 or 2 ring nitrogen atoms,
   wherein the C₁₋₆ alkyl or the 5- or 6-membered heterocyclyl is each independently unsubstituted or substituted with 1-3 R^{b};
   L₁ and L² are each independently selected from a chemical bond and a linear or branched C₁₋₁₂ alkylene, wherein each occurrence of the C₁₋₁₂ alkylene is optionally unsubstituted or substituted with 1 to 3 R^{c}; and
   Y is selected from the group consisting of -C(=X)-X-, -X-C(=X)-, -C(=X)-NH-, -NH-C(=X)-, -X-C(=X)-NH-, -NH-C(=X)-X-, -X-C(=X)-X-, -NH-C(=X)-NH-, and -X-X-; and
when A is nitrilo, B is C₁₋₆ alkyl, wherein the C₁₋₆ alkyl is optionally unsubstituted or substituted with 1-3 R^{d};
C is -L³-Y²-L⁴-CH=CR⁴R⁵, wherein
   L³ and L⁴ are each independently selected from a chemical bond and a linear or branched C₁₋₁₂ alkylene, wherein each occurrence of the C₁₋₁₂ alkylene is optionally unsubstituted or substituted with 1 to 3 R^{e},
   Y² is selected from the group consisting of -C(=X)-X-, -X-C(=X)-, -C(=X)-NH-, -NH-C(=X)-, -X-C(=X)-NH-, -NH-C(=X)-X-, -X-C(=X)-X-, -NH-C(=X)-NH-, and -X-X-, and
   R⁴ and R⁵ are each independently selected from a linear or branched C₂₋₁₂ alkyl group, wherein the C₂₋₁₂ alkyl group is optionally unsubstituted or substituted with 1 to 3 R^{f};
D is -L⁵-Y³-L⁶-R⁶, wherein
   L⁵ and L⁶ are each independently selected from a chemical bond and a linear or branched C₁₋₁₂ alkylene, wherein each occurrence of the C₁₋₁₂ alkylene is optionally unsubstituted or substituted with 1 to 3 R^{g},
   Y³ is selected from the group consisting of -C(=X)-X-, -X-C(=X)-, -C(=X)-NH-, -NH-C(=X)-, -X-C(=X)-NH-, -NH-C(=X)-X-, -X-C(=X)-X-, -NH-C(=X)-NH-, and -X-X-, and
   R⁶ is independently selected from a saturated or unsaturated, linear or branched C₆₋₁₈ hydrocarbon group or a steroid group, wherein the C₆₋₁₈ hydrocarbon group or the steroid group is optionally unsubstituted or substituted with 1-3 R^{h};
or, D is -L³-Y²-L⁴-CH=CR⁴R⁵;
each occurrence of X is independently O or S, and
each occurrence of R^{a}, R^{b}, R^{c}, R^{d}, R^{e}, R^{f}, R^{g} and R^{h} are each independently selected from the group consisting of C₁₋₃ alkyl, C₁₋₃ alkoxy, C₁₋₃ alkylamino, di(C₁₋₃ alkyl)amino, C₁₋₃ alkoxycarbonyl, C₁₋₃ alkylcarbonyloxy, C₁₋₃ alkylcarbonylamino, C₁₋₃ alkylaminocarbonyl, hydroxy, halogen, and cyano.

In an aspect, in the cationic lipid represented by Formula (I), X is O.

In another aspect, in the cationic lipid represented by Formula (I), B is R¹-L¹-Y¹-L²-, R¹ is R²R³N-, and R² and R³ are each independently C₁₋₆ alkyl, wherein the C₁₋₆ alkyl is optionally unsubstituted or substituted with 1-3 substituents independently selected from the group consisting of C₁₋₃ alkyl, C₁₋₃ alkoxy, hydroxyl, and halogen.

In another aspect, in the cationic lipid represented by Formula (I), R² and R³ are the same or different, and each independently selected from the group consisting of methyl, ethyl, *n*-propyl, isopropyl, n-butyl, isopropyl, and *tert*-butyl.

In another aspect, in the cationic lipid represented by Formula (I), B is R¹-L¹-Y¹-L²-, R¹ is a 5- or 6-membered heterocyclyl containing 1 or 2 ring nitrogen atoms, and the 5- or 6-membered heterocyclyl is a saturated 5- or 6-membered heterocyclyl unsubstituted or optionally substituted with 1-3 C₁₋₃ alkyl groups.

In another aspect, in the cationic lipid represented by Formula (I), the 5- or 6-membered heterocyclyl is selected from the group consisting of N-methyl-piperidin-4-yl, N-methyl-piperidin-2-yl, N-methyl-pyrrolidin-2-yl, N-ethyl-piperidin-4-yl, N-ethyl-piperidin-2-yl, N-ethyl-pyrrolidin-2-yl, and piperidine-1-yl.

In another aspect, in the cationic lipid represented by Formula (I), B is C₂₋₄ alkyl unsubstituted or substituted with 1 to 3 substituents selected from the group consisting of hydroxy, halogen, and cyano.

In another aspect, in the cationic lipid represented by Formula (I), B is selected from the group consisting of methyl, ethyl, *n*-propyl, isopropyl, *n*-butyl, isobutyl and *tert*-butyl, which are substituted with a primary hydroxyl group.

In another aspect, in the cationic lipid represented by Formula (I), C is -L³-Y²-L⁴-CH=CR⁴R⁵, wherein R⁴ and R⁵ are the same or different, and each independently selected from a linear C₂₋₁₂ alkyl.

In another aspect, in the cationic lipid represented by Formula (I), D is -L⁵-Y³-L⁶-R⁶, and R⁶ is a linear or branched C₆₋₁₈ hydrocarbon group containing one or two carbon-carbon double bonds.

In another aspect, in the cationic lipid represented by Formula (I), R⁶ is a linear or branched C₆₋₁₈ hydrocarbon group containing two carbon-carbon double bonds.

In another aspect, in the cationic lipid represented by Formula (I), D is -L⁵-Y³-L⁶-R⁶, and R⁶ is a C₆₋₁₈ alkyl substituted with a C₁₋₆ alkoxycarbonyl.

In another aspect, in the cationic lipid represented by Formula (I), D is -L³-Y²-L⁴-CH=CR⁴R⁵, wherein R⁴ and R⁵ are the same or different, and each independently selected from a linear C₂₋₁₂ alkyl.

In another aspect, in the cationic lipid represented by Formula (I), L¹, L², L³, L⁴, L⁵, and L⁶ are each independently selected from a chemical bond and a C₁₋₆ alkylene, and the C₁₋₆ alkylene is optionally unsubstituted or substituted with 1-3 substituents independently selected from the group consisting of C₁₋₃ alkyl, C₁₋₃ alkoxy, hydroxyl and halogen.

In another aspect, in the cationic lipid represented by Formula (I), L¹, L², L³, L⁴, L⁵, and L⁶ are each independently selected from a chemical bond and an unsubstituted C₁₋₆ alkylene.

In another aspect, in the cationic lipid represented by Formula (I), Y¹, Y², and Y³ are each independently selected from the group consisting of -O-C(=O)-, -C(=O)-O- and -O-C(=O)-O-.

In another aspect, the cationic lipid represented by Formula (I) has a structure selected from the group consisting of:

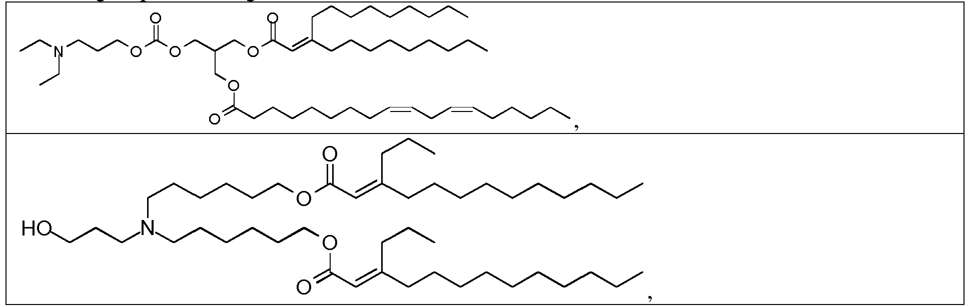

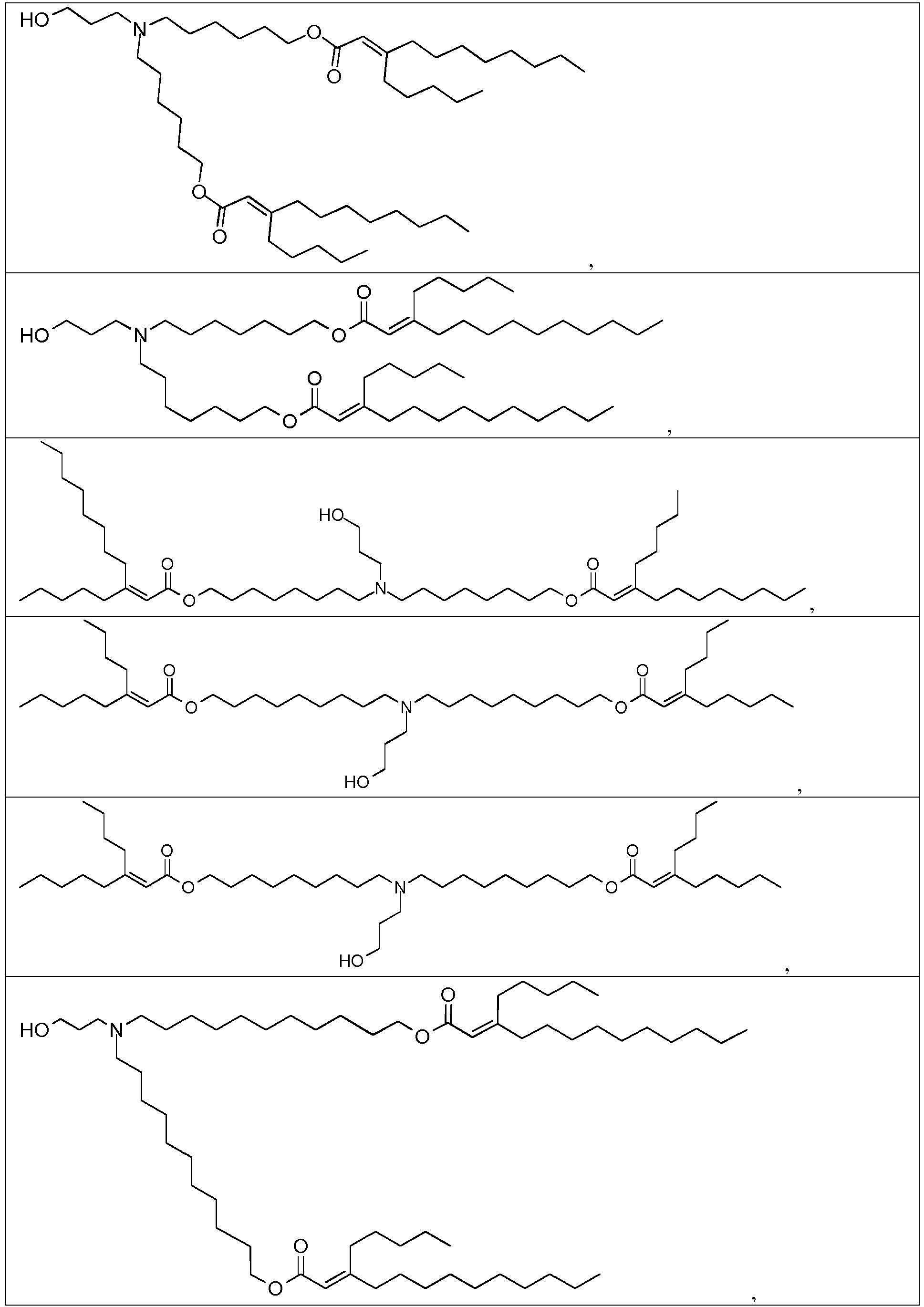

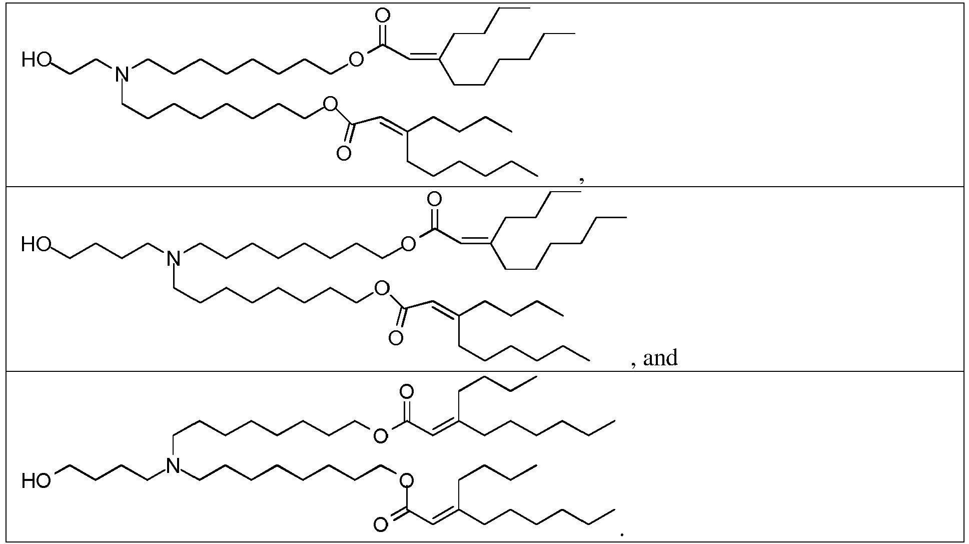

At least an embodiment of the present disclosure further discloses a liposome comprising the cationic lipid as described in any embodiment of the present disclosure.

At least an embodiment of the present disclosure further discloses a lipid nanoparticle comprising the cationic lipid as described in any embodiment of the present disclosure.

At least an embodiment of the present disclosure also discloses use of the cationic lipid as described in at least an embodiment of the present disclosure for preparing liposomes.

At least an embodiment of the present disclosure also discloses use of the cationic lipid as described in at least an embodiment of the present disclosure for preparing LNPs.

### DETAILED DESCRIPTION

For the purpose of the following detailed description, it should be understood that the present disclosure may adopt various alternative variations and step sequences, unless expressly specified to the contrary. In addition, except in any operating examples, or otherwise indicated, all numerals representing the amount of ingredients used in the description and claims should be understood to be modified by the term "about" in all cases. Therefore, the numerical parameters set forth in the following description and the appended claims are approximate values that vary according to the desired performance to be obtained by the present disclosure, unless otherwise indicated. At least, it is not intended to limit the application of the doctrine of equivalents to the scope of the claims, and each numerical parameter should at least be interpreted according to the number of reported significant figures and by applying ordinary rounding techniques.

Although the broad range of numerical values and parameters described in the present disclosure are approximate values, the numerical values set forth in the specific examples are reported as precisely as possible. Any numerical value, however, inherently contains certain errors necessarily resulting from the standard deviation found in their respective testing measurements.

In addition, it should be understood that any numerical ranges described herein are intended to include all subranges that fall within it. For example, a range of "1 to 10" is intended to include all subranges between (and including) the specified minimum value of 1 and the specified maximum value of 10, i.e., having a minimum value equal to or greater than 1 and a maximum value equal to or less than 10.

In the present disclosure, the use of the singular includes the plural and the plural includes the singular, unless otherwise expressly stated. In addition, in the present disclosure, the use of "or" means "and/or", even if "and/or" can be explicitly used in some cases, unless otherwise expressly stated. In addition, in the present disclosure, the use of "a" or "an" means "at least one", unless otherwise expressly stated. For example, "a" compound, "a" composition, etc. refer to any one or more of these items.

The compounds of the present disclosure can be synthesized by synthetic routes including methods similar to those known in the chemical field, particularly with reference to the descriptions contained herein. Starting materials are generally available from commercial sources, or can be easily prepared using methods known to those skilled in the art. For illustrative purposes, the reaction schemes depicted below show possible routes for synthesizing the disclosed compounds and key intermediates. To describe each reaction step in more detail, see the Examples section below. Those skilled in the art will appreciate that other suitable starting materials, reagents and synthetic routes can be used to synthesize the disclosed compounds and various derivatives thereof. In addition, with reference to the present disclosure, various compounds prepared by the following methods can be further modified using conventional chemical methods known to those skilled in the art.

The compounds of the present disclosure may include stereoisomers (e.g., *cis-* and *trans-*isomers), optical isomers (e.g., *R*- and *S*- enantiomers), tautomers (e.g., tautomers of enol and imine forms and tautomers of keto and enamine forms), atropisomers (i.e., isomers with restricted rotation) of the compounds of the present disclosure, and so on.

For purposes of the present invention as described and claimed herein, the following terms are defined as follows:

The term "cationic lipid" as used herein refers to a class of lipid materials charged under physiological condition, which can be used to prepare liposomes for the delivery of nucleic acid drugs. As used herein, the term "nucleic acid drug", also known as "nucleotide-based drug", refers to various ribonucleic acid (RNA) drugs and/or deoxyribonucleotide (DNA) drugs with different functions, which mainly play a role at the gene level. Examples of nucleic acid drugs can include, but is not limited to, RNA-based nucleic acid drugs, such as transfer RNA (tRNA), small interfering RNA (siRNA), Antisense Oligonucleotide, microRNA (miRNA), long non-coding RNA (lncRNA), nucleic acid aptamers (Apatmer), messenger RNA (mRNA), circular RNA (circRNA), and self-replicating RNA; and DNA-based nucleic acid drugs, such as plasmids, linearized plasmids, nanoplasmids, doggyboneDNA, closed-ended DNA, and oligomeric DNA (oligoDNA). At present, the nucleic acid drugs can be used as drugs for the treatment or prevention of diseases including but not limited to diseases such as tumors, autoimmune diseases, and infectious diseases, e.g., as tumor-targeted therapeutic agents, COVID-19 vaccines, etc.

The term "liposome" as used herein generally refers to a closed vesicle with a bilayer structure formed by dispersing lipids (lipidoids) and additives in an aqueous phase, which can usually fuse with cell membranes to deliver bioactive substances (e.g., nucleic acid drugs or other drugs) to the interior of cells. In general, the basic structure of cationic lipids is composed of a positively charged polar head and one or more hydrophobic tails. The positively charged polar head group generally includes an amine (primary amine, secondary amine, tertiary amine, or quaternary ammonium salt), a guanidine, an amidine group, etc., which can form a complex with a negatively charged nucleic acid through electrostatic interaction and enter cells through the endocytosis of cells. The hydrophobic tail can usually be a saturated or unsaturated fatty hydrocarbon chain or a cholesterol group, which can affect the pKa, lipophilicity, fluidity, and integration of cationic lipids, etc., thereby affecting the formation and effectiveness of cationic liposomes. A linker can also be included between the positively charged polar head group and the hydrophobic tail, which can connect the head to the tail, or can also be hidden in the tail. The linker can be divided into nonbiodegradable linkers (such as ethers, etc.) and biodegradable linkers (such as esters, amides, and thiols, etc.), which can affect the stability, biodegradability, cytotoxicity, and transfection efficiency of cationic liposomes. Cationic lipids can be regarded as multi-component molecules, of which each component needs to be precisely designed for safe and effective package and delivery of nucleic acids.

The term "lipid nanoparticle (Lipid Nano Particle, LNP)" as used herein refers to a class of nanocarriers for delivering nucleic acid substances to cells developed on the basis of liposomes, which is generally composed of a cationic lipid, a neutral auxiliary phospholipid, cholesterol, and a PEG lipid. LNPs are one of the important technologies in lipid carrier drug delivery systems and have become an important progress based on nucleic acid therapeutic drugs. Oligonucleotides encapsulated in LNPs are protected from enzymatic degradation during the delivery, and effectively delivered to cells, where the contents of the carrier particles are released and translated into therapeutic proteins.

Unless otherwise indicated, the term "hydrocarbon group" as used herein refers to a monovalent chain group consisting of carbon atoms and hydrogen atoms. The hydrocarbon group may be linear or branched, saturated or unsaturated, and may be substituted or unsubstituted. Non-limiting examples of hydrocarbon groups include, but are not limited to, alkyl, alkenyl, or alkynyl, or the like. For example, the term "C₂₋₁₈ hydrocarbon group" refers to a monovalent linear or branched alkyl, alkenyl, or alkynyl having 2-18 carbon atoms. Non-limiting examples of C₂₋₁₈ hydrocarbon groups may include, but are not limited to, methyl, ethyl, n-propyl, isopropyl, n-butyl, isobutyl, tert-butyl, n-pentyl, neopentyl, hexyl, heptyl, octyl, nonyl, decyl, lauryl, butenyl, hexenyl, octenyl, decenyl, myrcenyl, 1,3-butadienyl, 1,3-pentadienyl, octynyl, etc.

Unless otherwise indicated, the term "alkyl" as used herein refers to a saturated monovalent chain hydrocarbon group of the formula CₙH2ₙ₊₁-, wherein n is an integer between 1 and 18. Alkyl may be linear or branched, and may be substituted or unsubstituted. For example, the term "C₁₋₆ alkyl " refers to a monovalent linear or branched hydrocarbon group containing 1 to 6 carbon atoms. Non-limiting examples of C₁₋₆ alkyl groups include, but are not limited to, methyl, ethyl, propyl, isopropyl, *sec*-butyl, *tert*-butyl, *n*-propyl, *n*-butyl, isobutyl, *sec*-butyl, *n*-pentyl, 1-methylbutyl, 2-methylbutyl, 3-methylbutyl, neopentyl, 3,3-dimethylpropyl, 2-methylpentyl, hexyl, and the like. The alkyl moiety may be connected to another chemical moiety through any carbon atom on the carbon chain. As described herein, the alkyl group may be optionally substituted. In addition, when used in a compound word such as alkyloxy (alkoxy), the alkyl moiety has the same meaning as in the present disclosure, and may be connected to another chemical moiety through any carbon atom on the carbon chain.

Unless otherwise indicated, the term "alkenyl" as used herein refers to a linear or branched aliphatic hydrocarbon chain having 2 to 18 carbon atoms and containing at least one carbon-carbon double bond (e.g., -CH=CH-, -CH=CH₂). Non-limiting examples of alkenyl include ethenyl, 1-propenyl, 2-propenyl, isopropenyl, 1-butenyl, 2-butenyl, 3-butenyl, 2-pentenyl, hexenyl, octenyl, decenyl, myrcenyl, 1,3-butadienyl, 1,3-pentadienyl, and the like.

Unless otherwise indicated, the term "alkynyl" as used herein refers to a linear or branched aliphatic hydrocarbon chain having 2 to 18 carbon atoms and containing at least one carbon-carbon triple bond (e.g., -C=C- or -C=CH). Non-limiting examples of alkynyl include: ethynyl, 2-propynyl, 1-methyl-2-propynyl, 2-butynyl, 3-butynyl, 2-methyl-3-butynyl, and the like.

Unless otherwise indicated, the term "alkylene" as used herein refers to a saturated divalent chain hydrocarbon group of the formula -CₙH₂ₙ-, wherein n is an integer between 1 and 18. Alkylene may be linear or branched, and may be substituted or unsubstituted. For example, the term "C₁₋₁₂ alkylene" refers to a saturated divalent linear or branched hydrocarbon group containing 1 to 12 carbon atoms. Non-limiting examples of C₁₋₁₂ alkylene include, but are not limited to, methylene, ethylene, propylene, butylene, hexylene, octylene, decylene, 2-ethylhexyl, and the like. The alkylene moiety may be connected to another chemical moiety through any carbon atom on the carbon chain. As described herein, the alkylene may be optionally substituted.

Unless otherwise indicated, the term "methine" as used herein refers to a -CH< group, wherein the -CH< group may be optionally unsubstituted or substituted; and the term "nitrilo" refers to a -N< group.

Unless otherwise indicated, the term "halogen" as used herein refers to fluorine, chlorine, bromine or iodine.

Unless otherwise indicated, the term "hydroxy" as used herein refers to an -OH group.

Unless otherwise indicated, the term "alkoxy" as used herein refers to an alkyl-O- group, wherein alkyl is as defined in the present disclosure.

Unless otherwise indicated, the term "amino" as used herein refers to a -NH₂ group.

Unless otherwise indicated, the term "alkylamino" as used herein refers to an alkyl-NH-group; and the term "dialkylamino" refers to an (alkyl)₂ -N- group, wherein alkyl is as defined in the present disclosure.

Unless otherwise indicated, the term "cyano" as used herein refers to a -C=N group.

Unless otherwise indicated, the term "carbonyl" as used herein refers to a -(C=O)- group.

Unless otherwise indicated, the term "carbonyloxy" as used herein refers to a -C(=O)-O-or a -O-C(=O)- group.

Unless otherwise indicated, the term "alkoxycarbonyl" as used herein refers to an alkyl-O-C(=O)- group, wherein alkyl is as defined in the present disclosure.

Unless otherwise indicated, the term "steroid group" as used herein refers to a group containing a cyclopentenylperhydrophenanthrene structure or a structure derived from cyclopentenylperhydrophenanthrene.

An embodiment of the present disclosure provides a cationic lipid of Formula (I): 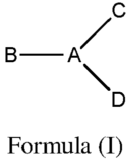
wherein A is selected from methine and nitrilo, wherein the methine may be optionally unsubstituted or substituted with R^{a};
wherein B is R¹-L¹-Y¹-L²- when A is methine, wherein
   R¹ is R²R³N-, in which R² and R³ are each independently C₁₋₆ alkyl,
   or R¹ is a 5- or 6-membered heterocyclyl containing 1 or 2 ring nitrogen atoms,
   wherein the C₁₋₆ alkyl or the 5- or 6-membered heterocyclyl is each independently unsubstituted or substituted with 1-3 R^{b};
   L₁ and L² are each independently selected from a chemical bond and a linear or branched C₁₋₁₂ alkylene, wherein each occurrence of the C₁₋₁₂ alkylene is optionally unsubstituted or substituted with 1 to 3 R^{c}; and
   Y is selected from the group consisting of -C(=X)-X-, -X-C(=X)-, -C(=X)-NH-, -NH-C(=X)-, -X-C(=X)-NH-, -NH-C(=X)-X-, -X-C(=X)-X-, -NH-C(=X)-NH-, and -X-X-; and
B is C₁₋₆ alkyl when A is nitrilo, wherein the C₁₋₆ alkyl is optionally unsubstituted or substituted with 1-3 R^{d};
C is -L³-Y²-L⁴-CH=CR⁴R⁵, wherein
   L³ and L⁴ are each independently selected from a chemical bond and a linear or branched C₁₋₁₂ alkylene, wherein each occurrence of the C₁₋₁₂ alkylene is optionally unsubstituted or substituted with 1 to 3 R^{e},
   Y² is selected from the group consisting of -C(=X)-X-, -X-C(=X)-, -C(=X)-NH-, -NH-C(=X)-, -X-C(=X)-NH-, -NH-C(=X)-X-, -X-C(=X)-X-, -NH-C(=X)-NH-, and -X-X-, and
   R⁴ and R⁵ are each independently selected from a linear or branched C₂₋₁₂ alkyl group, which is optionally unsubstituted or substituted with 1 to 3 R^{f};
D is -L⁵-Y³-L⁶-R⁶, wherein
   L⁵ and L⁶ are each independently selected from a chemical bond and a linear or branched C₁₋₁₂ alkylene, wherein each occurrence of the C₁₋₁₂ alkylene is optionally unsubstituted or substituted with 1 to 3 R^{g},
   Y³ is selected from the group consisting of -C(=X)-X-, -X-C(=X)-, -C(=X)-NH-, -NH-C(=X)-, -X-C(=X)-NH-, -NH-C(=X)-X-, -X-C(=X)-X-, -NH-C(=X)-NH-, and -X-X-, and
   R⁶ is independently selected from a saturated or unsaturated, linear or branched C₂₋₁₂ hydrocarbon group or a steroid group, wherein the C₂₋₁₂ hydrocarbon group or the steroid group is optionally unsubstituted or substituted with 1-3 R^{h};
or, D is -L³-Y²-L⁴-CH=CR⁴R⁵;
each occurrence of X is independently O or S, and
each occurrence of R^{a}, R^{b}, R^{c}, R^{d}, R^{e}, R^{f}, R^{g} and R^{h} are each independently selected from the group consisting of C₁₋₃ alkyl, C₁₋₃ alkoxy, C₁₋₃ alkylamino, di(C₁₋₃ alkyl)amino, C₁₋₃ alkoxycarbonyl, C₁₋₃ alkylcarbonyloxy, C₁₋₃ alkylcarbonylamino, C₁₋₃ alkylaminocarbonyl, hydroxy, halogen, and cyano.

In some aspects of the present disclosure, in the cationic lipid represented by Formula (I), A may be methine. Alternatively, in another aspect of the present disclosure, A may be nitrilo.

In another aspect of the present disclosure, in the cationic lipid represented by Formula (I), X may be O or S. For example, X may be O.

In another aspect of the present disclosure, in the cationic lipid represented by Formula (I), A is methine, B can be R¹-L¹-Y¹-L²-, wherein R¹ can be R²R³N-, and R² and R³ can each independently be C₁₋₆ alkyl, which is optionally unsubstituted or substituted with 1-3 substituents independently selected from the group consisting of C₁₋₃ alkyl, C₁₋₃ alkoxy, hydroxyl and halogen. For example, R² and R³ can each independently be C₂₋₄ alkyl, which is unsubstituted or substituted with 1-3 substituents independently selected from the group consisting of C₁₋₃ alkyl, C₁₋₃ alkoxy, hydroxyl and halogen, for example, methyl, ethyl, n-propyl, isopropyl, chloromethyl, chloroethyl, chloropropyl, hydroxymethyl, hydroxyethyl, hydroxypropyl, methoxymethyl, methoxyethyl, etc. In some aspects of the present disclosure, R² and R³ can be the same and can be methyl, ethyl, *n-*propyl, isopropyl, *n*-butyl, isopropyl or *tert*-butyl. In some aspects of the present disclosure, R² and R³ can be different and each independently selected from the group consisting of methyl, ethyl, *n-*propyl, isopropyl, *n*-butyl, isopropyl or *tert*-butyl.

Alternatively, in another aspect of the present disclosure, in the cationic lipid represented by Formula (I), A is methine, and B can also be R¹-L¹-Y¹-L²-, wherein R¹ can be a saturated 5- or 6-membered heterocyclyl containing 1 or 2 ring nitrogen atoms, which is unsubstituted or optionally substituted with 1-3 C₁₋₃ alkyl. For example, R¹ can be the group consisting of N-methyl-piperidin-4-yl, N-methyl-piperidin-2-yl, N-methyl-pyrrolidin-2-yl, N-ethyl-piperidin-4-yl, N-ethyl-piperidin-2-yl, N-ethyl-pyrrolidin-2-yl and piperidin-1-yl.

In another aspect of the present disclosure, in the cationic lipid represented by Formula (I), A is nitrilo, and B can be C₂₋₄ alkyl, which is unsubstituted or substituted with 1-3 substituents selected from the group consisting of hydroxyl, halogen and cyano. For example, B can be selected from the group consisting of methyl, ethyl, *n*-propyl, isopropyl, *n*-butyl, isobutyl and *tert*-butyl substituted with a primary hydroxyl group.

In another aspect of the present disclosure, in the cationic lipid represented by Formula (I), C can be -L³-Y²-L⁴-CH=CR⁴R⁵, wherein R⁴ and R⁵ can be the same or different and are each independently selected from linear C₂₋₁₂ alkyl.

In another aspect of the present disclosure, in the cationic lipid represented by Formula (I), D can be -L⁵-Y³-L⁶-R⁶, wherein R⁶ is a linear or branched C₆₋₁₈ hydrocarbon group containing one or more carbon-carbon double bonds; or, R⁶ can be a linear or branched C₆₋₁₈ hydrocarbon group containing two carbon-carbon double bonds; or, R⁶ can be a C₆₋₁₈ alkyl substituted with C₁₋₆ alkoxycarbonyl.

In alternative embodiments, in the cationic lipid represented by Formula (I), D can be - L³-Y²-L⁴-CH=CR⁴R⁵, wherein R⁴ and R⁵ are the same or different and are each independently selected from linear C₂₋₁₂ alkyl. For example, in some cases, C and D can be the same.

In another aspect of the present disclosure, in the cationic lipids represented by Formula (I), L¹, L², L³, L⁴, L⁵ and L⁶ can be each independently selected from a chemical bond and C₁₋₈ alkylene, wherein the C₁₋₈ alkylene is optionally unsubstituted or substituted with 1-3 substituents independently selected from the group consisting of C₁₋₃ alkyl, C₁₋₃ alkoxy, hydroxyl and halogen. In some cases, L¹, L², L³, L⁴, L⁵ and L⁶ can be each independently selected from a chemical bond and unsubstituted Ci-s alkylene, for example, chemical bond, methylene, ethylene, propylene, butylene, pentamethylene, hexamethylene, heptamethylene, octamethylene and 2,2-dimethylpropylene, and the like.

In another aspect of the present disclosure, in the cationic lipid represented by Formula (I), Y¹, Y² and Y³ can be each independently selected from the group consisting of -C(=X)-X-, -X-C(=X)-, -C(=X)-NH-, -NH-C(=X)-, -X-C(=X)-NH-, -NH-C(=X)-X-, -X-C(=X)-X-, -NH-C(=X)-NH-, and -X-X-, wherein each occurrence of X can be independently O or S. In certain cases, X can be O, and Y¹, Y² and Y³ can be each independently selected from the group consisting of - C(=O)-O-, -O-C(=O)-, -C(=O)-NH-, -NH-C(=O)-, -O-C(=O)-NH-, -NH-C(=O)-O-, -O-C(=O)-O-, -NH-C(=O)-NH-, and -O-O-. For example, Y¹, Y² and Y³ can be each independently selected from the group consisting of -C(=O)-O-, -O-C(=O)-, and -O-C(=O)-O-.

In some aspects of the present disclosure, the cationic lipid represented by Formula (I) may have a structure selected from the group consisting of:

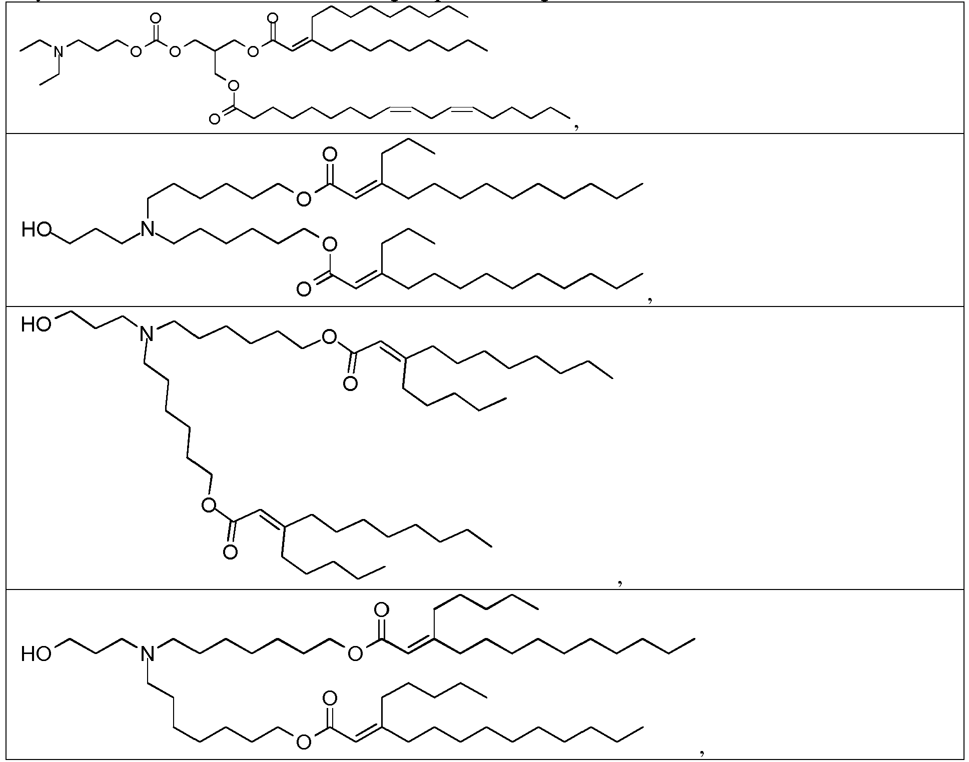

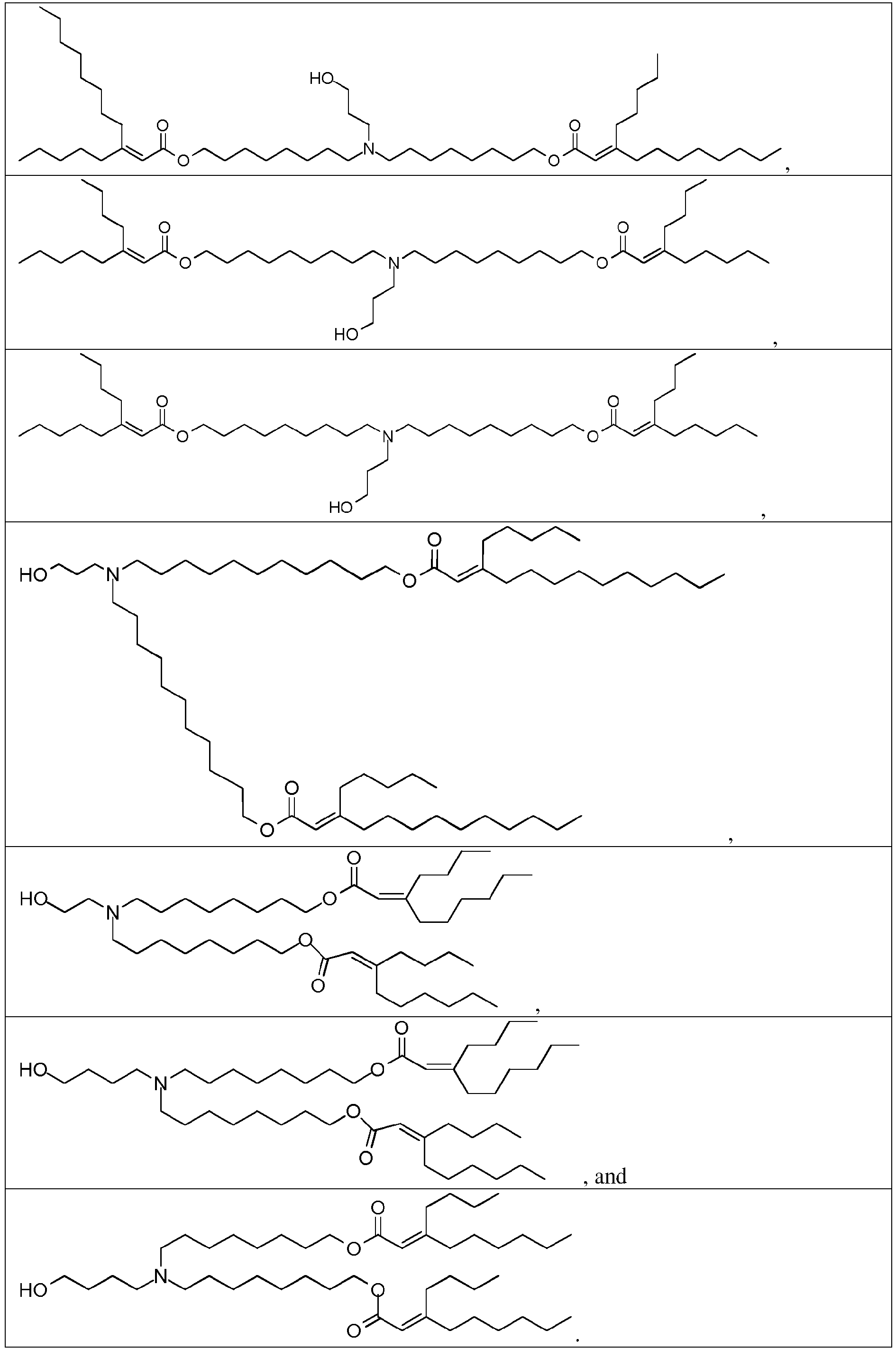

At least an embodiment of the present disclosure also discloses a liposome comprising any one of the above-mentioned cationic lipids.

At least an embodiment of the present disclosure also discloses a lipid nanoparticle comprising any one of the above-mentioned cationic lipids.

At least an embodiment of the present disclosure also discloses use of any of the above-mentioned cationic lipids for preparing liposomes.

At least an embodiment of the present disclosure also discloses use of any of the above-mentioned cationic lipids for preparing lipid nanoparticles.

The liposome or LNP according to at least an embodiment of the present disclosure can be used to encapsulate and deliver various nucleic acid drugs, including, but not limited to RNA-based nucleic acid drugs, such as tRNAs, siRNAs, antisense oligonucleotides, microRNAs, lncRNAs, nucleic acid aptamers, mRNAs, circular RNAs, self-replicating RNAs, and the like; and DNA-based nucleic acid drugs, such as plasmids, linearized plasmids, nanoplasmids, "doggybone" DNAs, closed-end RNAs, oligoDNAs, and the like. In some examples, the liposome or LNP according to the present disclosure can be used to deliver nucleic acid drugs for the treatment or prevention of diseases including but not limited to tumors, autoimmune diseases, infectious diseases, etc., such as to deliver tumor targeted therapeutic agents, COVID-19 vaccines, etc.

### EXAMPLES

The following examples provide a more detailed description of the preparation, characterization and properties of the compounds of the invention. It should be understood, however, that the invention as fully described herein and as set forth in the claims is not intended to be limited to the details of the following schemes or modes of preparation.

### ABBREVIATIONS:

The following abbreviations are used in the following examples:
r.t.: room temperature;
h/hr: hours
EA: ethyl acetate;
PE: petroleum ether;
DCM: dichloromethane;
DIEA: diisopropylethylamine;
EDCI: 1-(3-dimethylaminopropyl)-3-ethylcarbodiimide hydrochloride;
DMAP: 4-dimethylaminopyridine;
THF: tetrahydrofuran;
tBuONa: Sodium *tert*-butanolate;
tBuOK: potassium *tert-*butanolate;
MeOH: methanol;
TsOH: p-toluenesulfonic acid;
TfOH: trifluoroethanesulfonic acid;
ACN: acetonitrile;
DMF: N,N-dimethylformamide;
DMSO: dimethyl sulfoxide;
EtOH: ethanol;
NaH: sodium hydride;
DCC: dicyclohexylcarbodiimide;
TEA: triethylamine;
TsOH: p-toluenesulfonic acid;
NaOH: sodium hydroxide;
LiOH: lithium hydroxide;
NaI: sodium iodide;
K₂CO₃: potassium carbonate;
DSPC = distearoylphosphatidylcholine;
DMG-PEG2000 = 1,2-dimyristoyl-*rac*-glycero-3-methoxypolyethylene glycol-2000;
FFluc = firefly luciferase;
LNP= liposome nanoparticle;
PDI = polydispersity index; and
pKa = dissociation constant.

### PREPARATION EXAMPLES

### Example 1: Preparation of 3-(((3-(diethylamino)propoxy)carbonyl)oxy)-2-(((3-nonyldodec-2-enoyl)oxy)methyl)propyl (9Z,12Z)-octadeca-9,12-dienoate (CPL-001-01)

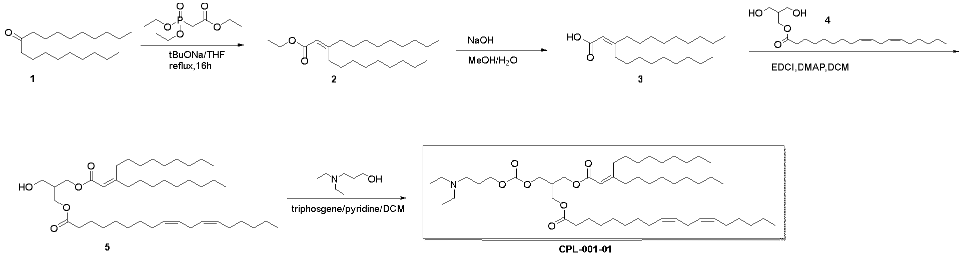

### Preparation of ethyl 3-nonyldodec-2-enoate (mixture 2)

To a mixture of ethyl 2-(diethoxyphosphoryl)acetate (33.6g, 150 mmol, 2.5 equivalent) in THF (200 mL) was added tBuONa (14.4g, 150 mmol, 2.5 equivalent) at 0°C under N₂ atmosphere, and then **Compound 1** (16.92 g, 60 mmol, 1.0 equivalent) in THF (136 mL) was added. The reaction was then stirred at reflux for 16 hours. 100 mL of water was added, and the mixture was extracted with EA (50 mL × 3), and washed with aqueous NaCl solution (50 mL × 3). The organic layer was dried with Na₂SO₄, filtered, concentrated, and purified by column chromatography on silica gel (PE:EA = 300/1) to obtain **Compound 2** (12.8 g, 60.6% yield) as a colorless oil.

### Preparation of 3-nonyldodec-2-enoic acid (Compound 3)

To a mixture of ethyl 3-nonyldodec-2-enoate (18g, 33.21mmol, 1.0 equivalent) in ethanol/water (180 mL/180 mL) was added NaOH (5.31g, 132.8 mmol, 4.0 equivalent) at 0°C under N₂ atmosphere. The reaction mixture was refluxed for 16 hours. The solvent was removed by evaporation. The mixture was adjusted to pH 4-5 with 1N HCl, extracted with EA (120 mL × 3), and washed with aqueous NaCl solution (120 mL). The organic layer was dried over Na₂SO₄, filtered, concentrated, and purified by column chromatography on silica gel (PE:EA=150/1) to obtain **Compound 3** (5.16 g, 30.2% yield) as a colorless oil.

¹H NMR (400 MHz, CDCl₃) δ 5.64 (s, 1H), 2.65-2.55 (m, 2H), 2.20-2.11 (m, 2H), 1.46-1.26 (m, 28H), 0.91-0.85 (m, 6H).

### Preparation of 3-hydroxy-2-(((3-nonyldodec-2-enoyl)oxy)methyl)propyl (9Z,12Z)-octadeca-9,12-dienoate (Compound 5)

To a solution of **Compound 3** (1.32 g, 4.08 mmol, 1.0 equivalent) in DCM (10 mL) were added DIEA(2.11 g, 16.32 mmol, 4.0 equivalent), EDCI (1.18 g, 6.12 mmol, 1.5 equivalent) and DMAP (100 mg, 0.82 mmol, 0.2 equivalent) at 0°C under N₂ atmosphere. A solution of **Compound 4** (1.5 g, 4.08 mmol, 1.0 equivalent) in DCM (5 mL) was then added. The reaction mixture was stirred at 30°C for 16 hours. 50 mL of water was added, and the mixture was extracted with DCM (50 mL × 3) and washed with aqueous NaCl solution (100 mL). The organic layer was dried over Na₂SO₄, filtered, concentrated, and purified by column chromatography on silica gel (PE:EA = 6/1) to obtain **Compound 5** (1.53 g, 55.6% yield) as a colorless oil.

¹H NMR (400 MHz, CDCl₃) δ 5.62 (s, 1H), 5.42-5.26 (m, 4H), 4.30-4.08 (m, 4H), 3.68-3.54 (m, 2H), 2.82-2.72 (m, 2H), 2.64-2.52 (m, 2H), 2.36-2.31 (m, 2H), 2.17-2.10 (m, 2H), 2.09-2.01 (m, 4H), 1.66-1.57 (m, 2H), 1.46-1.23 (m, 43H), 0.91-0.85 (m, 9H).

### Preparation of ethyl 3-nonyldodec-2-enoate (CPL-001-01)

To a solution of **Compound 5** (700 mg, 1.038 mmol, 1.0 equivalent) in DCM (10 mL) were added pyridine (820 mg, 10.38 mmol, 10.0 equivalent) and triphosgene (122 mg, 0.416 mmol, 0.4 equivalent) at 0°C under N₂ atmosphere. The reaction mixture was then stirred at 0°C for 0.5 hours. A solution of 3-(diethylamino)propan-1-ol (408 mg, 3.114 mmol, 3.0 equivalent) in DCM (1 mL) was then added at 0°C. The reaction mixture was stirred at 30°C for 16 hours. 20 mL of water was added, and the mixture was extracted with DCM (20 mL × 3), washed with aqueous NaCl solution (50 mL). The organic layer was dried over Na₂SO₄, filtered, concentrated, and purified by reverse phase chromatography to obtain the target **CPL-001-01** (101 mg, 11.6% yield) as a colorless oil.

LCMS: LC/MS (ESI) m/z: 832.7 (M+H)⁺.

¹H NMR (400 MHz, CDCl₃) δ 5.60 (s, 1H), 5.39-5.31 (m, 4H), 4.21-4.13 (m, 8H), 2.78-2.75 (m, 2H), 2.59-2.52 (m, 6H), 2.46-2.42 (m, 1H), 2.32-2.28 (m, 2H), 2.14-2.00 (m, 4H), 1.82-1.80 (m, 2H), 1.62-1.59 (m, 4H), 1.48-1.26 (m, 42H), 1.19-1.00 (m, 6H), 0.90-0.80 (m, 9H).

### Example 2: Preparation of ((3-hydroxypropyl)azanediyl)bis(hexane-6,1-diyl) (2E,2'E)-bis(3-propyltridec-2-enoate) (CPL-062-01)

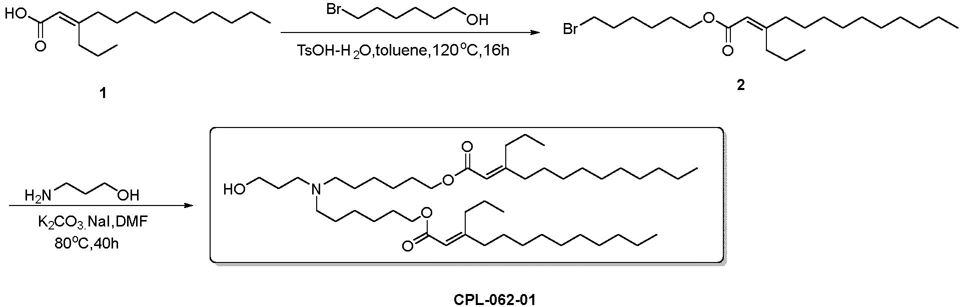

### Preparation of 6-bromohexyl (E)-3-propyltridec-2-enoate (Compound 2)

A mixture of **Compound 1** (514 mg, 2.02 mmol, 1.0 equivalent), 6-bromohexan-1-ol (438 mg, 2.42 mmol, 1.2 equivalent), TsOH-H₂O (38 mg, 0.202 mmol, 0.1 equivalent) in toluene (8 mL) was stirred at 120°C for 16 hours. The mixture was concentrated and purified by chromatography on silica gel (PE: EA= 150: 1) to obtain **Compound 2** (674 mg, 80% yield) as a yellow oil.

¹H NMR (400 MHz, CDCL₃) δ 5.62 (d, *J* = 5.6 Hz, 1H), 4.09 (t, *J* = 6.4 Hz, 2H), 3.42 (t, *J* = 6.8 Hz, 2H), 2.59-2.55 (m, 2H), 2.14-2.09 (m, 2H), 1.90-1.83 (m, 2H), 1.69-1.62 (m, 2H), 1.50-1.37 (m, 8H), 1.29-1.26 (m, 14H), 0.97-0.86 (m, 6H).

### Preparation of ((3-hydroxypropyl)azanediyl)bis(hexane-6,1-diyl) (2E,2'E)-bis(3-propyltridec-2-enoate) (CPL-062-01)

A mixture of **Compound 2** (674 mg, 1.62 mmol, 2.2 eq), 3-aminopropan-1-ol (55 mg, 0.736 mmol, 1.0 eq), K₂CO₃ (406 mg, 2.94 mmol, 4.0 eq) and NaI (44 mg, 0.294 mmol, 0.1 eq) in ACN (10 mL) was stirred at 80°C for 16 hours. The mixture was concentrated and purified by preparative HPLC to obtain the **target CPL-062-01** (257 mg, 46.2% yield) as a colorless oil.

LCMS: LC/MS (ESI) m/z: 748.23 (M+H)⁺.

¹H NMR (400 MHz, CDCL₃) δ 5.62 (d, *J* = 5.2 Hz, 1H 2H), 4.08 (t, *J* = 6.4 Hz, 4H), 3.83 (t, *J =* 5.2 Hz, 2H), 3.20-3.05 (m, 2H), 3.02-2.80 (m, 2H), 2.59-2.55 (m, 4H), 2.14-2.10 (m, 4H), 2.00-1.90 (m, 2H), 1.69-1.62 (m, 6H), 1.52-1.26 (m, 48H), 0.96-0.86 (m, 12H).

### Example 3: Preparation of ((3-hydroxypropyl)azanediyl)bis(hexane-6,1-diyl) (2E,2'E)-bis(3-pentylundec-2-enoate) (CPL-069-01)

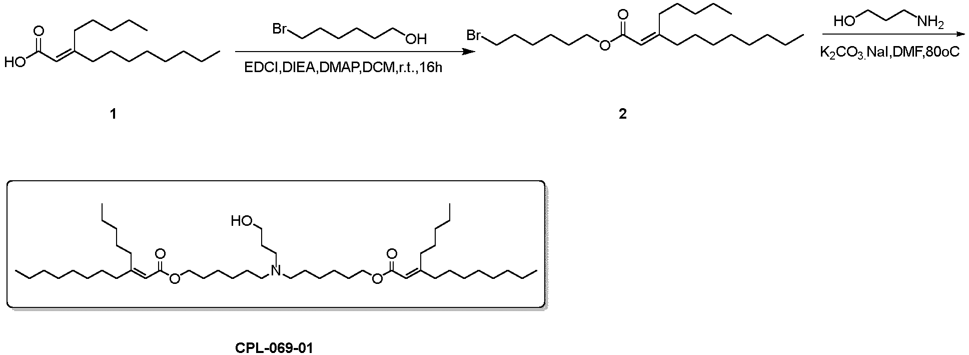

### Preparation of 6-bromohexyl (E)-3-pentylundec-2-enoate (Compound 2)

To a solution of (E)-3-pentylundec-2-enoic acid (600 mg, 2.4 mmol, 1.0 equivalent) and 6-bromoheptan-1-ol (514 mg, 2.12 mmol, 1.2 equivalent) in toluene (5 mL) was added TfOH.HzO (50 mg, 0.264 mmol, 0.1 equivalent) under nitrogen atmosphere. The reaction mixture was stirred at 120°C for 16 hours. The solvent was removed under vacuum. The residue was passed through a silica gel column purification column (PE/EA=50/1) to obtain **Compound 2** (600 mg, 60% yield) as a colorless oil. LCMS: LC/MS (ESI) m/z: 417.14 (M+H)⁺.

1H NMR (400 MHz, CDCl3) δ 5.61 (s, 1H), 4.10-4.06 (m, 2H), 3.46-3.40 (m, 2H), 2.60-2.56 (m, 2H), 2.40-2.37 (m, 2H), 2.18-2.10 (m, 2H), 1.89-1.85 (m, 2H), 1.68-1.55 (m, 2H), 1.51-1.23 (m, 21H), 0.86-0.92 (m, 6H).

### Preparation of ((3-hydroxypropyl)azanediyl)bis(hexane-6,1-diyl) (2E,2'E)-bis(3-pentylundec-2-enoate)

To a solution of 7-bromoheptyl (Z)-3-propylhept-2-enoate (600 mg, 1.43 mmol, 2.1 eq) and 3-aminopropan-1-ol (49 mg, 0.65 mmol, 1.0 eq) in ACN (5 mL) were added K₂CO₃ (360 mg, 2.535 mmol, 4.0 eq) and NaI (49 mg, 0.33 mmol, 0.5 eq) at 25°C. The reaction mixture was stirred at 80°C for 20 hours. 30 mL of water was added, and the mixture was extracted with EA (20 mL × 3). The organic layer was washed with aqueous NaCl solution (30 mL × 3) and dried over Na₂SO₄, filtered, concentrated and purified by preparative HPLC to obtain the **target CPL-069-01** (127.1 mg, 7% yield) as a colorless oil. LCMS: LCMS (ESI) m/z: 748.23 (M+H)⁺.

1H NMR (400 MHz, CDCl3) δ 5.61 (s, 2H), 4.08-4.05 (m, 4H), 3.81-3.72 (m, 2H), 2.65-2.56 (m, 6H), 2.42-2.39 (m, 4H), 2.16-2.11 (m, 4H), 1.62-1.27 (m, 54H), 0.93-0.86 (m, 12H).

### Example 4: Preparation of ((3-hydroxypropyl)azanediyl)di(heptane-7,1-diyl) (2E,2'E)-di(3-pentyltridec-2-enoate) (CPL-092-01)

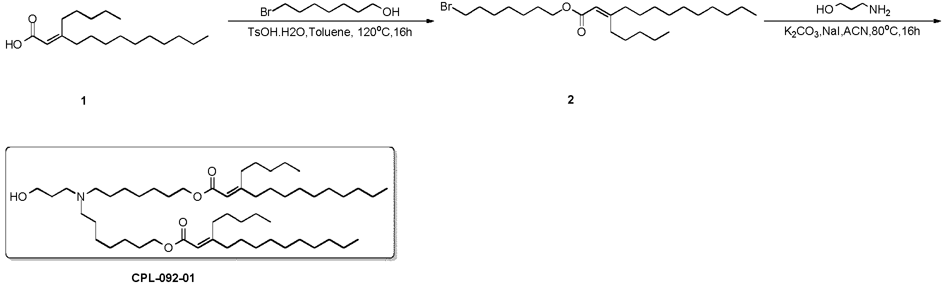

### Preparation of 7-bromohexyl (E)-3-pentyltridec-2-enoate (Compound 2)

To a solution of (E)-3-pentyltridec-2-enoic acid (600 mg, 2.126 mmol, 1.0 equivalent) and 7-bromoheptan-1-ol (412.5 mg, 2.126 mmol, 1.0 equivalent) in toluene (10 mL) was added TfOH.HzO (48.48 mg, 0.213 mmol, 0.1 equivalent) under nitrogen atmosphere. The reaction mixture was stirred at 120°C for 16 hours. The solvent was removed under vacuum. The residue was passed through a silica gel column purification column (PE/EA=50/1) to obtain **Compound 2** (700 mg, 71.8% yield) as a colorless oil. LCMS: LC/MS (ESI) m/z: 459.28 (M+H)⁺.

1H NMR (400 MHz, chloroform-d) δ 5.61 (s, 1H), 4.10-4.06 (m, 2H), 3.42-3.40 (m, 2H), 2.63-2.53 (m, 2H), 2.15-2.11 (m, 2H), 1.90-1.82 (m, 2H), 1.69-1.61 (m, 2H), 1.48-1.41 (m, 4H), 1.38-1.23 (m, 24H), 0.90-0.86 (m, 6H).

### Preparation of ((3-hydroxypropyl)azanediyl)di(heptane-7,1-diyl) (2E,2'E)-di(3-pentyltridec-2-enoate) (CPL-092-01)

To a solution of 7-bromoheptyl (E)-3-pentyltridec-2-enoate (700 mg, 1.527 mmol, 2.1 eq) and 3-aminopropan-1-ol (54.57 mg, 0.727 mmol, 1.0 eq) in ACN (10 mL) were added K₂CO₃ (401.30 mg, 2.908 mmol, 4.0 eq) and NaI (54.41 mg, 0.363 mmol, 0.5 eq) at 25°C, and the reaction mixture was stirred at 80°C for 20 hours. 30 mL of water was added to the mixture, and extracted with EA (20 mL×3). The organic layer was washed with aqueous NaCl solution (30 mL × 3), and dried over Na₂SO₄, filtered, concentrated and purified by preparative HPLC to obtain the **target CPL-092-01** (265.94 mg, 44.0% yield) as a colorless oil. LCMS: LCMS (ESI) m/z: 832.77 (M+H)⁺.

1H NMR (400 MHz, chloroform-d) δ 5.61 (s, 2H), 4.08-4.05 (m, 4H), 3.81-3.78 (m, 2H), 2.64-2.41 (m, 10H), 2.14-2.10 (m, 4H), 1.68-1.62 (m, 6H), 1.50-1.26 (m, 60H), 0.91-0.86 (m, 12H).

### Example 5: Preparation of ((3-hydroxypropyl)azanediyl)bis(octane-8,1-diyl) (2E,2'E)-bis(3-pentylundec-2-enoate) (CPL-113-01)

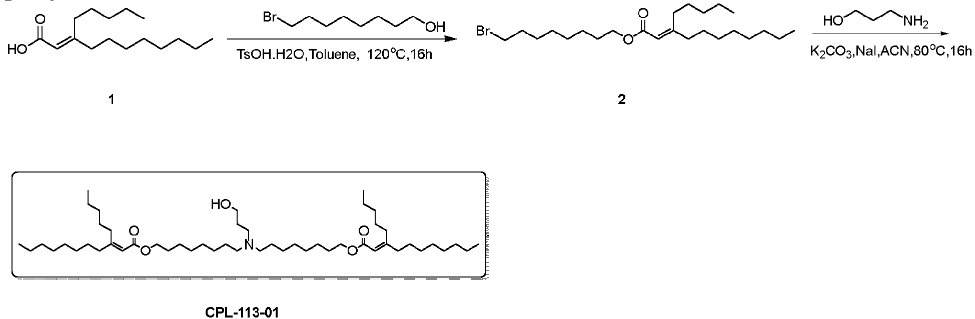

### Preparation of 8-bromooctyl (E)-3-pentylundec-2-enoate

To a solution of (E)-3-pentylundec-2-enoic acid (800 mg, 3.1 mmol, 1.0 equivalent) and 6-bromoheptan-1-ol (774 mg, 3.7 mmol, 1.2 equivalent) in toluene (5 mL) was added TfOH.HzO (59 mg, 0.31 mmol, 0.1 equivalent) under nitrogen atmosphere. The reaction mixture was stirred at 120°C for 16 hours. The solvent was removed under vacuum. The residue was purified by silica gel column (PE/EA=50/1) to obtain **Compound 2** (880 mg, 60% yield) as a colorless oil.

1H NMR (400 MHz, CDCl3) δ 5.61 (s, 1H), 4.09-4.05 (m, 2H), 3.42-3.39 (m, 2H), 2.60-2.56 (m, 2H), 2.14-2.11 (m, 2H), 1.89-1.60 (m, 2H), 1.44-1.27 (m, 28H), 0.91-0.86 (m, 6H).

### Preparation of ((3-hydroxypropyl)azanediyl)bis(octane-8,1-diyl) (2E,2'E)-bis(3-propyltridec-2-enoate)

To a solution of 7-bromoheptyl (Z)-3-propylhept-2-enoate (880 mg, 1.97 mmol, 2.1 eq) and 3-aminopropan-1-ol (68 mg, 0.9 mmol, 1.0 eq) in ACN (5 mL) were added K₂CO₃ (497 mg, 3.6 mmol, 4.0 eq) and NaI (67 mg, 0.45 mmol, 0.5 eq) at 25°C. The reaction mixture was stirred at 80°C for 20 hours. 30 mL of water was added, and the mixture was extracted with EA (20 mL × 3). The organic layer was washed with aqueous NaCl solution (30 mL × 3) and dried over Na₂SO₄, filtered, concentrated and purified by preparative HPLC to obtain the **target CPL-113-01** (101.27 mg, 5% yield) as a colorless oil. LCMS: LCMS (ESI) m/z: 804.34 (M+H)⁺.

1H NMR (400 MHz, CDCl3) δ 5.61 (s, 2H), 4.08-4.05 (m, 4H), 3.81-3.78 (m, 2H), 2.64-2.56 (m, 6H), 2.42-2.38 (m, 4H), 2.14-2.11 (m, 4H), 1.69-1.60 (m, 6H), 1.46-1.21 (m, 56H), 0.91-0.83 (m, 12H)

### Example 6: Preparation of ((3-hydroxypropyl)azanediyl)bis(nonane-9,1-diyl) (2E,2'E)-bis(3-butyloct-2-enoate) (CPL-133-01)

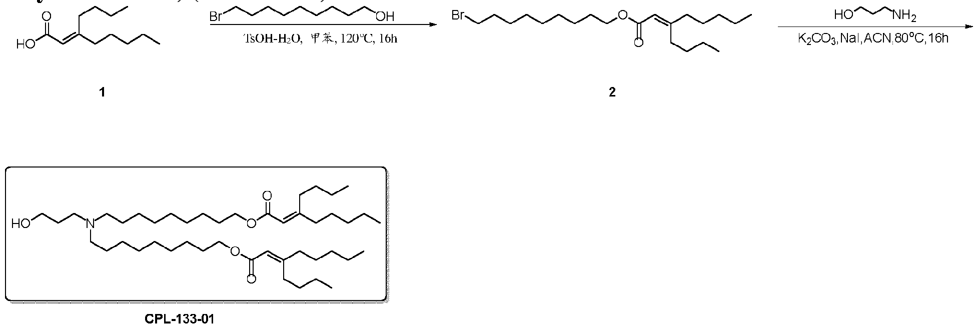

### Preparation of 9-bromononyl (E)-3-butyloctyl-2-enoate

A mixture of Compound **1** (500 mg, 2.53 mmol, 1.0 equivalent), 9-bromononan-1-ol (840 mg, 3.78 mmol, 1.5 equivalent), TsOH-H₂O (96 mg, 0.51 mmol, 0.2 equivalent) in toluene (10 mL) was stirred at 120°C for 16 hours. The mixture was concentrated and purified by chromatography on silica gel (PE: EA = 300: 1) to obtain **Compound 2** (720 mg, 70.8% yield) as a yellow oil thing.

¹H NMR (400 MHz, CDCl₃) δ 5.61 (s, 2H), 4.08-4.05 (m, 2H), 3.42-3.38 (m, 2H), 2.60-2.56 (m, 2H), 2.15-2.11 (m, 2H), 1.87-1.81 (m, 2H), 1.65-1.62 (m, 2H), 1.48-1.25 (m, 20H), 0.93-0.87(m, 6H).

### Preparation of ((3-hydroxypropyl)azanediyl)bis(nonane-9,1-diyl) (2E,2'E)-bis(3-butyloct-2-enoate) (CPL-133-01)

A mixture of **Compound 2** (720 mg, 1.79 mmol, 2.4 equivalent), 3-aminopropan-1-ol (56 mg, 0.75 mmol, 1.0 equivalent), K₂CO₃ (412 mg, 2.98 mmol, 4.0 equivalent) and NaI (23 mg, 0.15 mmol, 0.2 equivalent) in ACN (10 mL) was stirred at 80°C for 16 hours. The mixture was concentrated and purified by preparative HPLC to obtain the **target CPL-133-01** (129 mg, 24% yield) as a colorless oil

LCMS: LC/MS (ESI) m/z: 720.6 (M+H)⁺.

¹H NMR (400 MHz, CDCl₃) δ 5.61 (s, 2H), 4.08-4.03 (m, 4H), 3.83-3.81 (m, 2H), 3.03-2.93 (m, 2H), 2.73-2.60 (m, 4H), 2.59-2.56 (m, 4H), 2.15-2.11 (m, 4H), 2.01-1.86 (m, 2H), 1.65-1.27(m, 48H), 0.96-0.82 (m, 12H).

### Example 7: Preparation of ((3-hydroxypropyl)azanediyl)bis(undecane-11,1-diyl) (2E,2'E)-bis(3-butylnon-2-enoate) (CPL-174-01)

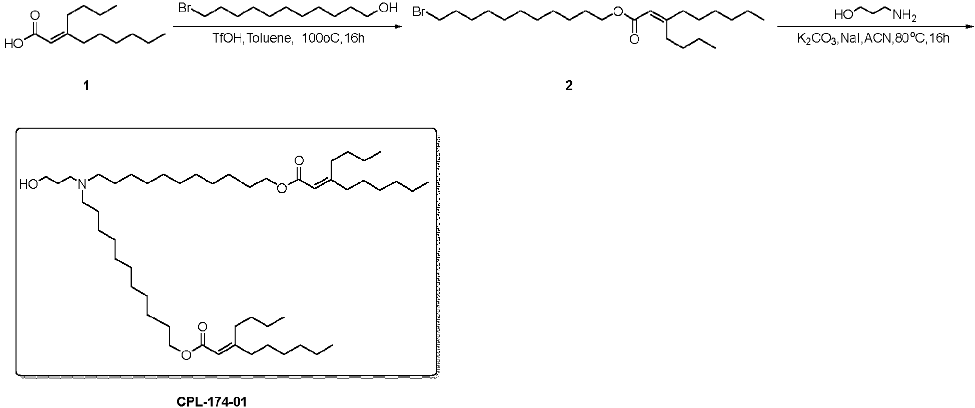

### Preparation of 11-bromoundecyl (E)-3-butylnon-2-enoate (Compound 2)

To a solution of (Z)-3-butylnon-2-enoic acid (500 mg, 2.356 mmol, 1.0 equivalent) and 11-bromoundecan-1-ol (589.21 mg, 2.356 mmol, 1.0 equivalent) in toluene (10 mL) was added TfOH (44.85 mg, 0.236 mmol, 0.1 equivalent) under nitrogen atmosphere. The reaction mixture was stirred at 120°C for 16 hours. The solvent was removed under vacuum. The residue was passed through a silica gel column purification column (PE/EA=50/1) to obtain **Compound 2** (730 mg, 69.8% yield) as a colorless oil. LCMS: LC/MS (ESI) m/z: 445.26 (M+H)⁺.

1H NMR (400 MHz, chloroform-d) δ 5.61 (s, 1H), 4.09-4.05 (m, 2H), 3.42-3.39 (m, 2H), 2.61-2.56 (m, 2H), 2.17-2.11 (m, 2H), 1.89-1.82 (m, 2H), 1.67-1.62 (m, 2H), 1.48-1.28 (m, 26H), 0.94-0.86 (m, 6H).

### Preparation of ((3-hydroxypropyl)azanediyl)bis(undecane-11,1-diyl) (2E,2'E)-bis(3-butyloct-2-enoate) (CPL-174-01)

To a solution of 11-bromoundecyl (E)-3-butylnon-2-enoate (730 mg, 1.643 mmol, 2.1 eq) and 3-aminopropan-1-ol (58.70 mg, 0.782 mmol, 1.0 eq) in ACN (10 mL) were added K₂CO₃ (431.66 mg, 3.128 mmol, 4.0 eq) and NaI (58.61 mg, 0.391 mmol, 0.5 eq) at 25°C, and the reaction mixture was stirred at 80°C for 20 hours. 40 mL of water was added to the mixture, and extracted with EA (30 mL×3). The organic layer was washed with aqueous NaCl solution (40 mL × 3), and dried over Na₂SO₄, filtered, concentrated and purified by preparative HPLC to obtain the **target CPL-174-01** (181.92 mg, 28.9% yield) as a colorless oil. LCMS: LCMS (ESI) m/z: 804.74 (M+H)⁺.

1H NMR(400 MHz, CDCl3-d) δ 5.61 (s, 2H), 4.08-4.05 (m, 4H), 3.81-3.79 (m, 2H), 2.65 - 2.41 (m, 10H), 2.15-2.11 (m, 4H), 1.67-1.62 (m, 6H), 1.46 -1.27 (m, 56H), 0.94-0.86 (m, 12H).

### Example 8: Preparation of ((3-hydroxypropyl)azanediyl)bis(undecane-11,1-diyl) (2E,2'E)-bis(3-pentyltridec-2-enoate) (CPL-180-01)

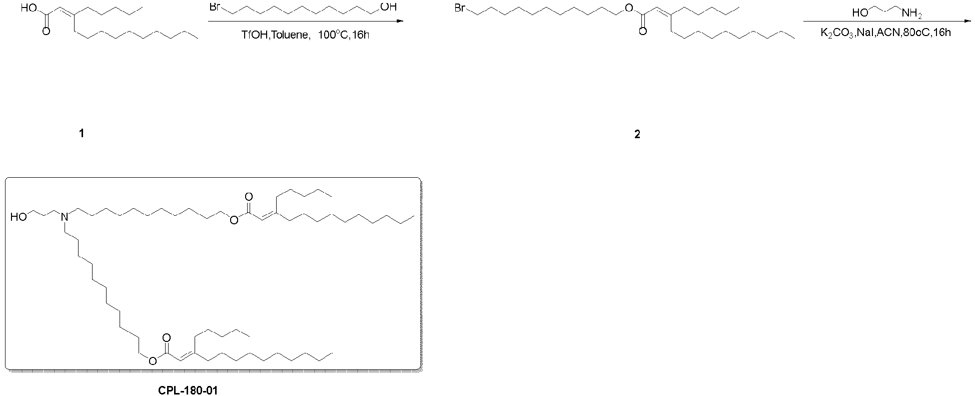

### Preparation of 11-bromoundecyl (Z)-3-propyltridec-2-enoate (Compound 2)

To a solution of (Z)-3-pentyltridec-2-enoic acid (600 mg, 1.569 mmol, 1.0 equivalent) and 11-bromoundecan-1-ol (392.39 mg, 1.569 mmol, 1.0 equivalent) in toluene (5 mL) was added TfOH.HzO (29.64 mg, 0.156 mmol, 0.1 equivalent) under nitrogen atmosphere. The reaction mixture was stirred at 120°C for 16 hours. The solvent was removed under vacuum. The residue was passed through a silica gel column purification column (PE/EA=50/1) to obtain **Compound 2** (440 mg, 54.6% yield) as a colorless oil. LCMS: LC/MS (ESI) m/z: 515.34 (M+H)⁺.

1H NMR(400 MHz, CDCl3-d) δ 5.61 (s, 1H), 4.07 (m, 2H), 3.40 (m, 2H), 2.61 - 2.53 (m, 2H), 2.12 (m, 2H), 1.85 (m, 2H), 1.63 (m, 2H), 1.45 (d, J = 7.4 Hz, 4H), 1.29 (m, 32H), 0.89 (m, 6H).

### Preparation of ((3-hydroxypropyl)azanediyl)bis(undecane-11,1-diyl) (2E,2'E)-bis(3-pentyltridec-2-enoate) (CPL-180-01)

To a solution of 11-bromoundecyl (Z)-3-pentyltridec-2-enoate (440 mg, 0.855 mmol, 2.1 eq) and 3-aminopropan-1-ol (30.55 mg, 0.407 mmol, 1.0 eq) in ACN (5 mL) were added K₂CO₃ (224.66 mg, 1.628 mmol, 4.0 eq) and NaI (30.58 mg, 0.204 mmol, 0.5 eq) at 25°C, and the reaction mixture was stirred at 80°C for 20 hours. 30 mL of water was added to the mixture, and extracted with EA (20 mL × 3). The organic layer was washed with aqueous NaCl solution (30 mL × 3), and dried over Na₂SO₄, filtered, concentrated and purified by preparative HPLC to obtain the **target CPL-180-01** (194.57 mg, 50.6% yield) as a colorless oil. LCMS: LCMS (ESI) m/z: 944.89 (M+H)⁺.

1H NMR (400 MHz, CDCl3-d) δ 5.61 (s, 2H), 4.08-4.05 (m, 4H), 3.82-3.81 (m, 2H), 2.84-2.56 (m, 10H), 2.14-2.10 (m, 4H), 1.80 -1.61 (m, 6H), 1.48-1.41 (m, 8H), 1.38 - 1.23 (m, 68H), 0.91-0.86 (m, 12H).

### Example 9: Preparation of ((2-hydroxyethyl)azanediyl)bis(octane-8,1-diyl) (2E,2'E)-bis(3-butylnon-2-enoate) (CPL-455-01)

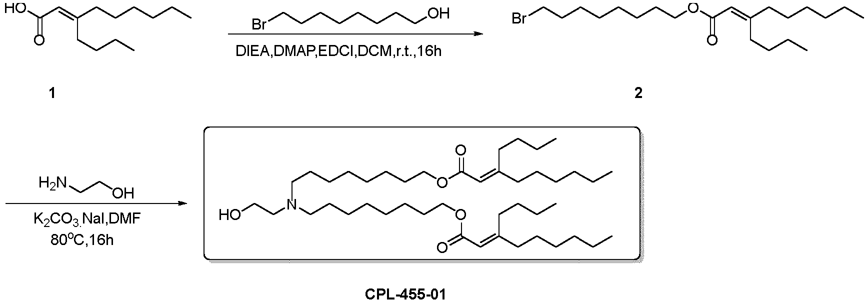

### Preparation of 8-bromooctyl 3-butylhept-2-enoate (Compound 2)

A mixture of **Compound 1** (1.5 g, 7.07 mmol, 1.0 equivalent), 8-bromooctan-1-ol (1.77 g, 8.48 mmol, 1.2 equivalent) and TsOH-H₂O (135 mg, 0.707 mmol, 0.1 equivalent) in toluene (30 mL) was stirred for 16 hours at 120°C under nitrogen atmosphere. The mixture was concentrated and purified by silica gel column to obtain **Compound 2** (2.69 g, 94.7% yield) as a colorless oil.

¹H NMR (400 MHz, CDCL₃) δ 5.61(s, 1H), 4.08-4.04 (m, 2H), 3.42-3.39 (m, 2H), 2.60-2.56 (m, 2H), 2.18-2.11 (m, 2H), 1.89-1.82 (m, 2H), 1.67-1.60 (m, 2H), 1.46-1.27 (m, 20H), 0.93-0.87 (m, 6H).

### Preparation of ((2-hydroxyethyl)azanediyl)bis(octane-8,1-diyl) (2E,2'E)-bis(3-butylnon-2-enoate) (CPL-455 -01)

A mixture of **Compound 2** (906 mg, 2.248 mmol, 2.2 equivalent), 2-aminoethan-1-ol (62 mg, 1.022 mmol, 1.0 equivalent), K₂CO₃ (564 mg, 4.088 mmol, 4.0 equivalent) and NaI (15 mg, 0.1022 mmol, 0.1 equivalent) in ACN (15 mL) was stirred at 80°C for 16 hours. The mixture was concentrated and purified by preparative HPLC to obtain the **target CPL-455-01** (322 mg, 44.6% yield) as a colorless oil.

LCMS: LC/MS (ESI) m/z: 706.7 (M+H)⁺.

¹H NMR (400 MHz, CDCl₃) δ 5.61 (s, 2H), 4.08-4.03 (m, 4H), 3.90-3.80 (m, 2H), 3.05-2.87 (m, 6H), 2.60-2.56 (m, 4H), 2.15-2.11 (m, 4H), 1.70-1.60 (m, 4H), 1.46-1.27 (m, 44H), 0.93-0.86 (m, 12H).

### Example 10: Preparation of ((3-hydroxypropyl)azanediyl)bis(octane-8,1-diyl) (2E,2'E)-bis(3-butylnon-2-enoate) (CPL-456-01)

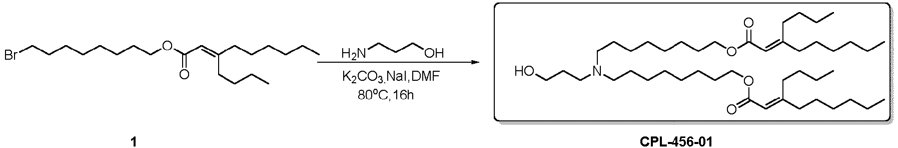

### Preparation of ((3-hydroxypropyl)azanediyl)bis(octane-8,1-diyl) (2E,2'E)-bis(3-butyloct-2-enoate) (CPL-456-01)

A mixture of **Compound 1** (903 mg, 2.24 mmol, 2.2 eq), 3-aminopropan-1-ol (76 mg, 1.02 mmol, 1.0 eq), K₂CO₃ (562 mg, 4.07 mmol, 4.0 eq) and NaI (15 mg, 0.10 mmol, 0.1 eq) in ACN (15 mL) was stirred at 80°C for 16 hours. The mixture was concentrated and purified by preparative HPLC to obtain the **target CPL-456-01** (351 mg, 47.8% yield) as a colorless oil.

LCMS: LC/MS (ESI) m/z: 720.0 (M+H)⁺.

¹H NMR (400 MHz, CDCL₃) δ 5.61 (s, 2H), 4.08-4.03 (m, 4H), 3.83-3.80 (m, 2H), 3.03-2.85 (m, 2H), 2.88-2.65 (m, 2H), 2.60-2.56 (m, 4H), 2.15-2.11 (m, 4H), 1.95-1.80 (m, 2H), 1.72-1.55 (m, 6H), 1.46-1.27 (m, 44H), 0.93-0.86 (m, 12H).

### Example 11: Preparation of ((4-hydroxybutyl)azanediyl)bis(octane-8,1-diyl) (2E,2'E)-bis(3-butylnon-2-enoate) (CPL-457-01)

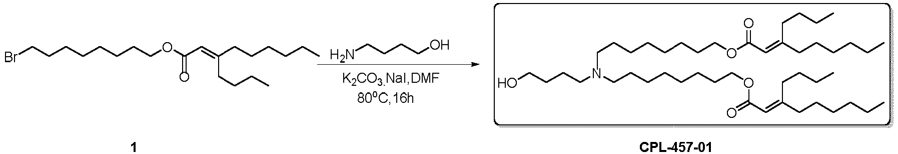

### Preparation of ((4-hydroxybutyl)azanediyl)bis(octane-8,1-diyl) (2E,2'E)-bis(3-butylnon-2-enoate) (CPL-457)

A mixture of **Compound 1** (881 mg, 2.186 mmol, 2.2 eq), 4-aminobutan-1-ol (88 mg, 0.994 mmol, 1.0 eq), K₂CO₃ (548 mg, 3.976 mmol, 4.0 eq) and NaI (15 mg, 0.0994 mmol, 0.1 eq) in ACN (15 mL) was stirred at 80°C for 16 hours. The mixture was concentrated and purified by preparative HPLC to obtain the **target CPL-457-01** (334 mg, 45.8% yield) as a colorless oil.

LCMS: LC/MS (ESI) m/z: 734.7 (M+H)⁺.

¹H NMR (400 MHz, CDCl₃) δ 5.61 (s, 2H), 4.08-4.03 (m, 4H), 3.73-3.70 (m, 2H), 3.03-2.87 (m, 4H), 2.60-2.56 (m, 4H), 2.15-2.12 (m, 4H), 2.00-1.93 (m, 2H), 1.72-1.62 (m, 6H), 1.46-1.28 (m, 46H), 0.93-0.86 (m, 12H).

### Example 12: Preparation of pentyl 5-((2-hydroxyethyl)(6-((3-octylundec-2-enoyl)oxy)hexyl)amino)dodecanoate (CPL-002)

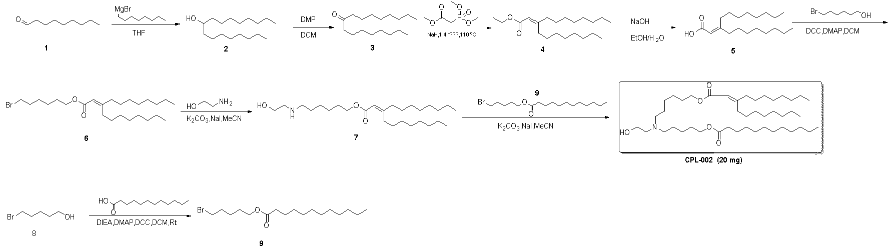

A mixture of compound 5-bromopentyl dodecanoate (131 mg, 0376 mmol, 1.1 equivalent), 6-(2-hydroxyethylamino)-hexyl 3-octylundec-2-enoate (150 mg, 0.342 mmol, 1.0 equivalent), K₂CO₃ (47 mg, 0.342 mmol, 1 equivalent), Cs₂CO₃ (33 mg, 0.1026 mmol, 0.3 equivalent) and NaI (15 mg, 0.1026 mmol, 0.3 equivalent) in ACN (6 mL) was stirred at 85°C for 16 hours. 40 mL of water was added and extracted three times with 20 mL of ethyl acetate. The organic phase was washed three times with 40 mL of sodium chloride solution, filtered and dried over sodium sulfate. The mixture was concentrated and purified by preparative HPLC to obtain the target CPL-002 (60 mg, 25% yield) as a colorless oil.

LCMS: LC/MS (ESI) m/z: 708.7 (M+H)⁺.

1HNMR(400 MHz, CDCl3) δ 5.54 (s, 1H), 3.97-4.01 (m, 4H), 3.50-3.53 (m, 2H), 2.56-2.59 (m, 2H), 2.54 - 2.39 (m, 6H), 2.22 (t,J = 7.5 Hz, 2H), 2.09 - 2.00 (m, 2H), 1.53-1.61 (m, 6H), 1.48 - 1.42 (m, 3H), 1.40 - 1.34 (m, 4H), 1.33 - 1.28 (m, 4H), 1.20 (d,J = 10.3 Hz, 40H), 0.79-0.82 (m,9H).

### Example 13: Preparation of ((4-hydroxybutyl)azanediyl)bis(pentane-5,1-diyl)(2E,2'E)-bis(3-hexylundec-2-enoate) (CPL-003)

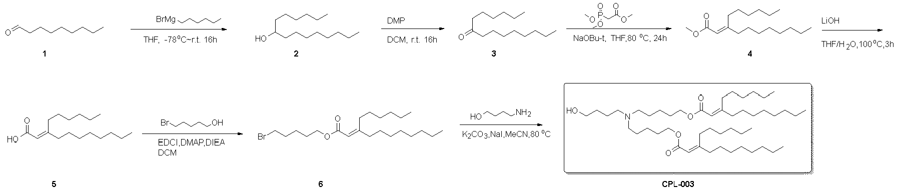

A mixture of (E)-5-bromopentyl-3-hexylundec-2-enoate (50 mg, 0.12 mmol, 1.0 equivalent), 4-amino-1-butanol (5.34 mg, 0.06 mmol, 0.3 equivalent), K₂CO₃ (49.68 mg, 0.36 mmol, 3.0 equivalent) and NaI (5.4 mg, 0.036 mmol, 0.3 equivalent) in ACN (1 mL) was stirred at 80°C for 24 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-003 (28.81 mg, 30.7% yield) as a colorless oil.

LCMS: LCMS (ESI) m/z: 762.69 (M+H)⁺.

1H NMR (400 MHz, Chloroform-d) δ 5.61 (s, 1H), 4.07 (q, J = 6.2 Hz, 4H), 3.73 - 3.65 (m, 2H), 3.00 (d, J = 23.7 Hz, 2H), 2.90 (d, J = 13.6 Hz, 4H), 2.58 (m, 3H), 2.13 (t, J = 7.7 Hz, 3H), 2.06 - 2.00 (m, 2H), 1.92 - 1.85 (m, 2H), 1.83 - 1.74 (m, 4H), 1.69 (d, J = 6.9 Hz, 4H), 1.43 (m, 11H), 1.29 (m, 36H), 0.89 (m, 12H).

### Example 14: Preparation of ((3-hydroxypropyl)azanediyl)bis(butane-4,1-diyl)(2E,2'E)-bis(3-butylundec-2-enoate) (CPL-021)

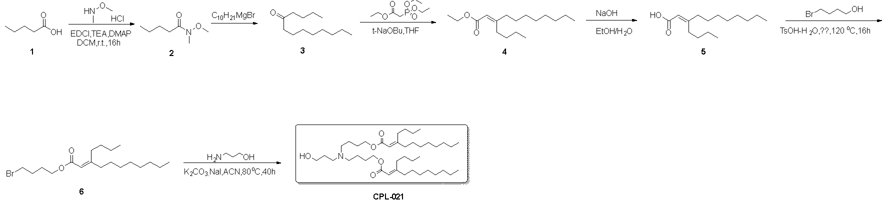

A mixture of (E)-4-bromobutyl-3-butylundec-2-enoate (286 mg, 0.762 mmol, 2.4 equivalent), 3-amino-1-propanol (24 mg, 0.317 mmol, 1 equivalent), K₂CO₃ (175 mg, 1.268 mmol, 4.0 equivalent), and NaI (24 mg, 0.159 mmol, 0.5 equivalent) in ACN (15 mL) was stirred at 80°C for 40 hours. The mixture was concentrated and purified by preparative HPLC to obtain the target CPL-021 (49 mg, 23.1% yield) as a colorless oil.

LCMS: LC/MS (ESI) m/z: 663.58 (M+H)⁺

1H NMR(400 MHz, CDCL3) δ 5.62 (s, 1H), 4.10-4.06 (m, 4H), 3.81-3.78 (m, 2H), 2.65-2.56 (m, 6H), 2.48-2.44 (m, 4H), 2.15-2.10 (m, 4H), 1.70-1.54 (m, 6H), 1.50-1.19 (m, 37H), 0.93-0.87 (m, 12H).

### Example 15: Preparation of ((3-hydroxypropyl)azanediyl)bis(butane-4,1-diyl) (2E,2'E)-bis(2-hexylidenedecanoate) CPL-025)

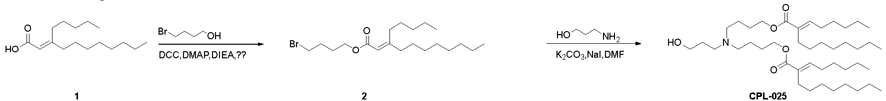

A mixture of (E)-4-bromobutyl-3-pentylundecyl-2-enoate (300 mg, 0.77 mmol, 2.2 equivalent), 3-amino-1-propanol (24.19 mg, 0.32 mmol, 1.0 equivalent), K₂CO₃ (89.02 mg, 0.64 mmol, 2.0 equivalent), and NaI (9.65 mg, 0.06 mmol, 0.2 equivalent) in DMF (5 mL) was stirred at 80°C for 48 hours. The mixture was concentrated and purified by preparative HPLC to obtain the target CPL-025 (66.75 mg, 29.93% yield) as a colorless oil.

LCMS: LC/MS (ESI) m/z: 892.16(M+H)⁺.

1H NMR (400 MHz, CDCl3) δ 5.62 (s, 2H), 4.08 (s, 4H), 3.79 (s, 2H), 2.64 (d, J = 3.7 Hz, 2H), 2.58 (s, 4H), 2.45 (s, 4H), 2.13 (s, 4H), 1.25 (s, 46H), 0.88 (s, 12H).

### Example 16: Preparation of ((3-hydroxypropyl)azanediyl)bis(butane-4,1-diyl) (2Z,2'Z)-bis(3-pentyltridec-2-enoate) (CPL-026)

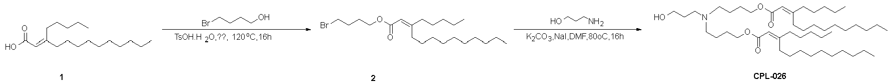

A mixture of (Z)-4-bromobutyl-3-pentyltridec-2-enoate (410 mg, 0.985 mmol, 2.1 equivalent), 3-amino-1-propanol (35.78 mg, 0.47 mmol, 1.0 equivalent), K₂CO₃ (259.44 mg, 1.88 mmol, 4.0 equivalent) and NaI (35.22 mg, 0.235 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-026 (111.03 mg, 31.6% yield) as a colorless oil.

LCMS: LCMS (ESI) m/z: 748.67 (M+H)⁺.

1H NMR (400 MHz, Chloroform-d) δ 5.61 (d, J = 1.3 Hz, 2H), 4.08 (t, J = 6.3 Hz, 4H), 3.79 (t, J = 5.2 Hz, 2H), 2.65 (s, 2H), 2.61- 2.55 (m, 4H), 2.46 (s, 4H), 2.13 (m, 4H), 1.66 (m, 4H), 1.57 (d, J = 7.0 Hz, 4H), 1.44 (t, J = 7.1 Hz, 6H), 1.35 - 1.23 (m, 40H), 0.90 - 0.86 (m, 12H).

### Example 17: Preparation of ((3-hydroxypropyl)azanediyl)bis(butane-4,1-diyl) (2E,2'E)-bis(3-hexyldec-2-enoate) (CPL-031)

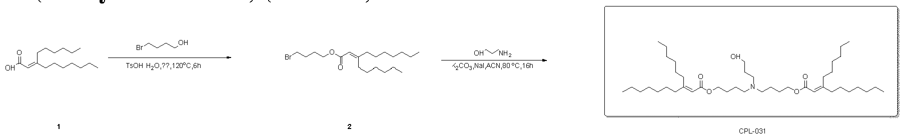

A mixture of (Z)-4-bromobutyl-3-hexyldec-2-enoate (180 mg, 0.4 mmol, 2.1 equivalent), 3-amino-1-propanol (15 mg, 0.23 mmol, 1.0 equivalent), K₂CO₃ (110.5 mg, 0.8 mmol, 4.0 equivalent) and NaI (15 mg, 0.1 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-031 (49.42 mg, 9% yield) as a colorless oil.

LCMS (ESI) m/z:692.12 (M+H)⁺.

1H NMR (400 MHz, CDCl3) δ 5.61 (s, 2H), 4.09 (t, J = 6.4 Hz, 4H), 3.84 - 3.73 (m, 2H), 2.72 - 2.54 (m, 6H), 2.46 (s, 4H), 2.15 - 2.08 (m, 4H), 1.65 (s, 4H), 1.31 - 1.24 (m, 42H), 0.94-0.87 (m, 12H).

### Example 18: Preparation of ((3-hydroxypropyl)azanediyl)bis(butane-4,1-diyl)(2Z,2'Z)-bis(3-heptylundec-2-enoate) (CPL-032)

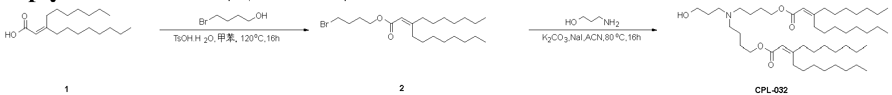

A mixture of (Z)-4-bromobutyl-3-heptylundecyl-2-enoate (230 mg, 0.553 mmol, 2.1 equivalent), 3-amino-1-propanol (19.74 mg, 0.236 mmol, 1.0 equivalent), K₂CO₃ (145.59 mg, 1.052 mmol, 4.0 equivalent) and NaI (19.59 mg, 0.131 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-032 (79.86 mg, 40.6% yield) as a colorless oil.

LCMS (ESI) m/z: 748.67 (M+H)⁺.

1H NMR (400 MHz, Chloroform-d) δ 5.61 (s, 2H), 4.13 - 4.06 (m, 4H), 3.81 (t, J = 5.3 Hz, 2H), 3.18 - 2.41 (m, 10H), 2.18 - 2.09 (m, 4H), 1.58 - 1.42 (m, 10H), 1.29 (t, J = 6.7 Hz, 44H), 0.88 (m, 12H).

### Example 19: Preparation of ((3-hydroxypropyl)azanediyl)bis(pentane-5,1-diyl)(2E,2'E)-bis(3-propylundec-2-enoate) (CPL-039)

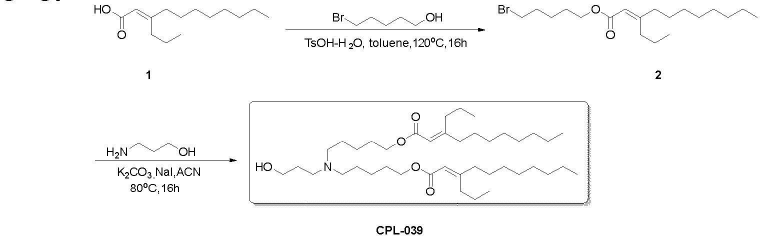

A mixture of (E)-5-bromopentyl-3-propylundec-2-enoate (313 mg, 0.834 mmol, 2.4 equivalent), 3-amino-1-propanol (26 mg, 0.347 mmol, 1.0 equivalent), K₂CO₃ (191 mg, 1.388 mmol, 4.0 equivalent), and NaI (5.2 mg, 0.0347 mmol, 0.1 equivalent) in ACN (8 mL) was stirred at 80°C for 16 hours. The mixture was concentrated and purified by preparative HPLC to obtain the target CPL-039 (72 mg, 31.3% yield) as a colorless oil.

LCMS: LC/MS (ESI) m/z: 664.5 (M+H)⁺.

1H NMR (400 MHz, CDCL3) δ 5.62 (d, J = 5.2 Hz, 2H), 4.09 (t, J = 6.8 Hz, 4H), 3.80 (t, J = 5.2 Hz, 2H), 2.64 (t, J = 5.2 Hz, 2H), 2.59-2.55 (m, 4H), 2.44-2.40 (m, 4H), 2.14-2.09 (m, 4H), 1.70-1.62 (m, 6H), 1.54-1.41 (m, 10H), 1.37-1.26 (m, 26H), 0.96-0.86 (m, 12H).

### Example 20: Preparation of ((3-hydroxypropyl)azanediyl)bis(pentane-5,1-diyl)(2E,2'E)-bis(3-butylundec-2-enoate) (CPL-043)

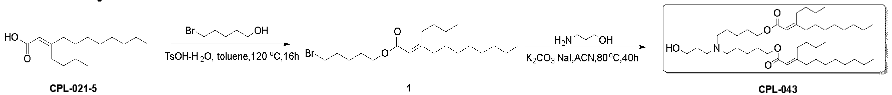

A mixture of (E)-5-bromopentyl-3-butylundec-2-enoate (379 mg, 0.973 mmol, 2.4 equivalent), 3-amino-1-propanol (30 mg, 0.406 mmol 1.0 equivalent), K₂CO₃ (224 mg, 1.624 mmol, 4.0 equivalent), and NaI (30 mg, 0.203 mmol, 0.5 equivalent) in ACN (4 mL) was stirred at 80°C for 40 hours. The mixture was concentrated and purified by preparative HPLC to obtain the target CPL-043 (99 mg, 35.9% yield) as a colorless oil.

LCMS: LC/MS (ESI) m/z: 691.61 (M+H)⁺.

1H NMR (400 MHz, CDCL3) δ 5.62 (s, 1H), 4.09-4.06 (m, 4H), 3.80-3.78 (m, 2H), 2.66-2.56 (m, 6H), 2.44-2.41 (m, 4H), 2.15-2.12 (m, 4H), 1.79-1.63 (m, 6H), 1.56-1.19 (m, 41H), 0.96-0.83 (m, 12H).

### Example 21: Preparation of ((3-hydroxypropyl)azanediyl)bis(pentane-5,1-diyl)(2E,2'E)-bis(3-butyltridec-2-enoate) (CPL-044)

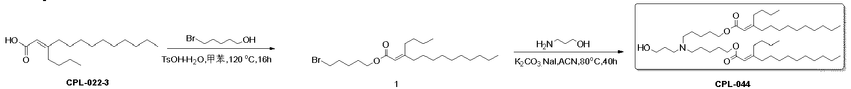

A mixture of (E)-5-bromopentyl-3-butyldodec-2-enoate (350 mg, 0.838 mmol, 2.4 equivalent), 3-amino-1-propanol (26 mg, 0.349 mmol, 1.0 equivalent), K₂CO₃ (193 mg, 1.397 mmol, 4.0 equivalent), and NaI (26 mg, 0.175 mmol, 0.5 equivalent) in ACN (4 mL) was stirred at 80°C for 40 hours. The mixture was concentrated and purified by preparative HPLC to obtain the target CPL-044 (94.27 mg, 36.1% yield) as a colorless oil.

LCMS: LC/MS (ESI) m/z: 747.67 (M+H)⁺.

1H NMR (400 MHz, CDCL3) δ 5.61 (s, 2H), 4.09-4.05 (m, 4H), 3.80-3.77 (m, 2H), 2.64-2.63 (m, 2H), 2.58-2.53 (m, 4H), 2.52-2.43 (m, 4H), 2.13-2.04 (m, 4H), 1.68-1.65 (m, 6H), 1.35-1.26 (m, 49H), 0.93-0.79 (m, 12H).

### Example 22: Preparation of ((3-hydroxypropyl)azanediyl)bis(hexane-6,1-diyl) (2E,2'E)-bis(3-pentylundec-2-enoate) (CPL-047)

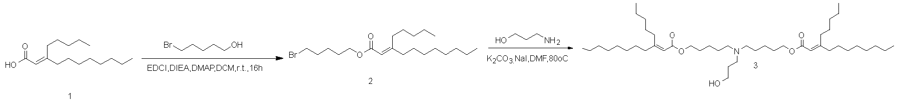

A mixture of (E)-5-bromopentyl-3-pentylundec-2-enoate (570 mg, 1.4 mmol, 2.2 equivalent), 3-amino-1-propanol (45 mg, 0.6 mmol, 1.0 equivalent), K₂CO₃ (333 mg, 1.31 mmol, 2 equivalent), and NaI (44 mg, 0.02 mmol, 0.2 equivalent) in DMF (5 mL) was stirred at 80°C for 40 hours. The mixture was concentrated and purified by preparative HPLC to obtain the target CPL-047 (23.12 mg, 2% yield) as a colorless oil.

LCMS: LC/MS (ESI) m/z:720.1(M+H)⁺.

1H NMR (400 MHz, CDCl3) δ 5.61 (s, 2H), 4.08 (t, J = 6.4 Hz, 4H), 3.78 (d, J = 5.5 Hz, 3H), 3.21 (s, 2H), 3.03 (s, 4H), 2.66 - 2.56 (m, 4H), 2.20 - 2.10 (m, 4H), 1.99 (d, J = 35.8 Hz, 4H), 1.62 - 1.25 (m, 143H), 0.90 - 0.88 (m, 12H).

### Example 23: Preparation of ((3-hydroxypropyl)azanediyl)bis(pentane-5,1-diyl) (2E,2'E)-bis(3-pentyltridec-2-enoate) (CPL-048)

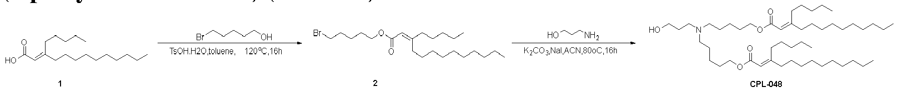

A mixture of (E)-5-bromopentyl-3-pentyltridec-2-enoate (210 mg, 0.488 mmol, 2.1 equivalent), 3-amino-1-propanol (17.44 mg, 0.232 mmol, 1.0 equivalent), K₂CO₃ (325.82 mg, 0.928 mmol, 4.0 equivalent) and NaI (17.38 mg, 0.116 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-048 (67.42 mg, 37.4% yield) as a colorless oil.

LCMS (ESI) m/z: 776.71 (M+H)⁺.

1H NMR (400 MHz, Chloroform-d) δ 5.61 (s, 2H), 4.07 (t, J = 6.7 Hz, 4H), 3.79 (t, J = 5.1 Hz, 2H), 2.64 (s, 2H), 2.60 - 2.53 (m, 4H), 2.46 - 2.38 (m, 4H), 2.16 - 2.09 (m, 4H), 1.66 (s, 4H), 1.49 - 1.39 (m, 10H), 1.35 - 1.23 (m, 44H), 0.90 - 0.86 (m, 12H).

### Example 24: Preparation of ((3-hydroxypropyl)azanediyl)bis(pentane-5,1-diyl)(2E,2'E)-bis(3-hexylnon-2-enoate) (CPL-049)

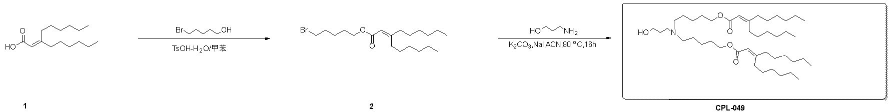

A mixture of (E)-6-bromopentyl-3-hexylnon-2-enoate (480 mg, 1.24 mmol, 2.4 equivalent), 3-amino-1-propanol (39 mg, 0.51 mmol, 1.0 equivalent), K₂CO₃ (285 mg, 2.06 mmol, 4.0 equivalent), and NaI (9 mg, 0.1 mmol, 0.2 equivalent) in ACN (10 mL) was stirred at 80°C for 16 hours. The mixture was concentrated and purified by preparative HPLC to obtain the target CPL-049 (78 mg, 21% yield) as a colorless oil.

LC/MS (ESI) m/z: 692.7 (M+H)⁺.

1H NMR (400 MHz, CDCl3) δ 5.61 (s, 2H), 4.17 - 4.00 (m, 4H), 3.94 - 3.60 (m, 2H), 3.11- 2.85 (m, 2H), 2.86 - 2.62 (m, 4H), 2.62 - 2.51 (m, 4H), 2.20 - 2.09 (m, 4H), 1.94 - 1.79 (m, 2H), 1.73 - 1.33 (m, 26H), 0.97 - 0.81 (m, 12H).

### Example 25: Preparation of ((3-hydroxypropyl)azanediyl)bis(pentane-5,1-diyl) (2E,2'E)-bis(3-butyldec-2-enoate) (CPL-052)

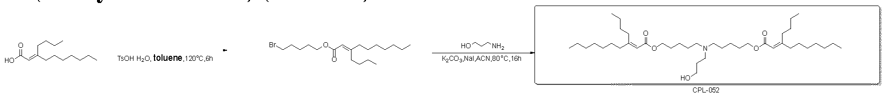

A mixture of (E)-5-bromopentyl-3-butyldec-2-enoate (420 mg, 1.12 mmol, 2.1 equivalent), 3-amino-1-propanol (38.2 mg, 0.48 mmol, 1.0 equivalent), K₂CO₃ (280.6 mg, 1.9 mmol, 4.0 equivalent) and NaI (45 mg, 0.24 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-052 (93.01 mg, 12% yield) as a colorless oil.

LCMS (ESI) m/z:664 (M+H)⁺.

1H NMR (400 MHz, CDCl3) δ 5.61 (s, 2H), 4.07 (t, J = 6.8 Hz, 4H), 3.86 - 3.74 (m, 2H), 2.69 - 2.52 (m, 6H), 2.45 - 2.36 (m, 4H), 2.12 (d, J = 6.8 Hz, 4H), 1.52 - 1.24 (m, 22H), 0.94 - 0.87 (m, 12H).

### Example 26: Preparation of ((3-hydroxypropyl)azanediyl)bis(hexane-6,1-diyl) (2E,2'E)-bis(3-propylundec-2-enoate) (CPL-061)

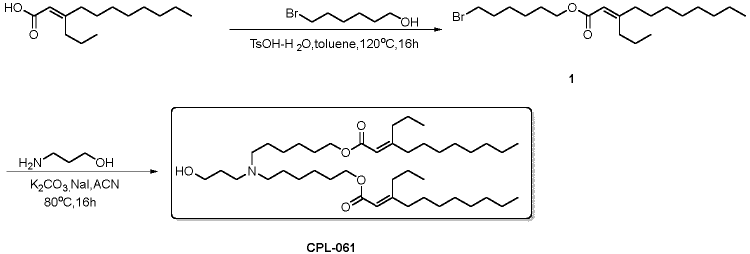

A mixture of (E)-6-bromohexyl-3-propylundec-2-enoate (440 mg, 1.13 mmol, 2.4 eq), 3-amino-1-propanol (35 mg, 0.47 mmol, 1.0 eq), K₂CO₃ (259 mg, 1.88 mmol, 4.0 eq) and NaI (7 mg, 0.047 mmol, 0.1 eq) in ACN (6 mL) was stirred at 80°C for 16 hours. The mixture was concentrated and purified by preparative HPLC to obtain the target CPL-061 (105 mg, 32.3% yield) as a colorless oil.

LCMS: LC/MS (ESI) m/z: 692.6 (M+H)⁺.

1H NMR (400 MHz, CDCL3) δ 5.62 (d, J = 5.2 Hz, 2H), 4.08 (t, J = 6.8 Hz, 4H), 3.80 (t, J = 5.2 Hz, 2H), 2.64 (t, J = 5.2 Hz, 2H), 2.59-2.55 (m, 4H), 2.41-2.38 (m, 4H), 2.14-2.09 (m, 4H), 1.69-1.61 (m, 6H), 1.52-1.27 (m, 40H), 0.96-0.86 (m, 12H).

### Example 27: Preparation of ((3-hydroxypropyl)azanediyl)bis(hexane-6,1-diyl) (2E,2'E)-bis(3-butylundec-2-enoate) (CPL-065)

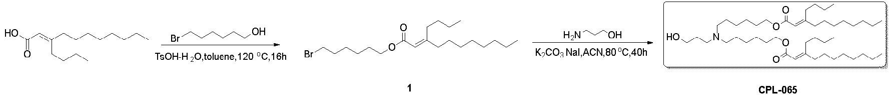

A mixture of (E)-6-bromohexyl-3-butylundec-2-enoate (401 mg, 0.997 mmol, 2.4 eq), 3-amino-1-propanol (31 mg, 0.415 mmol, 1.0 eq), K₂CO₃ (229 mg, 1.66 mmol, 4.0 eq) and NaI (31 mg, 0.208 mmol, 0.5 eq) in ACN (4 mL) was stirred at 80°C for 40 h. The mixture was concentrated and purified by preparative HPLC to obtain the target CPL-065 (102 mg, 34.3% yield) as a colorless oil.

LCMS: LC/MS (ESI) m/z: 719.64 (M+H)⁺.

1H NMR (400 MHz, CDCL3) δ 5.61 (s, 1H), 4.08-4.05 (m, 4H), 3.81-3.78 (m, 2H), 2.64-2.56 (m, 6H), 2.43-2.39 (m, 4H), 2.15-2.11 (m, 4H), 1.69-1.61 (m, 6H), 1.50-1.19 (m, 45H), 0.94-0.87 (m, 12H).

### Example 28: Preparation of ((3-hydroxypropyl)azanediyl)bis(hexane-6,1-diyl) (2E,2'E)-bis(3-butyl-2-tridecenoate) (CPL-066)

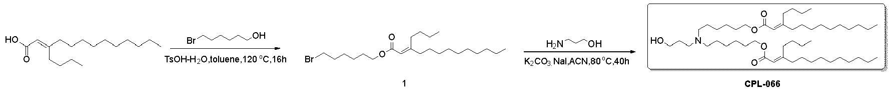

A mixture of (E)-6-bromohexyl-3-butyltridec-2-enoate (408 mg, 0.946 mmol, 2.4 eq), 3-amino-1-propanol (30 mg, 0.394 mmol, 1.0 eq), K₂CO₃ (218 mg, 1.576 mmol, 4.0 eq) and NaI (30 mg, 0.197 mmol, 0.5 eq) in ACN (4 mL) was stirred at 80°C for 40 h. The mixture was concentrated and purified by preparative HPLC to obtain the target CPL-066 (126.09 mg, 41.22% yield) as a colorless oil.

LCMS: LC/MS (ESI) m/z: 776.29 (M+H)⁺.

1H NMR (400 MHz, CDCL3) δ 5.61 (s, 2H), 4.09-4.05 (m, 4H), 3.80-3.77 (m, 2H), 2.64-2.63 (m, 2H), 2.58-2.53 (m, 4H), 2.52-2.43 (m, 4H), 2.13-2.04 (m, 4H), 1.68-1.65 (m, 6H), 1.35-1.26 (m, 53H), 0.93-0.79 (m, 12H).

### Example 29: Preparation of ((3-hydroxypropyl)azanediyl)bis(hexane-6,1-diyl) (2Z,2'Z)-bis(3-pentyltridec-2-enoate) (CPL-070)

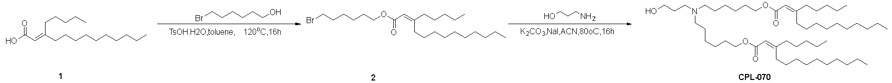

A mixture of (E)-6-bromohexyl-3-pentyltridec-2-enoate (430 mg, 0.968 mmol, 2.1 equivalent), 3-amino-1-propanol (34.57 mg, 0.461 mmol, 1.0 equivalent), K₂CO₃ (254.47 mg, 1.84 mmol, 4.0 equivalent) and NaI (34.5 mg, 0.230 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-070 (90.91 mg, 24.32% yield) as a colorless oil.

LCMS (ESI) m/z: 804.74 (M+H)⁺.

1H NMR (400 MHz, Chloroform-d) δ 5.61 (s, 2H), 4.07 (t, J = 6.7 Hz, 4H), 3.79 (t, J = 5.1 Hz, 2H), 2.64 (s, 2H), 2.61 - 2.54 (m, 4H), 2.41 (d, J = 8.6 Hz, 4H), 2.12 (t, J = 7.7 Hz, 4H), 1.64 (d, J = 7.7 Hz, 4H), 1.46 - 1.37 (m, 10H), 1.34 - 1.22 (m, 48H), 0.89 (m, 12H).

### Example 30: Preparation of ((3-hydroxypropyl)azanediyl)bis(hexane-6,1-diyl) (2E,2'E)-bis(3-hexylnon-2-enoate) (CPL-071)

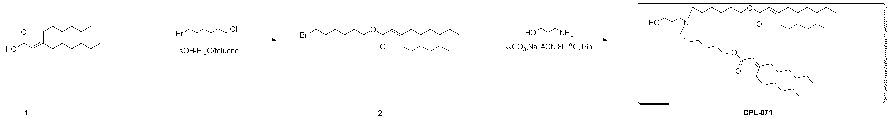

A mixture of (E)-6-bromohexyl-3-hexylnon-2-enoate (400 mg, 0.99 mmol, 2.4 equivalent), 3-amino-1-propanol (31 mg, 0.41 mmol, 1.0 equivalent), K₂CO₃ (229 mg, 1.576 mmol, 4.0 equivalent), and NaI (13 mg, 0.08 mmol, 0.2 equivalent) in ACN (10 mL) was stirred at 80°C for 16 hours. The mixture was concentrated and purified by preparative HPLC to obtain the target CPL-071 (101 mg, 34% yield) as a colorless oil.

LC/MS (ESI) m/z: 720.7 (M+H)⁺.

1H NMR (400 MHz, CDCl3) δ 5.61 (s, 2H), 4.19 - 3.97 (m, 4H), 3.93 - 3.71 (m, 2H), 3.04 - 2.87 (m, 2H), 2.85 - 2.64 (m, 4H), 2.64 - 2.49 (m, 4H), 2.20 - 2.08 (m, 4H), 1.92 - 1.79 (m, 2H), 1.77 - 1.35 (m, 48H), 0.97 - 0.78 (m, 12H).

### Example 31: Preparation of ((3-hydroxypropyl)azanediyl)bis(hexane-6,1-diyl) (2E,2'E)-bis(3-butyldec-2-enoate)) (CPL-074)

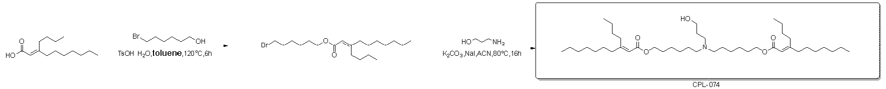

A mixture of (E)-6-bromohexyl-3-butyldec-2-enoate (387 mg, 0.95 mmol, 2.1 equivalent), 3-amino-1-propanol (36 mg, 0.48 mmol, 1.0 equivalent), K₂CO₃ (263 mg, 1.9 mmol, 4.0 equivalent) and NaI (36 mg, 0.24 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-074 (86.55 mg, 13% yield) as a colorless oil.

LCMS: LCMS (ESI) m/z:692.12 (M+H)⁺.

1H NMR (400 MHz, CDCl3) δ 5.61 (s, 2H), 4.07 (t, J = 6.7 Hz, 4H), 3.86 - 3.74 (m, 2H), 2.69 - 2.52 (m, 6H), 2.45 - 2.36 (m, 4H), 2.12 (d, J = 6.8 Hz, 4H), 1.52 - 1.24 (m, 51H), 0.94 - 0.87 (m, 12H).

### Example 32: Preparation of ((3-hydroxypropyl)azanediyl)bis(hexane-6,1-diyl) (2E,2'E)-bis(3-octyltridec-2-enoate) (CPL-079)

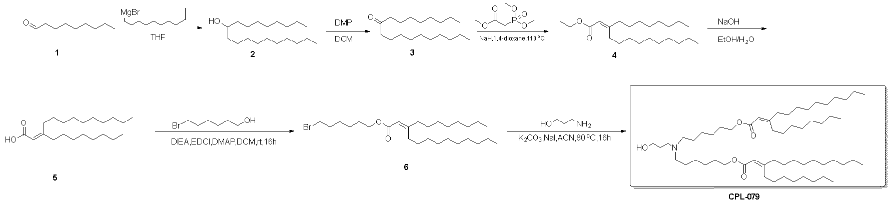

A mixture of (E)-6-bromohexyl-3-octyltridec-2-enoate (420 mg, 0.8642 mmol, 2.3 equivalent), 3-amino-1-propanol (28 mg, 0.3757 mmol, 1.0 equivalent), K₂CO₃ (156 mg, 1.1271 mmol, 3.0 equivalent), and Cs₂CO₃ (61 mg, 0.1879 mmol, 0.5 equivalent) in ACN (8 mL) was stirred at 90°C for 24 hours. 40 mL of water was added and extracted three times with 20 mL of ethyl acetate. The organic phase was washed three times with 40 mL of sodium chloride solution, filtered and dried over sodium sulfate. The mixture was concentrated and purified by preparative HPLC to obtain the target CPL-079 (76.07 mg, 10% yield) as a colorless oil.

LCMS: LC/MS (ESI) m/z: 888.9 (M+H)⁺.

1HNMR(400 MHz, CDCl3) δ 5.54 (s, 2H), 4.00 (t,J = 6.7 Hz, 4H), 3.71 (dd,J = 10.5, 5.4 Hz, 2H), 2.52 (dd,J = 19.0, 11.4 Hz, 6H), 2.34 (s, 2H), 2.10 - 2.02 (m, 4H), 1.98 - 1.93 (m, 1H), 1.59 (dd,J = 13.9, 7.0 Hz, 6H), 1.40 - 1.36 (m, 6H), 1.19 (s, 64H), 0.81 (dd,J = 6.9, 5.6 Hz, 12H).

### Example 33: Preparation of ((3-hydroxypropyl)azanediyl)bis(heptane-7,1-diyl) (2E,2'E)-bis(3-pentylnon-2-enoate) (CPL-090)

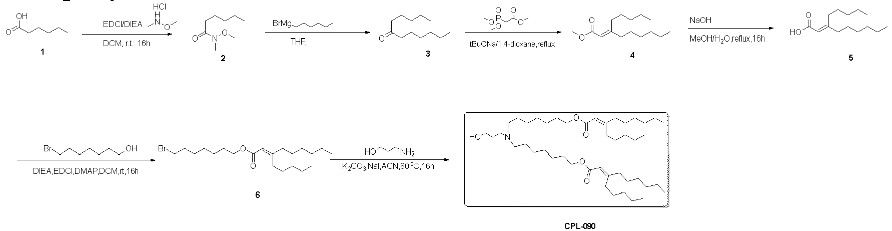

A mixture of (E)-6-bromoheptyl-3-pentylnon-2-enoate (565 mg, 1.4055 mmol, 2.3 equivalent), 3-amino-1-propanol (45 mg, 0.6111 mmol, 1.0 equivalent), K₂CO₃ (253 mg, 1.833 mmol, 3.0 equivalent), Cs₂CO₃ (99 mg, 0.3056 mmol, 0.5 equivalent) and NaI (45 mg, 0.3056 mmol, 0.5 equivalent) in ACN (8 mL) was stirred at 80°C for 48 hours. 40 mL of water was added and extracted three times with 20 mL of ethyl acetate. The organic phase was washed three times with 40 mL of sodium chloride solution, filtered and dried over sodium sulfate. The mixture was concentrated and purified by preparative HPLC to obtain the target CPL-090 (148.8 mg, 15% yield) as a colorless oil.

LCMS: LC/MS (ESI) m/z: 720.7 (M+H)⁺.

1HNMR(400 MHz, CDCl3) δ 5.54 (s, 2H), 4.00 (t,J = 6.7 Hz, 4H), 3.75 - 3.70 (m, 2H), 2.52 (dd,J = 20.2, 12.6 Hz, 6H), 2.41 - 2.27 (m, 4H), 2.10 - 2.02 (m, 4H), 1.58 (dd,J = 13.9, 7.2 Hz, 6H), 1.38 (d,J = 7.0 Hz, 8H), 1.23 (ddd,J = 18.4, 10.2, 5.8 Hz, 36H), 0.84 - 0.80 (m, 12H).

### Example 34: Preparation of ((3-hydroxypropyl)azanediyl)bis(heptane-7,1-diyl)(2E,2'E)-bis(3-butyldec-2-enoate) (CPL-096)

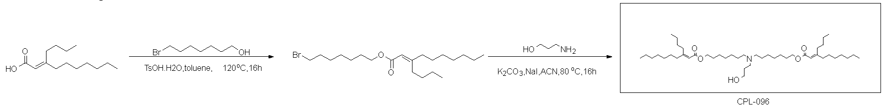

A mixture of (E)-6-bromoheptyl-3-butyldec-2-enoate (421 mg, 0.95 mmol, 2.1 equivalent), 3-amino-1-propanol (33 mg, 0.44 mmol, 1.0 equivalent), K₂CO₃ (235 mg, 1.7 mmol, 4.0 equivalent) and NaI (32 mg, 0.22 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-096 (101.27 mg, 5% yield) as a colorless oil.
LCMS: LCMS (ESI) m/z:720.18 (M+H)⁺.
1H NMR (400 MHz, CDCl3) δ 5.63 (d, J = 9.3 Hz, 2H), 4.07 (s, 4H), 3.81 (s, 2H), 2.59 (s, 8H), 2.41 (s, 5H), 2.13 (s, 6H), 1.66 (s, 10H), 1.28 (s, 50H), 0.90 (s, 12H)

### Example 35: Preparation of ((3-hydroxypropyl)azanediyl)bis(heptane-7,1-diyl)(2Z,2'Z)-bis(3-octyltridec-2-enoate) (CPL-101)

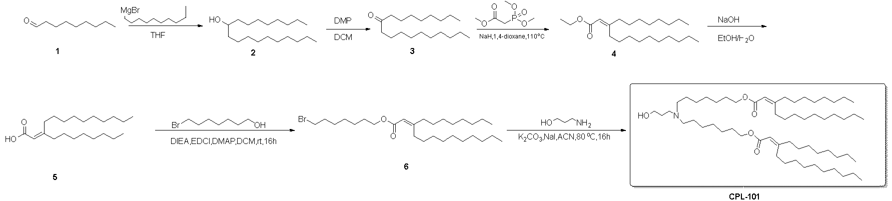

A mixture of (Z)-6-bromoheptyl-3-octyltridec-2-enoate (400 mg, 0.80 mmol, 2.3 equivalent), 3-amino-1-propanol (26 mg, 0.3478 mmol, 1.0 equivalent), K₂CO₃ (144 mg, 1.0434 mmol, 3.0 equivalent), Cs₂CO₃ (57 mg, 0.1739 mmol, 0.5 equivalent) and NaI (26 mg, 0.1739 mmol, 0.5 equivalent) in ACN (6 mL) was stirred at 85°C for 48 hours. 40 mL of water was added and extracted three times with 20 mL of ethyl acetate. The organic phase was washed three times with 40 mL of sodium chloride solution, filtered and dried over sodium sulfate. The mixture was concentrated and purified by preparative HPLC to obtain the target CPL-101 (145.15 mg, 20% yield) as a colorless oil.

LCMS: LC/MS (ESI) m/z: 916.7 (M+H)⁺.

1HNMR(400 MHz, CDCl3) δ 5.54 (s, 2H), 4.01 - 3.97 (m, 4H), 3.77 - 3.74 (m, 2H), 2.96 (s, 2H), 2.90 (s, 1H), 2.53 - 2.49 (m, 2H), 1.99 (ddd,J = 31.2, 16.6, 9.0 Hz, 10H), 1.37 (dd,J = 3.4, 1.3 Hz, 2H), 1.29 (s, 6H), 1.21 (d,J = 14.6 Hz, 70H), 0.82 (d,J = 6.5 Hz, 12H).

### Example 36: Preparation of ((3-hydroxypropyl)azanediyl)bis(octane-8,1-diyl)(2E,2'E)-bis(3-pentylnon-2-enoate) (CPL-112)

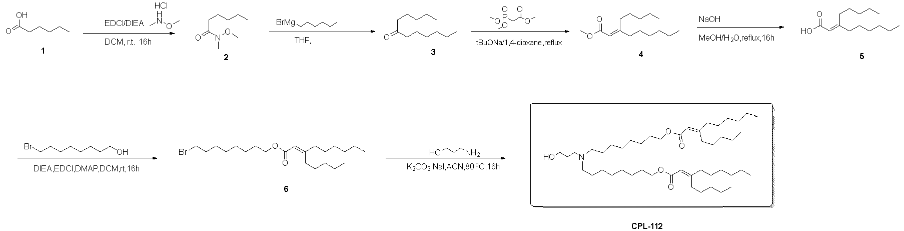

A mixture of (E)-8-bromooctanyl-3-pentylnon-2-enoate (400 mg, 0.9615 mmol, 2.3 equivalent), 3-amino-1-propanol (31 mg, 0.4181 mmol, 1.0 equivalent), K₂CO₃ (173 mg, 1.2543 mmol, 3.0 equivalent), Cs₂CO₃ (68 mg, 0.2091 mmol, 0.5 equivalent) and NaI (31 mg, 0.2091 mmol, 0.5 equivalent) in ACN (8 mL) was stirred at 85°C for 48 hours. 40 mL of water was added and extracted three times with 20 mL of ethyl acetate. The organic phase was washed three times with 40 mL of sodium chloride solution, filtered and dried over sodium sulfate. The mixture was concentrated and purified by preparative HPLC to obtain the target CPL-112 (118.53 mg, 17% yield) as a colorless oil.

LC/MS (ESI) m/z: 748.7 (M+H)⁺.

1HNMR(400 MHz, CDCl3) δ 5.54 (s, 2H), 4.00 (t,J = 6.7 Hz, 4H), 3.77 - 3.66 (m, 2H), 2.52 (dd,J = 20.6, 13.0 Hz, 6H), 2.34 (s, 2H), 2.10 - 2.02 (m, 4H), 1.61- 1.53 (m, 8H), 1.38 (d,J = 5.1 Hz, 8H), 1.30 - 1.17 (m, 40H), 0.85 - 0.80 (m, 12H).

### Example 37: Preparation of ((3-hydroxypropyl)azanediyl)bis(nonane-9,1-diyl) (2E,2'E)-bis(3-propylundec-2-enoate) (CPL-127)

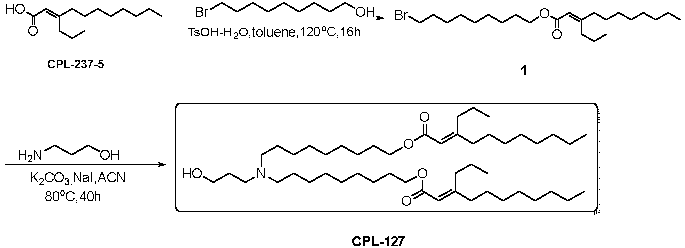

A mixture of (E)-9-bromononyl-3-propylundec-2-enoate (514 mg, 1.193 mmol, 2.4 equivalent), 3-amino-1-propanol (37 mg, 0.497 mmol, 1.0 equivalent), K₂CO₃ (275 mg, 1.988 mmol, 4.0 equivalent) and NaI (37 mg, 0.249 mmol, 0.5 equivalent) in ACN (4 mL) was stirred at 80°C for 40 hours. The mixture was concentrated and purified by preparative HPLC to obtain the target CPL-127 (200.08 mg, 51.89% yield) as a colorless oil.

LCMS: LC/MS (ESI) m/z: 775.71 (M+H)⁺.

1H NMR (400 MHz, CDCL3) δ 5.62-5.61 (m, 2H), 4.09-4.05 (m, 4H), 3.80 (t, J = 5.2 Hz, 2H), 2.70-2.66 (m, 2H), 2.59-2.54 (m, 4H), 2.55-2.40 (m, 4H), 2.14-2.09 (m, 4H), 1.68-1.65 (m, 6H), 1.35-1.26 (m, 53H), 0.96-0.86 (m, 12H).

### Example 38: Preparation of 9-((3-hydroxypropyl)(9-(E)-3-pentyltridecyl-2-enoyl)oxy)nonyl)amino)nonyl (Z)-3-pentyltridec-2-enoate (CPE-136)

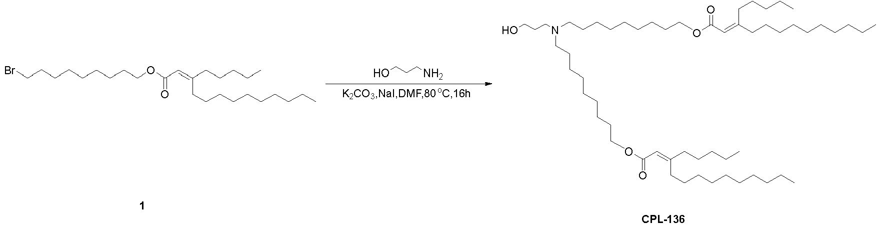

A mixture of (E)-9-bromononyl-3-pentyltridec-2-enoate (600 mg, 1.23 mmol, 2.1 equivalent), 3-amino-1-propanol (44 mg, 0.587 mmol, 1.0 equivalent), K₂CO₃ (324.02 mg, 2.348 mmol, 4.0 equivalent) and NaI (43.92 mg, 0.293 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-136 (269.34 mg, 51.63% yield) as a colorless oil.

LCMS (ESI) m/z: 888.83 (M+H)⁺.

1H NMR (400 MHz, Chloroform-d) δ 5.61 (s, 2H), 4.07 (t, J = 6.8 Hz, 4H), 3.79 (t, J = 5.1 Hz, 2H), 2.70 - 2.61 (m, 2H), 2.61 - 2.52 (m, 4H), 2.46 - 2.36 (m, 4H), 2.12 (t, J = 7.7 Hz, 4H), 2.09 - 1.99 (m, 2H), 1.65 - 1.60 (m, 4H), 1.47 - 1.43 (m, 6H), 1.34 - 1.24 (m, 62H), 0.89 (d, J = 6.2 Hz, 12H).

### Example 39: Preparation of ((3-hydroxypropyl)azanediyl)bis(decane-10,1-diyl) (2E,2'E)-bis(3-propylnon-2-enoate) (CPL-148)

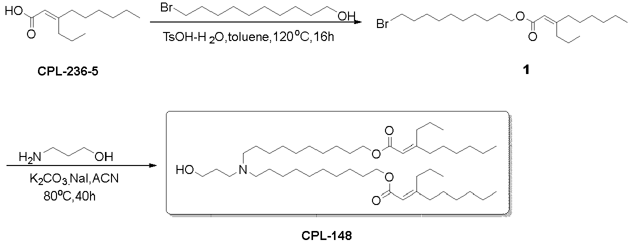

A mixture of (E)-10-bromodecyl-3-propanenon-2-enoate (611 mg, 1.469 mmol, 2.4 equivalent), 3-amino-1-propanol (46 mg, 0.306 mmol, 0.5 equivalent), K₂CO₃ (338 mg, 2.448 mmol, 4.0 equivalent) and NaI (46 mg, 0.306 mmol, 0.5 equivalent) in ACN (4 mL) was stirred at 80°C for 40 h. The mixture was concentrated and purified by preparative HPLC to obtain the target CPL-148 (151.61 mg, 33.18% yield) as a colorless oil.

LCMS: LC/MS (ESI) m/z: 747.67 (M+H)⁺.

1H NMR (400 MHz, CDCL3) δ 5.62-5.61 (m, 2H), 4.09-4.05 (m, 4H), 3.80 (t, J = 5.2 Hz, 2H), 2.70-2.66 (m, 2H), 2.59-2.54 (m, 4H), 2.55-2.40 (m, 4H), 2.14-2.09 (m, 4H), 1.68-1.65 (m, 6H), 1.35-1.26 (m, 49H), 0.96-0.86 (m, 12H).

### Example 40: Preparation of ((3-hydroxypropyl)azanediyl)bis(decane-10,1-diyl) (2Z,2'Z)-bis(3-butylnon-2-enoate) CPL-152

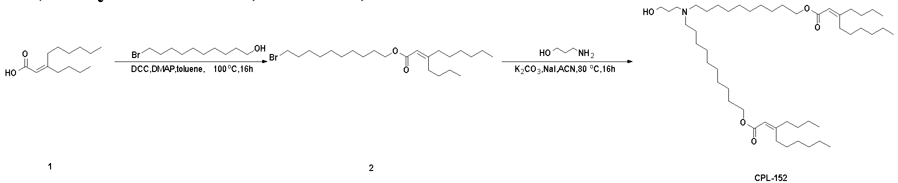

A mixture of (E)- 10-bromodecyl-3-butylnon-2-enoate (215 mg, 0.5 mmol, 2.1 equivalent), 3-amino-1-propanol (17.86 mg, 0.238 mmol, 1.0 equivalent), K₂CO₃ (131.38 mg, 0.952 mmol, 4.0 equivalent) and NaI (17.84 mg, 0.119 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-152 (103.64 mg, 56.13% yield) as a colorless oil.

LCMS (ESI) m/z: 776.71 (M+H)⁺.

1H NMR (400 MHz, Chloroform-d) δ 5.61 (s, 2H), 4.07 (t, J = 6.8 Hz, 4H), 3.83 (t, J = 5.3 Hz, 2H), 2.95 (d, J = 63.2 Hz, 4H), 2.58 (m, 4H), 2.17 - 2.09 (m, 4H), 1.91 (s, 2H), 1.62 (d, J = 7.2 Hz, 6H), 1.45 - 1.25 (m, 52H), 0.89 (m, 12H).

### Example 41: Preparation of ((3-hydroxypropyl)azanediyl)bis(undecane-11,1-diyl) (2E,2'E)-bis(3-butyldec-2-enoate) (CPL-184)

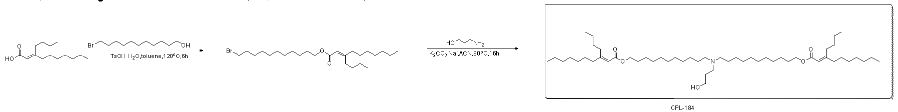

A mixture of (E)-11-bromoundecyl-3-butyldec-2-enoate (154 mg, 0.95 mmol, 2.1 equivalent), 3-amino-1-propanol (11.25 mg, 0.15 mmol, 1.0 equivalent), K₂CO₃ (82.8 mg, 0.6 mmol, 4.0 equivalent) and NaI (11 mg, 0.075 mmol, 0.5 equivalent) in ACN (5 mL) was stirred at 80°C for 20 hours. 30 mL of water was added and extracted three times with 20 mL of ethyl acetate. The organic phase was washed three times with 30 mL of sodium chloride solution, filtered and dried over sodium sulfate. The mixture was concentrated and purified by preparative HPLC to obtain the target CPL-184 (26.73 mg, 9% yield) as a colorless oil.

LCMS (ESI) m/z:832.39 (M+H)⁺.

1H NMR (400 MHz, CDCl3) δ 5.61 (s, 2H), 4.07 (t, J = 6.7 Hz, 4H), 3.86 - 3.74 (m, 2H), 2.69 - 2.52 (m, 6H), 2.45 - 2.36 (m, 4H), 2.12 (d, J = 6.8 Hz, 4H), 1.52 - 1.24 (m, 69H), 0.94 - 0.87 (m, 12H).

### Example 42: Preparation of 3-(((E)-3-butyloct-2-enoyl)oxy)-2-((((2-(dimethylamino)ethoxy)carbonyl)oxy)methyl)propyl (9Z,12Z)-octadeca-9,12-dienoate (CPL-199)

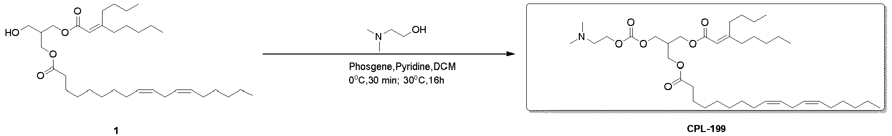

A mixture of 3-((E)-3-butyloct-2-enoate)-2-(hydroxymethyl)propyl-(9Z,12Z)-octadeca-9,12-dienoate (250 mg, 0.27 mmol, 1.0 equivalent), pyridine (216 mg, 2.73 mmol, 10.0 equivalent) and triphosgene (33 mg, 0.10 mmol, 0.4 equivalent) in dichloromethane (3 mL) was stirred at 0°C for 30 minutes. N,N-dimethylethanolamine (74 mg, 0.82 mmol, 3.0 equivalent) was added and stirred at 30°C for 16 hours. 10 mL of water was added and extracted three times with 10 mL of dichloromethane. The organic phase was washed three times with 20 mL of sodium chloride solution, filtered and dried over sodium sulfate. The mixture was concentrated and purified by preparative HPLC to obtain the target CPL-199 (62 mg, 34% yield) as a colorless oil.

LC/MS (ESI) m/z: 664.6 (M+H)⁺.

1H NMR (400 MHz, CDCl3) δ 5.61 (s, 1H), 5.46 - 5.19 (m, 4H), 4.33 - 3.98 (m, 8H), 2.83 - 2.69 (m, 2H), 2.68 - 2.53 (m, 4H), 2.50 - 2.38 (m, 1H), 2.36 - 2.29 (m, 4H), 2.28 (s, 6H), 2.17 - 2.10 (m, 2H), 2.09 - 1.99 (m, 4H), 1.50 - 1.23 (m, 26H), 0.99 - 0.84 (m, 9H).

### Example 43: Preparation of 3-((((2-(dimethylamino)ethoxy)carbonyl)oxy)-2-((((E)-3-hexylundec-2-enoyl)oxy)methyl)propyl (9Z,12Z)-octadeca-9,12-dienoate (CPL-203)

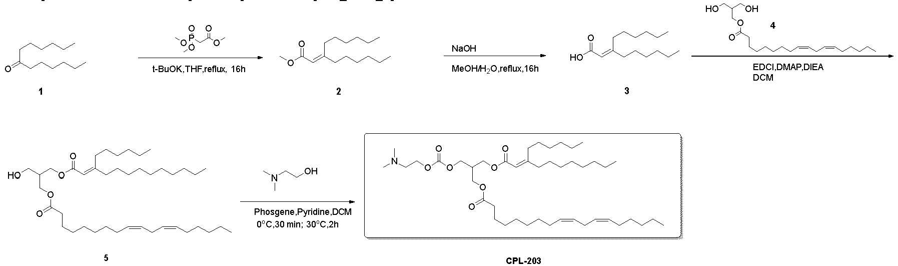

A mixture of 3-((E)-3-hexylundec-2-enoate)-2-(hydroxymethyl)propyl-(9Z,12Z)-octadeca-9,12-dienoate (200 mg, 0.339 mmol, 1.0 equivalent), pyridine (268 mg, 3.39 mmol, 10.0 equivalent) and triphosgene (41 mg, 0.136 mmol, 0.4 equivalent) in dichloromethane (3 mL) was stirred at 0°C for 30 minutes. N,N-dimethylethanolamine (61 mg, 0.678 mmol, 2.0 equivalent) was added and stirred at 30°C for 16 hours. 10 mL of water was added and extracted three times with 10 mL of dichloromethane. The organic phase was washed three times with 20 mL of sodium chloride solution, filtered and dried over sodium sulfate. The mixture was concentrated and purified by preparative HPLC to obtain the target CPL-203 (40 mg, 16.8% yield) as a colorless oil.

LC/MS (ESI) m/z: 706.6 (M+H)⁺.

1H NMR (400 MHz, CDCl3) δ 5.60 (s, 1H), 5.46 - 5.30 (m, 4H), 4.34 - 4.07 (m, 8H), 2.85 - 2.71 (m, 2H), 2.71 - 2.62 (m, 2H), 2.62 - 2.54 (m, 2H), 2.48 - 2.23 (m, 9H), 2.18 - 2.09 (m, 2H), 2.09 - 1.94 (m, 5H), 1.69 - 1.20 (m, 30H), 0.91 - 0.82 (m, 9H).

### Example 44: Preparation of 3-(((2-(dimethylamino)ethoxy)carbonyl)oxy)-2-((((E)-3-heptylundec-2-enoyl)oxy)methyl)propyl (9Z,12Z)-octadeca-9,12-dienoate (CPL-208)

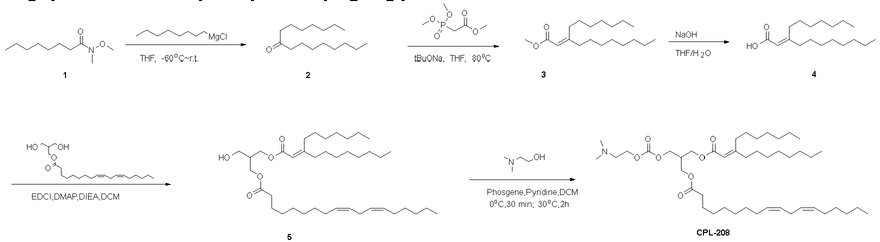

A mixture of 3-((E)-3-heptylundec-2-enoate)-2-(hydroxymethyl)propyl-(9Z,12Z)-octadeca-9,12-dienoate (200 mg, 0.316 mmol, 1.0 equivalent), pyridine (249.9 mg, 3.16 mmol, 10.0 equivalent) and triphosgene (38.57 mg, 0.13 mmol, 0.4 equivalent) in dichloromethane (5 mL) was stirred at 0°C for 30 minutes. N,N-dimethylpropanolamine (56.29 mg, 0.632 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-208 (28.01 mg, 11.84% yield) as a colorless oil.

LCMS (ESI) m/z: 748.6 (M+H)⁺.

1H NMR (400 MHz, Chloroform-d) δ 5.60 (s, 1H), 5.40 - 5.29 (m, 4H), 4.32 - 4.19 (m, 4H), 4.15 (m, 4H), 2.81 - 2.61 (m, 4H), 2.61 - 2.54 (m, 2H), 2.44 (m, 1H), 2.42 - 2.25 (m, 8H), 2.13 (t, J = 7.7 Hz, 2H), 2.04 (t, J = 6.9 Hz, 4H), 1.44 (d, J = 7.3 Hz, 2H), 1.38 - 1.25 (m, 36H), 0.91- 0.86 (m, 9H).

### Example 45: Preparation of 3-(((2-(dimethylamino)ethoxy)carbonyl)oxy)-2-(((3-octylundec-2-enoyl)oxy)methyl)propyl (9Z,12Z)-octadeca-9,12-dienoate (CPL-210)

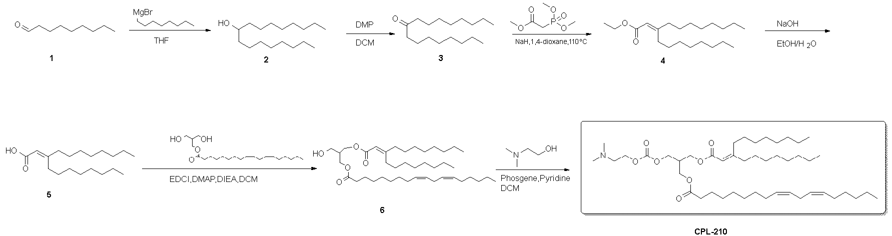

A mixture of 3-((E)-3-octylundecyl-2-enoate)-2-(hydroxymethyl)propyl-(9Z,12Z)-octadeca-9,12-dienoate (200 mg, 0.3096 mmol, 1.0 equivalent), N,N-dimethylpropanolamine (55 mg, 0.6192 mmol, 2.0 equivalent), pyridine (245 mg, 3.096 mmol, 10.0 equivalent) and triphosgene (37 mg, 0.1238 mmol, 0.4 equivalent) in dichloromethane (7 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-210 (25.97 mg, 11% yield) as a colorless oil.

LC/MS (ESI) m/z: 761.62 (M+H)⁺.

1H NMR (400 MHz, CDCl3) δ 5.60 (s, 1H), 5.43 - 5.29 (m, 4H), 4.25 - 4.18 (m, 4H), 4.17 - 4.10 (m, 4H), 2.77 (t, J = 6.4 Hz, 2H), 2.58 (dd, J = 15.0, 7.3 Hz, 3H), 2.48 - 2.31 (m, 2H), 2.29 (s, 6H), 2.12 (dd, J = 18.8, 10.9 Hz, 2H), 2.07 - 2.01 (m, 4H), 1.44 (dd, J = 14.6, 7.0 Hz, 4H), 1.36 - 1.23 (m, 38H), 0.90 - 0.86 (m, 9H).

### Example 46: Preparation of 3-(((Z)-3-butylnon-2-enoyl)oxy)-2-((((3-(dimethylamino)propoxy)carbonyl)oxy)methyl)propyl (9Z,12Z)-octadeca-9,12-dienoate (CPL-218)

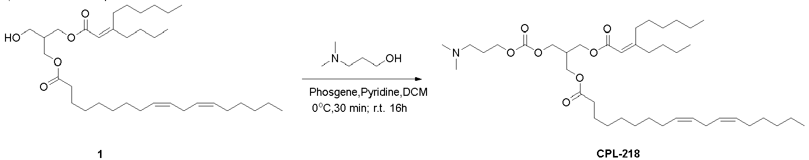

A mixture of 3-((E)-3-butylnon-2-enoate)-2-(hydroxymethyl)propyl-(9Z,12Z)-octadeca-9,12-dienoate (150 mg, 0.267 mmol, 1.0 equivalent), pyridine (212.2 mg, 2.67 mmol, 10.0 equivalent) and triphosgene (31.69 mg, 0.107 mmol, 0.4 equivalent) in dichloromethane (2 mL) was stirred at 0°C for 30 minutes. N,N-dimethylpropanolamine (54.95 mg, 0.533 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-218 (27.16 mg, 14.71% yield) as a colorless oil.

LCMS (ESI) m/z: 692.54 (M+H)⁺.

1H NMR (400 MHz, Chloroform-d) δ 5.61 (s, 1H), 5.42 - 5.29 (m, 4H), 4.25 - 4.12 (m, 8H), 2.77 (t, J = 6.5 Hz, 2H), 2.63 - 2.53 (m, 2H), 2.45 (m, 1H), 2.39 (t, J = 7.3 Hz, 2H), 2.31 (t, J = 7.6 Hz, 2H), 2.25 (s, 6H), 2.18 - 2.11 (m, 2H), 2.05 (m, 4H), 1.43 (m, 4H), 1.40 - 1.21 (m, 26H), 0.90 (m, 9H).

### Example 47: Preparation of 3-(((E)-3-butyloct-2-enoyl)oxy)-2-((((3-(dimethylamino)propoxy)carbonyl)oxy)methyl)propyl (9Z,12Z)-octadeca-9,12-dienoate (CPL-221)

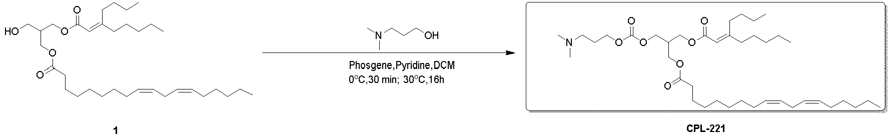

A mixture of 3-((E)-3-butyloct-2-enoate)-2-(hydroxymethyl)propyl-(9Z,12Z)-octadeca-9,12-dienoate (150 mg, 0.27 mmol, 1.0 equivalent), pyridine (216 mg, 2.73 mmol, 10.0 equivalent) and triphosgene (33 mg, 0.10 mmol, 0.4 equivalent) in dichloromethane (3 mL) was stirred at 0°C for 30 minutes. N,N-dimethylpropanolamine (83 mg, 0.82 mmol, 3.0 equivalent) was added and stirred at room temperature for 16 hours. 10 mL of water was added and extracted three times with 10 mL of dichloromethane. The organic phase was washed three times with 20 mL of sodium chloride solution, filtered and dried over sodium sulfate. The mixture was concentrated and purified by preparative HPLC to obtain the target CPL-221 (60 mg, 32% yield) as a colorless oil.

LC/MS (ESI) m/z: 678.6 (M+H)⁺.

1H NMR (400 MHz, CDCl3) δ 5.61 (s, 1H), 5.48 - 5.20 (m, 4H), 4.31 - 4.01 (m, 8H), 2.82 - 2.69 (m, 2H), 2.64 - 2.50 (m, 2H), 2.50 - 2.39 (m, 1H), 2.38 - 2.28 (m, 4H), 2.23 (s, 6H), 2.17 - 2.10 (m, 2H), 2.09 - 2.00 (m, 4H), 1.90 - 1.78 (m, 2H), 1.50 - 1.24 (m, 26H), 0.97 - 0.85 (m, 9H).

### Example 48: Preparation of 3-(((3-(dimethylamino)propoxy)carbonyl)oxy)-2-((((E)-3-pentylnon-2-enoyl)oxy)methyl)propyl (9Z,12Z)-octadeca-9,12-dienoate (CPL-222)

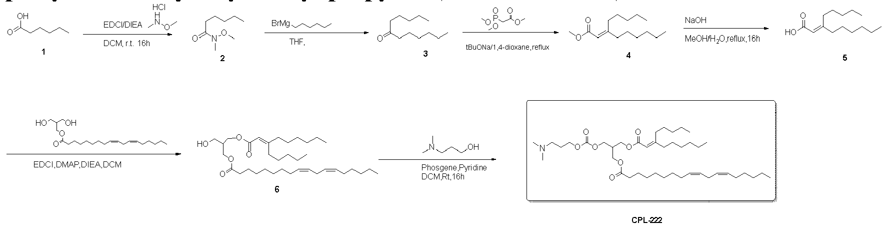

A mixture of 3-((E)-3-pentylnon-2-enoate)-2-(hydroxymethyl)propyl-(9Z,12Z)-octadeca-9,12-dienoate (200 mg, 0.3472 mmol, 1.0 equivalent), N,N-dimethylpropanolamine (72 mg, 0.6944 mmol, 2.0 equivalent), pyridine (274 mg, 3.472 mmol, 10.0 equivalent) and triphosgene (41 mg, 0.1389 mmol, 0.4 equivalent) in dichloromethane (8 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-222 (71.12 mg, 29% yield) as a colorless oil.

LC/MS (ESI) m/z: 705.9 (M+H)⁺.

1HNMR(400 MHz, CDCl3) δ 5.54 (s, 1H), 5.33 - 5.21 (m, 4H), 4.16 - 4.07 (m, 8H), 2.70 (t,J = 6.5 Hz, 2H), 2.55 - 2.47 (m, 2H), 2.39 - 2.29 (m, 3H), 2.24 (t,J = 7.6 Hz, 2H), 2.18 (s, 6H), 2.09 - 2.04 (m, 2H), 1.98 (q,J = 6.9 Hz, 4H), 1.82 - 1.75 (m, 2H), 1.38 (dd,J = 10.0, 6.4 Hz, 4H), 1.22 (dd,J = 13.9, 11.3 Hz, 26H), 0.82 (dd, J = 7.0, 4.4 Hz, 9H).

### Example 49: Preparation of 3-(((3-(dimethylamino)propoxy)carbonyl)oxy)-2-((((E)-3-pentylundec-2-enoyl)oxy)methyl)propyl (9Z,12Z)-octadeca-9,12-dienoate (CPL-223)

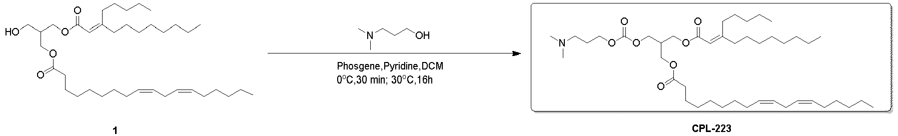

A mixture of 3-((E)-3-pentylundec-2-enoate)-2-(hydroxymethyl)propyl-(9Z,12Z)-octadeca-9,12-dienoate (150 mg, 0.237 mmol, 1.0 equivalent), pyridine (188 mg, 2.37 mmol, 10.0 equivalent) and triphosgene (29 mg, 0.09 mmol, 0.4 equivalent) in dichloromethane (3 mL) was stirred at 0°C for 30 minutes. A solution of N,N-dimethylpropanolamine (73 mg, 0.71 mmol, 3.0 equivalent) in 1 mL of dichloromethane was added and stirred at 30°C for 16 hours. 10 mL of water was added and extracted three times with 10 mL of dichloromethane. The organic phase was washed three times with 20 mL of sodium chloride solution, filtered and dried over sodium sulfate. The mixture was concentrated and purified by preparative HPLC to obtain the target CPL-223 (36 mg, 21% yield) as a colorless oil.

LC/MS (ESI) m/z: 734.6 (M+H)⁺.

1H NMR (400 MHz, CDCl3) δ 5.61 (s, 1H), 5.44 - 5.26 (m, 4H), 4.30 - 4.00 (m, 8H), 2.84 - 2.69 (m, 2H), 2.62 - 2.53 (m, 2H), 2.49 - 2.39 (m, 1H), 2.39 - 2.27 (m, 4H), 2.23 (s, 6H), 2.17 - 2.10 (m, 2H), 2.10 - 1.99 (m, 4H), 1.92 - 1.78 (m, 2H), 1.47 - 1.27 (m, 34H), 0.96 - 0.85 (m, 9H).

### Example 50: Preparation of 3-(((3-(dimethylamino)propoxy)carbonyl)oxy)-2-((((E)-3-pentyltridec-2-enoyl)oxy)methyl)propyl (9Z,12Z)-octadeca-9,12-dienoate (CPL-224)

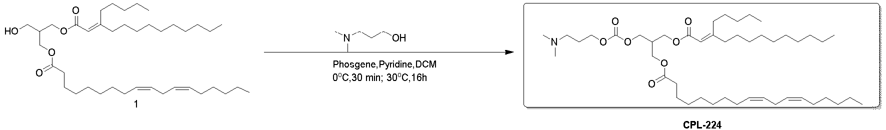

A mixture of 3-((E)-3-pentyltridec-2-enoate)-2-(hydroxymethyl)propyl-(9Z,12Z)-octadeca-9,12-dienoate (200 mg, 0.327 mmol, 1.0 equivalent), pyridine (252 mg, 3.2 mmol, 10.0 equivalent) and triphosgene (37 mg, 0.128 mmol, 0.4 equivalent) in dichloromethane (2 mL) was stirred at 0°C for 30 minutes. N,N-dimethylpropanolamine (98.9 mg, 0.96 mmol, 3.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-224 (45.26 mg, 18% yield) as a colorless oil.

### Example 51: Preparation of 3-(((3-(dimethylamino)propoxy)carbonyl)oxy)-2-((((E)-3-heptyltridec-2-enoyl)oxy)methyl)propyl (9Z,12Z)-octadeca-9,12-dienoate (CPL-231)

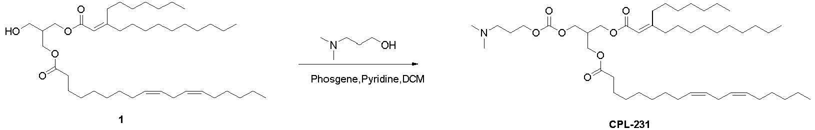

A mixture of 3-((E)-3-heptyltridec-2-enoate)-2-(hydroxymethyl)propyl-(9Z,12Z)-octadeca-9,12-dienoate (200 mg, 0.303 mmol, 1.0 equivalent), pyridine (239.67 mg, 3.03 mmol, 10.0 equivalent) and triphosgene (35.90 mg, 0.121 mmol, 0.4 equivalent) in dichloromethane (5 mL) was stirred at 0°C for 30 minutes. N,N-dimethylpropanolamine (62.48 mg, 0.606 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-231 (51.52 mg, 21.5% yield) as a colorless oil.

LCMS (ESI) m/z: 790.65 (M+H)⁺.

1H NMR (400 MHz, Chloroform-d) δ 5.60 (s, 1H), 5.35 (m, 4H), 4.23 - 4.13 (m, 8H), 2.77 (t, J = 6.5 Hz, 2H), 2.61 - 2.53 (m, 2H), 2.49 - 2.42 (m, 1H), 2.39 (t, J = 7.5 Hz, 2H), 2.31 (t, J = 7.6 Hz, 2H), 2.25 (s, 6H), 2.13 (t, J = 7.7 Hz, 2H), 2.05 (m, 4H), 1.86 (m, 2H), 1.44 (m, 6H), 1.28 (m, 36H), 0.88 (m, 9H).

### Example 52: Preparation of 3-((3-decyltridec-2-enoyl)oxy)-2-((((3-(dimethylamino)propoxy)carbonyl)oxy)methyl)propyl (9Z,12Z)-octadeca-9,12-dienoate (CPL-234)

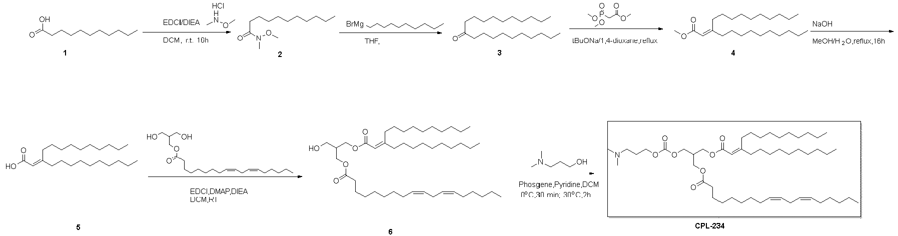

A mixture of 3-((E)-3-decyltridec-2-enoate)-2-(hydroxymethyl)propyl-(9Z,12Z)-octadeca-9,12-dienoate (190 mg, 0.2707 mmol, 1.0 equivalent), N,N-dimethylpropanolamine (56 mg, 0.5413 mmol, 2.0 equivalent), pyridine (214 mg, 2.707 mmol, 10.0 equivalent) and triphosgene (32 mg, 0.1083 mmol, 0.4 equivalent) in dichloromethane (8 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-234 (40.04 mg, 18% yield) as a colorless oil.

LC/MS (ESI) m/z: 832.7 (M+H)⁺.

1HNMR(400 MHz, CDCl3) δ 5.53 (s, 1H), 5.31 - 5.22 (m, 4H), 4.17 - 4.05 (m, 8H), 2.70 (t,J = 6.2 Hz, 2H), 2.55 - 2.47 (m, 2H), 2.33 (dd,J = 14.9, 7.4 Hz, 2H), 2.27 - 2.15 (m, 9H), 2.09-2.02 (m, 2H), 1.98 (q,J = 6.7 Hz, 5H), 1.84 - 1.73 (m, 3H), 1.37 (d,J = 4.5 Hz, 4H), 1.24 - 1.17 (m, 42H), 0.83 - 0.80 (m, 9H).

### Example 53: Preparation of 3-(((4-(dimethylamino)butoxy)carbonyl)oxy)-2-(((E)-3-heptylundec-2-enoyl)oxy)methyl)propyl (9Z,12Z)-octadeca-9,12-dienoate (CPL-252)

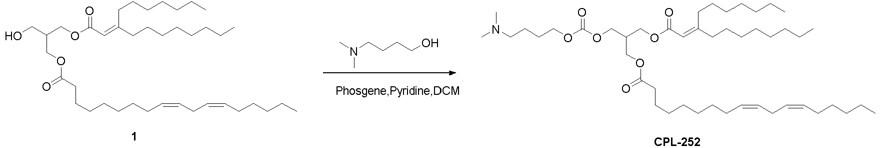

A mixture of 3-((E)-3-heptylundec-2-enoate)-2-(hydroxymethyl)propyl-(9Z,12Z)-octadeca-9,12-dienoate (200 mg, 0.316 mmol, 1.0 equivalent), pyridine (249.9 mg, 3.16 mmol, 10.0 equivalent) and triphosgene (38.57 mg, 0.130 mmol, 0.4 equivalent) in dichloromethane (5 mL) was stirred at 0°C for 30 minutes. N,N-dimethylbutanolamine (74.02 mg, 0.632 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-252 (30.28 mg, 12.3% yield) as a colorless oil.

LCMS (ESI) m/z: 776.63 (M+H)⁺.

1H NMR (400 MHz, Chloroform-d) δ 5.61 (d, J = 6.2 Hz, 1H), 5.42 - 5.29 (m, 4H), 4.26 - 4.09 (m, 8H), 2.77 (t, J = 6.5 Hz, 2H), 2.58 (t, J = 7.8 Hz, 2H), 2.44 (m, 1H), 2.35 -2.18 (m, 10H), 2.13 (t, J = 7.7 Hz, 2H), 2.06 (t, J = 6.6 Hz, 4H), 1.70 (m, 2H), 1.44 (d, J = 7.6 Hz, 4H), 1.37 - 1.24 (m, 36H), 0.89 (m, 9H).

### Example 54: Preparation of 3-((3-decyltridec-2-enoyl)oxy)-2-((((4-(dimethylamino)butyloxy)carbonyl)oxy)methyl)propyl (9Z,12Z)-octadeca-9,12-dienoate (CPL-256)

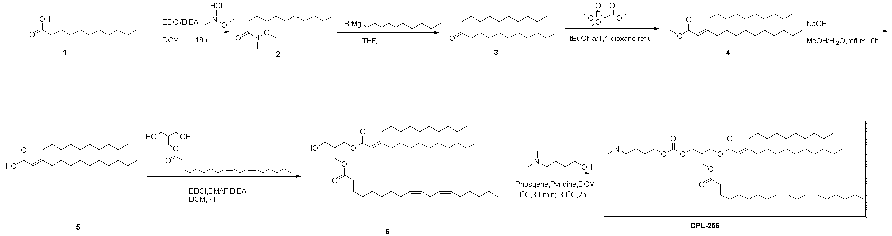

A mixture of 3-((E)-3-decyltridec-2-enoate)-2-(hydroxymethyl)propyl-(9Z,12Z)-octadeca-9,12-dienoate (190 mg, 0.2707 mmol, 1.0 equivalent), N,N-dimethylbutanolamine (63 mg, 0.5413 mmol, 2.0 equivalent), pyridine (214 mg, 2.707 mmol, 10.0 equivalent) and triphosgene (32 mg, 0.1083 mmol, 0.4 equivalent) in dichloromethane (6 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-256 (36.08 mg, 16% yield) as a colorless oil.

LC/MS (ESI) m/z: 846.7 (M+H)⁺.

1HNMR(400 MHz, CDCl3) δ 5.53 (s, 1H), 5.35 - 5.21 (m, 4H), 4.18 - 4.03 (m, 8H), 2.70 (t,J = 6.5 Hz, 2H), 2.55 - 2.45 (m, 2H), 2.37 (dt,J = 9.2, 4.7 Hz, 1H), 2.27 - 2.18 (m, 4H), 2.14 (s, 6H), 2.09 - 2.03 (m, 2H), 1.98 (q,J = 6.6 Hz, 5H), 1.63 (dd,J = 13.0, 6.3 Hz, 3H), 1.35 (d,J = 4.7 Hz, 2H), 1.24 - 1.18 (m, 46H), 0.82 - 0.79 (m, 9H).

### Example 55: Preparation of 3-(((3-(diethylamino)propoxy)carbonyl)oxy)-2-((((E)-3-pentylnon-2-enoyl)oxy)methyl)propyl (9Z,12Z)-octadeca-9,12-dienoate (CPL-266)

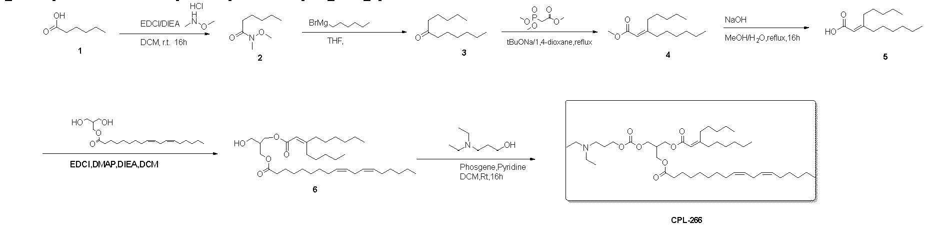

A mixture of 3-((E)-3-pentylnon-2-enoate)-2-(hydroxymethyl)propyl-(9Z,12Z)-octadeca-9,12-dienoate (200 mg, 0.3472 mmol, 1.0 equivalent), N,N-diethylpropanolamine (91 mg, 0.6944 mmol, 2.0 equivalent), pyridine (274 mg, 3.407 mmol, 10.0 equivalent) and triphosgene (41 mg, 0.1389 mmol, 0.4 equivalent) in dichloromethane (8 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-266 (35.80 mg, 14% yield) as a colorless oil.

LC/MS (ESI) m/z: 735.1 (M+H)⁺.

1HNMR(400 MHz, CDCl3) δ 5.54 (s, 1H), 5.33 - 5.22 (m, 4H), 4.16 - 4.07 (m, 8H), 2.70 (t,J = 6.5 Hz, 2H), 2.53 - 2.34 (m, 9H), 2.24 (t,J = 7.6 Hz, 2H), 2.10 - 2.03 (m, 2H), 1.98 (q,J = 6.8 Hz, 4H), 1.77 (s, 2H), 1.38 (dd,J = 8.5, 5.5 Hz, 4H), 1.22 (dd,J = 13.9, 11.3 Hz, 26H), 0.97 (t,J = 7.0 Hz, 6H), 0.82 (dd,J = 7.0, 4.3 Hz, 9H).

### Example 56: Preparation of 3-(((3-(diethylamino)propoxy)carbonyl)oxy)-2-((((E)-3-heptylundec-2-enoyl)oxy)methyl)propyl (9Z,12Z)-octadeca-9,12-dienoate (CPL-274)

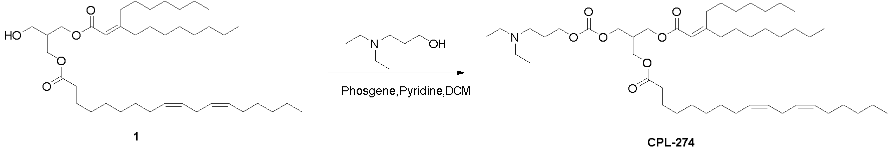

A mixture of 3-((E)-3-heptylundec-2-enoate)-2-(hydroxymethyl)propyl-(9Z,12Z)-octadeca-9,12-dienoate (200 mg, 0.316 mmol, 1.0 equivalent), pyridine (249.9 mg, 3.16 mmol, 10.0 equivalent) and triphosgene (38.57 mg, 0.130 mmol, 0.4 equivalent) in dichloromethane (5 mL) was stirred at 0°C for 30 minutes. N,N-diethylpropanolamine (82.93 mg, 0.632 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-274 (34.53 mg, 13.8% yield) as a colorless oil.

LCMS (ESI) m/z: 790.65 (M+H)⁺.

1H NMR (400 MHz, Chloroform-d) δ 5.60 (s, 1H), 5.41 - 5.30 (m, 4H), 4.23 - 4.14 (m, 8H), 2.77 (t, J = 6.5 Hz, 2H), 2.67 - 2.38 (m, 9H), 2.30 (t, J = 7.6 Hz, 2H), 2.13 (t, J = 7.7 Hz, 2H), 2.04 (t, J = 6.9 Hz, 4H), 1.84 (s, 2H), 1.48 - 1.40 (m, 4H), 1.35 - 1.25 (m, 34H), 1.04 (s, 6H), 0.89 (m, 9H).

### Example 57: Preparation of 3-(((2-(1-methylpiperidin-4-yl)ethoxy)carbonyl)oary)-2-((((E)-3-propylnon-2-enoyl)oxy)methyl)propyl (9Z,12Z)-octadeca-9,12-dienoate (CPL-280)

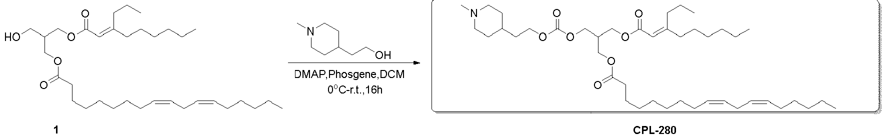

3-((E)-3-propylnon-2-enoate)-2-(hydroxymethyl)propyl-(9Z,12Z)-octadeca-9,12-dienoate (183 mg, 0.334 mmol, 1.0 equivalent) and triphosgene (39 mg, 0.133 mmol, 0.4 equivalent) were mixed in dichloromethane (6 mL), and 4-dimethylaminopyridine (81 mg, 0.668 mmol, 2.0 equivalent) was added dropwise at 0°C under nitrogen protection and stirred for 20 minutes. N-methyl-4-(2-hydroxyethyl)piperidine (95 mg, 0.668 mmol, 2.0 equivalent) was added dropwise and stirred at room temperature overnight. 15 mL of ice-water was added and extracted three times with 20 mL of dichloromethane. The organic phase was washed three times with 20 mL of sodium chloride solution, filtered and dried over sodium sulfate. The mixture was concentrated and purified by preparative HPLC to obtain the target CPL-280 (72 mg, 30.1 % yield) as a colorless oil.

LC/MS (ESI) m/z: 718.7 (M+H)⁺.

1H NMR (400 MHz, CDCL3) δ 5.62 (d, J = 5.2 Hz, 1H), 5.40-5.31 (m, 4H), 4.22-4.12 (m, 8H), 2.78-2.72 (m, 4H), 2.65-2.54 (m, 3H), 2.45-2.42 (m,1H), 2.32 (t, J = 7.6 Hz, 2H), 2.15-2.10 (m, 2H), 2.07-1.99 (m, 5H), 1.95-1.80 (m, 3H), 1.72-1.57 (m, 4H), 1.52-1.25 (m, 29H), 0.97-0.83 (m, 9H).

### Example 58: Preparation of 3-(((2-(1-methylpiperidin-4-yl)ethoxy)carbonyl)oary)-2-((((E)-3-pentylundec-2-enoyl)oxy)methyl)propyl (9Z,12Z)-octadeca-9,12-dienoate (CPL-289)

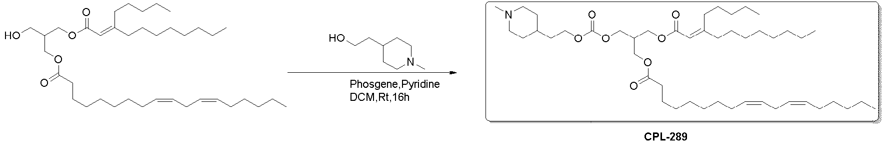

A mixture of 3-((E)-3-pentylundec-2-enoate)-2-(hydroxymethyl)propyl-(9Z,12Z)-octadeca-9,12-dienoate (100 mg, 0.1653 mmol, 1.0 equivalent), N-methyl-4-(2-hydroxyethyl)piperidine (71.03 mg, 0.4959 mmol, 3.0 equivalent), pyridine (130.76 mg, 1.65 mmol, 10.0 equivalent) and triphosgene (19.62 mg, 0.0601 mmol, 0.4 equivalent) in dichloromethane (5 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-289 (89.14 mg, 64.66% yield) as a colorless oil.

LC/MS (ESI) m/z: 774.8 (M+H)⁺.

1HNMR(400 MHz, CDCl3) δ 5.60 (s, 1H), 5.37 - 5.31 (m, 4H), 4.20 (d,J = 4.8 Hz, 2H), 4.18 - 4.14 (m,6H), 2.82 (s, 2H), 2.76 (d,J = 6.4 Hz, 2H), 2.61 - 2.55 (m, 2H), 2.46 - 2.42 (m, 1H), 2.30 (d,J = 6.8 Hz,2H), 2.26 (s, 2H), 2.12 (d,J = 7.4 Hz, 2H), 2.03 (d,J = 5.8 Hz, 3H), 1.91 (d,J = 12.4 Hz, 2H), 1.61 (s,6H), 1.25 (s, 36H), 0.89 (d,J = 2.8 Hz, 10H).

### Example 59: Preparation of 3-(((2-(1-methylpiperidin-4-yl)ethoxy)carbonyl)oary)-2-(((E)-3-pentyltridec-2-enoyl)oxy)methyl)propyl (9Z,12Z)-octadeca-9,12-dienoate (CPL-290)

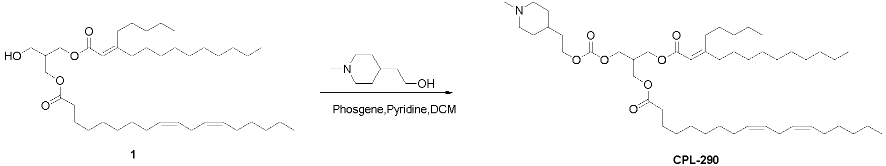

A mixture of 3-((E)-3-pentyltridec-2-enoate)-2-(hydroxymethyl)propyl-(9Z,12Z)-octadeca-9,12-dienoate (300 mg, 0.45 mmol, 1.0 equivalent), pyridine (308.50 mg, 4.5 mmol, 10.0 equivalent) and triphosgene (46.29 mg, 0.18 mmol, 0.4 equivalent) in dichloromethane (5 mL) was stirred at 0°C for 30 minutes. N-methyl-4-(2-hydroxyethyl)piperidine (113.13 mg, 0.90 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-290 (32.51 mg, 8.99% yield) as a colorless oil.

LCMS (ESI) m/z: 803.1 (M+H)⁺.

1H NMR (400 MHz, Chloroform-d) δ 5.60 (s, 1H), 5.43 - 5.28 (m, 4H), 4.23 - 4.14(m, 8H), 2.87 (d, J = 11.3 Hz, 2H), 2.77 (t, J = 6.5 Hz, 2H), 2.62 - 2.54 (m, 2H), 2.49 -2.40 (m, 1H), 2.31 (d, J = 7.5 Hz, 2H), 2.28 (d, J = 1.6 Hz, 3H), 2.16 - 2.10 (m, 2H),2.05 (q, J = 6.9 Hz, 4H), 1.95 (t, J = 11.4 Hz, 2H), 1.76 - 1.67 (m, 3H), 0.89 (tt, J =4.2, 2.4 Hz, 9H).

### Example 60: Preparation of 3-(((E)-3-hearylundec-2-enoyl)oxy)-2-((((2-(1-methylpiperidin-4-yl)ethoxy)carbonyl)oxy)methyl)propyl (9Z,12Z)-octadeca-9,12-dienoate (CPL-292)

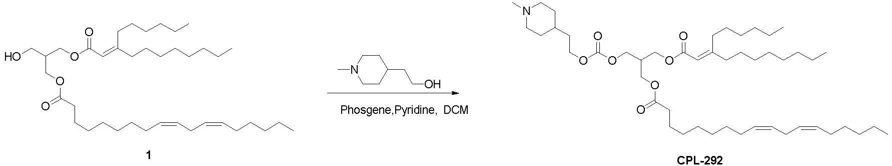

A mixture of 3-((E)-3-hexylundec-2-enoate)-2-(hydroxymethyl)propyl-(9Z,12Z)-octadeca-9,12-dienoate (150 mg, 0.24 mmol, 1.0 equivalent), pyridine (0.2 mL, 2.5 mmol, 10.0 equivalent) and triphosgene (30 mg, 0.101 mmol, 0.4 equivalent) in dichloromethane (3 mL) was stirred at 0°C for 30 minutes. N-methyl-4-(2-hydroxyethyl)piperidine (75 mg, 0.52 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-292 (17.10 mg, 8.95% yield) as a colorless oil.

LCMS (ESI) m/z: 788.6 (M+H)⁺.

1H NMR (400 MHz, Chloroform-d) δ 5.60 (s, 1H), 5.35 (s, 4H), 4.24 - 4.10 (m, 6H), 3.99 (d, J = 4.6 Hz, 2H), 2.98 (s, 2H), 2.77 (s, 2H), 2.57 (s, 2H), 2.42 (d, J = 11.8 Hz, 2H), 2.36 - 2.24 (m, 3H), 2.12 (d, J = 8.0 Hz, 2H), 2.04 (d, J = 6.8 Hz, 8H), 1.74 (s, 4H), 1.47 - 1.05 (m, 63H), 0.94 - 0.74 (m, 18H).

### Example 61: Preparation of 3-(((3-(dimethylamino)-2,2-dimethylpropoxy)carbonyl)oxy)-2-((((E)-3-propyl-2-enoyl)oxy)methyl)propyl (9E,12E)-octadeca-9,12-dienoate (CPL-304)

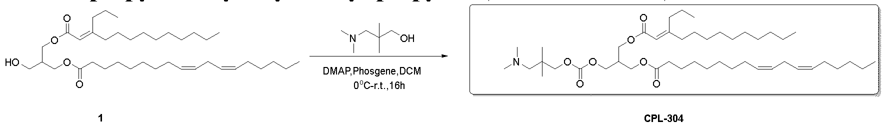

3-((E)-3-propyltridec-2-enoate)-2-(hydroxymethyl)propyl-(9Z,12Z)-octadeca-9,12-dienoate (237 mg, 0.392 mmol, 1.0 equivalent) and triphosgene (46 mg, 0.157 mmol, 0.4 equivalent) were mixed in dichloromethane (4 mL), and 4-dimethylaminopyridine (95 mg, 0.784 mmol, 2.0 equivalent) was added dropwise at 0°C under nitrogen protection and stirred for 20 minutes. 3-Dimethylamino-2,2-dimethyl-1-propanol (103 mg, 0.784 mmol, 2.0 equivalent) was added dropwise and stirred at room temperature overnight. 15 mL of ice-water was added and extracted three times with 20 mL of dichloromethane. The organic phase was washed three times with 20 mL of sodium chloride solution, filtered and dried over sodium sulfate. The mixture was concentrated and purified by preparative HPLC to obtain the target CPL-304 (34 mg, 11.4% yield) as a colorless oil.

LC/MS (ESI) m/z: 762.6 (M+H)⁺.

1H NMR (400 MHz, CDCL3) δ 5.55-5.53 (m, 1H), 5.34-5.23 (m, 4H), 4.15-4.08 (m, 6H), 3.87 (s, 2H), 2.71 (t, J = 6.4 Hz, 2H), 2.51-2.47 (m, 2H), 2.41-2.35 (m, 1H), 2.25-2.20 (m, 8H), 2.09-2.03 (m, 4H), 2.00-1.95 (m, 4H), 1.53 (s, 6H), 1.47-1.35 (m, 4H), 1.32-1.19 (m, 24H), 0.89-0.76 (m, 15H).

### Example 62: Preparation of 3-(((E)-3-butyloct-2-enoyl)oxy)-2-((((3-(dimethylamino)-2,2-dimethylpropoxy)carbonyl)oxy)methyl)propyl (9Z,12Z)-octadeca-9,12-dienoate (CPL-309)

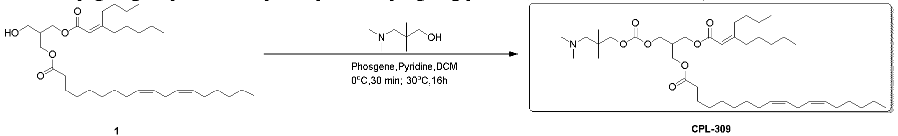

A mixture of 3-((E)-3-butyloct-2-enoate)-2-(hydroxymethyl)propyl-(9Z,12Z)-octadeca-9,12-dienoate (150 mg, 0.27 mmol, 1.0 equivalent), pyridine (216 mg, 2.73 mmol, 10.0 equivalent) and triphosgene (33 mg, 0.10 mmol, 0.4 equivalent) in dichloromethane (3 mL) was stirred at 0°C for 30 minutes. 3-Dimethylamino-2,2-dimethyl-1-propanol (130 mg, 0.82 mmol, 3.0 equivalent) was added and stirred at room temperature for 16 hours. 10 mL of water was added and extracted three times with 10 mL of dichloromethane. The organic phase was washed three times with 20 mL of sodium chloride solution, filtered and dried over sodium sulfate. The mixture was concentrated and purified by preparative HPLC to obtain the target CPL-309 (95 mg, 49% yield) as a colorless oil.

LC/MS (ESI) m/z: 706.6 (M+H)⁺.

1H NMR (400 MHz, CDC13) δ 5.61 (s, 1H), 5.46 - 5.26 (m, 4H), 4.28 - 4.09 (m, 6H), 3.95 (s, 2H), 2.82 - 2.70 (m, 2H), 2.63 - 2.54 (m, 2H), 2.52 - 2.37 (m, 1H), 2.32 - 2.28 (m, 4H), 2.28 (s, 3H), 2.19 - 2.10 (m, 4H), 2.10 - 1.97 (m, 4H), 1.66 - 1.60 (m, 2H), 1.47 - 1.20 (m, 25H), 1.00 - 0.75 (m, 15H).

### Example 63: Preparation of 3-(((E)-3-butyldec-2-enoyl)oxy)-2-((((3-(dimethylamino)-2,2-dimethylpropoxy)carbonyl)oxy)methyl)propyl (9Z,12Z)-octadeca-9,12-dienoate (CPL-316)

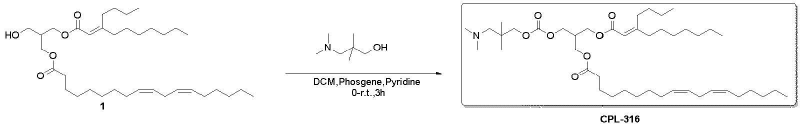

A mixture of 3-((E)-3-butyldec-2-enoate)-2-(hydroxymethyl)propyl-(9Z,12Z)-octadeca-9,12-dienoate (200 mg, 0.33 mmol, 1.0 equivalent), pyridine (260.7 mg, 3.3 mmol, 10.0 equivalent) and triphosgene (40 mg, 0.13 mmol, 0.4 equivalent) in dichloromethane (2 mL) was stirred at 0°C for 30 minutes. 3-Dimethylamino-2,2-dimethyl-1-propanol (131 mg, 1.04 mmol, 3.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-316 (98.2 mg, 76% yield) as a colorless oil.

LCMS (ESI) m/z:734.12(M+H)⁺.

1H NMR (400 MHz, CDC13) δ 5.61 (s, 1H), 5.43 - 5.24 (m, 4H), 4.22-4.12 (m, 6H), 3.94 (s, 2H), 2.77 (s, 2H), 2.62 - 2.51 (m, 1H), 2.47 - 2.41 (m, 1H), 2.36 - 2.23 (m, 8H), 2.16 (s, 3H), 2.08 - 1.98 (m, 6H), 1.61 (s, 2H), 1.33 - 1.27 (m, 28H), 0.93-0.80 (m, 15H).

### Example 64: Preparation of 3-((3-decyltridec-2-enoyl)oxy)-2-((((3-(dimethylamino)-2,2-dimethylpropoxy)carbonyl)oxy)methyl)propyl (9Z,12Z)-octadeca-9,12-dienoate (CPL-322)

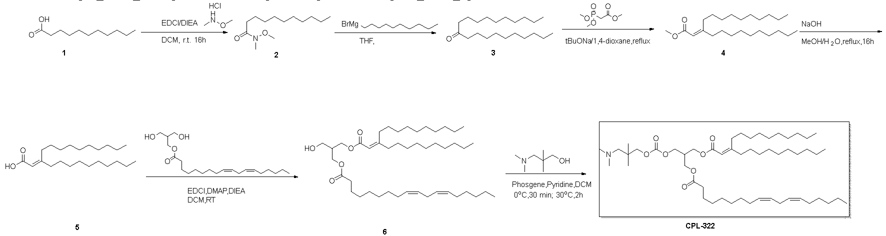

A mixture of 3-((E)-3-decyltridec-2-enoate)-2-(hydroxymethyl)propyl-(9Z,12Z)-octadeca-9,12-dienoate (190 mg, 0.2707 mmol, 1.0 equivalent), 3-dimethylamino-2,2-dimethyl-1-propanol (71 mg, 0.5413 mmol, 2.0 equivalent), pyridine (214 mg, 2.707 mmol, 10.0 equivalent) and triphosgene (32 mg, 0.1083 mmol, 0.4 equivalent) in dichloromethane (6 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-322 (39.69 mg, 17% yield) as a colorless oil.

LC/MS (ESI) m/z: 860.8 (M+H)⁺.

1HNMR(400 MHz, CDC13) δ 5.53 (s, 1H), 5.35 - 5.22 (m, 4H), 4.10 (ddd,J = 14.5, 11.9, 7.4 Hz, 6H), 3.88 (s, 2H), 2.70 (t,J = 6.5 Hz, 2H), 2.58 - 2.45 (m, 2H), 2.38 (dt,J = 11.8, 5.9 Hz, 1H), 2.29 - 2.16 (m, 8H), 2.02 (ddd,J = 20.3, 14.5, 7.3 Hz, 8H), 1.37 (d,J = 7.4 Hz, 2H), 1.26 - 1.17 (m, 46H), 0.85 - 0.79 (m, 15H).

### Example 65: Preparation of 3-((((1-methylpyrrolidin-3-yl)methoxy)carbonyl)oxy)-2-((((E)-3-propylnon-2-enoyl)oxy)methyl)propyl (9Z,12Z)-octadeca-9,12-dienoate (CPL-324)

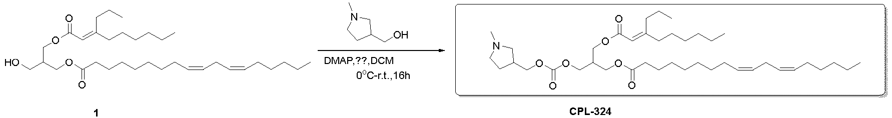

3-((E)-3-propylnon-2-enoate)-2-(hydroxymethyl)propyl-(9Z,12Z)-octadeca-9,12-dienoate (300 mg, 0.547 mmol, 1.0 equivalent) and triphosgene (65 mg, 0.218 mmol, 0.4 equivalent) were mixed in dichloromethane (6 mL), and 4-dimethylaminopyridine (133 mg, 1.094 mmol, 2.0 equivalent) was added dropwise at 0°C under nitrogen protection and stirred for 20 minutes. 1-Methyl-3-pyrrolidinemethanol (126 mg, 1.094 mmol, 2.0 equivalent) was added dropwise and stirred at room temperature overnight. 15 mL of ice-water was added and extracted three times with 20 mL of dichloromethane. The organic phase was washed three times with 20 mL of sodium chloride solution, filtered and dried over sodium sulfate. The mixture was concentrated and purified by preparative HPLC to obtain the target CPL-324 (112 mg, 29.7% yield) as a colorless oil.

LC/MS (ESI) m/z: 690.6 (M+H)⁺.

1H NMR (400 MHz, CDCL3) δ 5.61 (d, J = 5.2 Hz, 1H), 5.39-5.31 (m, 4H), 4.22-4.01 (m, 8H), 2.78 (t, J = 6.8 Hz, 2H), 2.66-2.64 (m, 1H), 2.59-2.51 (m, 5H), 2.45-2.42 (m, 1H), 2.35 (s, 3H), 2.32 (t, J = 7.6 Hz, 2H), 2.15-2.10 (m, 2H), 2.07-2.00 (m, 5H), 1.62-1.60 (m, 2H), 1.52-1.42 (m, 4H), 1.37-1.25 (m, 22H), 0.96-0.85 (m, 9H).

### Example 66: Preparation of 3-(((E)-3-butylundec-2-enoyl)oary)-2-(((((1-methylpyrrolidin-3-yl)methoxy)carbonyl)oxy)methyl)propyl (9Z,12Z)-octadeca-9,12-dienoate (CPL-329)

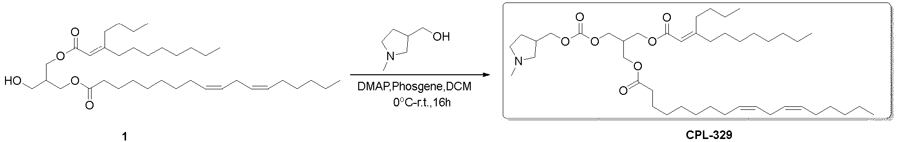

3-((E)-3-butylundec-2-enoate)-2-(hydroxymethyl)propyl-(9Z,12Z)-octadeca-9,12-dienoate (200 mg, 0.339 mmol, 1.0 equivalent) and triphosgene (40 mg, 0.135 mmol, 0.4 equivalent) were mixed in dichloromethane (5 mL), and 4-dimethylaminopyridine (82 mg, 0.678 mmol, 2.0 equivalent) was added dropwise at 0°C under nitrogen protection and stirred for 20 minutes. 1-Methyl-3-pyrrolidinemethanol (78 mg, 0.678 mmol, 2.0 equivalent) was added dropwise and stirred at room temperature overnight. 15 mL of ice-water was added and extracted three times with 20 mL of dichloromethane. The organic phase was washed three times with 20 mL of sodium chloride solution, filtered and dried over sodium sulfate. The mixture was concentrated and purified by preparative HPLC to obtain the target CPL-329 (57 mg, 22.9% yield) as a colorless oil.

LC/MS (ESI) m/z: 732.1 (M+H)⁺.

1H NMR (400 MHz, CDCL3) δ 5.60 (s, 1H), 5.40-5.31 (m, 4H), 4.22-4.12 (m, 8H), 2.90-2.65 (m, 7H), 2.59-2.55 (m, 2H), 2.48-2.40 (m, 1H), 2.32-2.20 (m, 3H), 2.16-2.11 (m, 2H), 2.07-2.02 (m, 6H), 1.90-1.78 (m, 2H), 1.62-1.59 (m, 2H), 1.46-1.26 (m, 30H), 0.93-0.83 (m, 9H).

### Example 67: Preparation of 3-(((E)-3-butyldec-2-enoyl)oxy)-2-(((((l-methylpyrrolidin-3-yl)methoxy)carbonyl)oxy)methyl)propyl (9Z,12Z)-octadeca-9,12-dienoate (CPL-338)

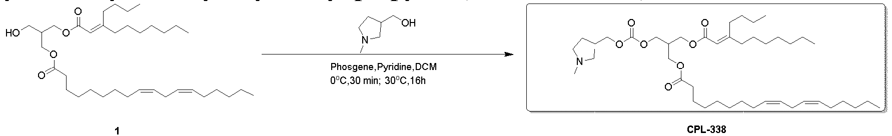

A mixture of 3-((E)-3-butyldec-2-enoate)-2-(hydroxymethyl)propyl-(9Z,12Z)-octadeca-9,12-dienoate (200 mg, 0.35 mmol, 1.0 equivalent), pyridine (276.5 mg, 3.5 mmol, 10.0 equivalent) and triphosgene (41.16 mg, 0.14 mmol, 0.4 equivalent) in dichloromethane (2 mL) was stirred at 0°C for 30 minutes. 1-Methyl-3-pyrrolidinemethanol (115.17 mg, 1.04 mmol, 3.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-338 (36.08 mg, 14% yield) as a colorless oil.

LCMS (ESI) m/z: 718.07 (M+H)⁺.

1H NMR (400 MHz, CDCl3) δ 5.61 (s, 1H), 5.36 (dt, J = 12.4, 6.8 Hz, 4H), 4.26 - 4.01 (m, 8H), 2.77 (t, J = 6.5 Hz, 2H), 2.59 (dd, J = 29.1, 20.1 Hz, 6H), 2.47 - 2.40 (m, 1H), 2.34 (d, J = 11.0 Hz, 4H), 2.18 - 2.10 (m, 2H), 2.07 - 2.01 (m, 4H), 1.28 (d, J = 18.0 Hz, 34H), 0.96 - 0.89 (m, 9H).

### Example 68: Preparation of 3-((((1-methylpyrrolidin-3-yl)methoxy)carbonyl)oxy)-2-((((Z)-3-octyltridec-2-enoyl)oxy)methyl)propyl (9Z,12Z)-octadeca-9,12-dienoate (CPL-343)

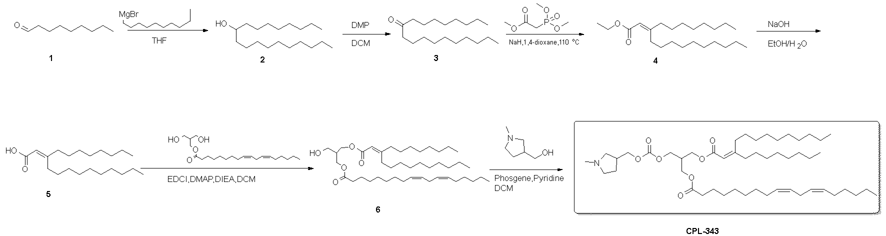

A mixture of 3-((E)-3-octyltridec-2-enoate)-2-(hydroxymethyl)propyl-(9Z,12Z)-octadeca-9,12-dienoate (190 mg, 0.2819 mmol, 1.0 equivalent), 1-methyl-3-pyrrolidinemethanol (65 mg, 0.5638 mmol, 2.0 equivalent), pyridine (223 mg, 2.819 mmol, 10.0 equivalent) and triphosgene (33 mg, 0.1128 mmol, 0.4 equivalent) in dichloromethane (8 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-343 (31.78 mg, 14% yield) as a colorless oil.

LC/MS (ESI) m/z: 816.6 (M+H)⁺.

1HNMR(400 MHz, CDCl3) δ 5.53 (s, 1H), 5.35 - 5.22 (m, 4H), 4.17 - 3.93 (m, 8H), 2.70 (t,J = 6.1 Hz, 2H), 2.50 (dd,J = 14.6, 6.5 Hz, 5H), 2.41 - 2.33 (m, 1H), 2.25 (dd,J = 17.3, 9.8 Hz, 6H), 2.10 - 2.02 (m, 2H), 1.98 (dd,J = 13.5, 6.5 Hz, 5H), 1.40 -1.34 (m, 4H), 1.25 -1.17 (m, 42H), 0.82 - 0.79 (m, 9H).

### Example 69: Preparation of 3-(((E)-3-butylundec-2-enoyl)oary)-2-(((((1-methylpyrrolidin-3-yl)methoxy)carbonyl)oxy)methyl)propyl (9Z,12Z)-octadeca-9,12- dienoate (CPL-348)

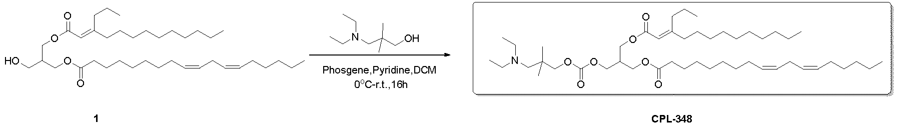

3-((E)-3-propyltridec-2-enoate)-2-(hydroxymethyl)propyl-(9Z,12Z)-octadeca-9,12-dienoate (250 mg, 0.414 mmol, 1.0 equivalent) and triphosgene (49 mg, 0.165 mmol, 0.4 equivalent) were mixed in dichloromethane (6 mL), and a solution of 4-dimethylaminopyridine (101 mg, 0.828 mmol, 2.0 equivalent) in 0.5 mL dichloromethane was added dropwise at 0°C under nitrogen protection and stirred for 20 minutes. 3-Dimethylamino-2,2-dimethyl-1-propanol (131 mg, 0.828 mmol, 2.0 equivalent) was added dropwise and stirred at room temperature overnight. 15 mL of ice-water was added and extracted three times with 20 mL of dichloromethane. The organic phase was washed three times with 20 mL of sodium chloride solution, filtered and dried over sodium sulfate. The mixture was concentrated and purified by preparative HPLC to obtain the target CPL-348 (151 mg, 46.1% yield) as a colorless oil.

LC/MS (ESI) m/z: 791.2 (M+H)⁺.

1H NMR (400 MHz, CDCL3) δ 5.62 (d, J = 5.6 Hz, 1H), 5.41-5.29 (m, 4H), 4.21-4.12 (m, 6H), 3.91 (s, 2H), 2.78 (t, J = 6.8 Hz, 2H), 2.58-2.54 (m, 2H), 2.52-2.41 (m, 5H), 2.32 (t, J = 7.6 Hz, 2H), 2.23 (s, 2H), 2.14-2.10 (m, 2H), 2.07-2.00 (m, 4H), 1.62-1.60 (m, 2H), 1.54-1.42 (m, 4H), 1.39-1.26 (m, 28H), 0.97-0.85 (m, 21H).

### Example 70: Preparation of 3-(((E)-3-butylundec-2-enoyl)oxy)-2-(((((diethylamino)-2,2-dimethylpropoxy)carbonyl)oxy)methyl)propyl (9Z,12Z)-octadeca-9,12-dienoate (CPL-351)

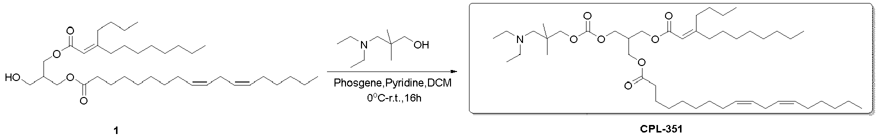

3-((E)-3-butylundec-2-enoate)-2-(hydroxymethyl)propyl-(9Z,12Z)-octadeca-9,12-dienoate (200 mg, 0.339 mmol, 1.0 equivalent) and triphosgene (40 mg, 0.135 mmol, 0.4 equivalent) were mixed in dichloromethane (5 mL), and 4-dimethylaminopyridine (82 mg, 0.678 mmol, 2.0 equivalent) was added dropwise at 0°C under nitrogen protection and stirred for 20 minutes. 3-Dimethylamino-2,2-dimethyl-1-propanol (107 mg, 0.678 mmol, 2.0 equivalent) was added dropwise and stirred at room temperature overnight. 15 mL of ice-water was added and extracted three times with 20 mL of dichloromethane. The organic phase was washed three times with 20 mL of sodium chloride solution, filtered and dried over sodium sulfate. The mixture was concentrated and purified by preparative HPLC to obtain the target CPL-351 (60 mg, 22.8% yield) as a colorless oil.

LC/MS (ESI) m/z: 776.7 (M+H)⁺.

1H NMR (400 MHz, CDCL3) δ 5.60 (s, 1H), 5.41-5.29 (m, 4H), 4.21-4.12 (m, 6H), 3.91 (s, 2H), 2.78 (t, J = 6.4 Hz, 2H), 2.59-2.41 (m, 6H), 2.32 (t, J = 7.6 Hz, 2H), 2.26-2.20 (m, 1H), 2.15-2.12 (m, 2H), 2.07-2.00 (m, 6H), 1.62-1.60 (m, 2H), 1.46-1.26 (m, 35H), 0.96-0.87 (m, 15H).

### Example 71: Preparation of 3-(((3-(diethylamino)-2,2-dimethylpropoxy)carbonyl)oxy)-2-((((E)-3-pentylundec-2-enoyl)oxy)methyl)propyl (9Z,12Z)-octadeca-9,12-dienoate (CPL-355)

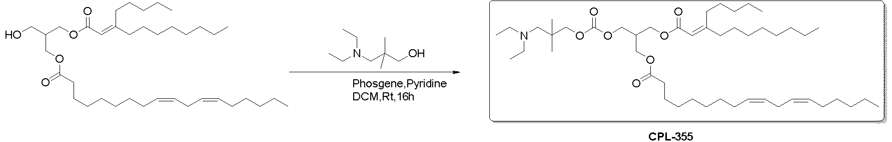

A mixture of 3-((E)-3-pentylundec-2-enoate)-2-(hydroxymethyl)propyl-(9Z,12Z)-octadeca-9,12-dienoate (200 mg, 0.3306 mmol, 1.0 equivalent), 3-dimethylamino-2,2-dimethyl-1-propanol (157.97 mg, 0.9918 mmol, 3.0 equivalent), pyridine (261.51 mg, 3.31 mmol, 10.0 equivalent) and triphosgene (39.12 mg, 0.1322 mmol, 0.4 equivalent) in dichloromethane (5 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-355 (114.7 mg, 43.9% yield) as a colorless oil.

LC/MS (ESI) m/z: 790.8 (M+H)⁺.

1HNMR(400 MHz, CDCl3) δ 5.60 (s, 1H), 5.35 (d,J = 6.4 Hz, 4H), 4.22 - 4.12 (m, 6H), 3.92 (s, 2H), 2.77 (t,J = 5.8 Hz, 2H), 2.62 - 2.54 (m, 2H), 2.48 (d,J = 6.8 Hz, 2H), 2.30 (t,J = 7.8 Hz, 2H), 2.24 (s, 2H), 2.16 - 2.11 (m, 2H), 2.04 (s, 4H), 1.56 (s, 6H), 1.44 (s, 6H), 1.25 (s, 32H), 0.94 (d,J = 7.8 Hz, 6H),0.88 (s, 8H).

### Example 72: Preparation of 3-(((3-(diethylamino)-2,2-dimethylpropoxy)carbonyl)oxy)-2-((((E)-3-heptyltridec-2-enoyl)oxy)methyl)propyl (9Z,12Z)-octadeca-9,12-dienoate (CPL-363)

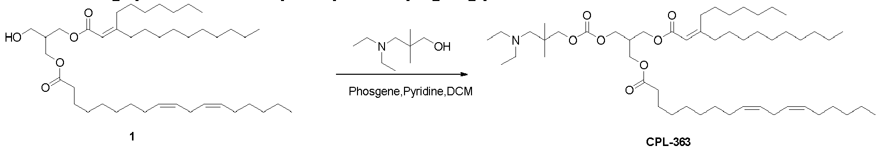

A mixture of 3-((E)-3-heptyltridec-2-enoate)-2-(hydroxymethyl)propyl-(9Z,12Z)-octadeca-9,12-dienoate (200 mg, 0.303 mmol, 1.0 equivalent), pyridine (239.67 mg, 3.03 mmol, 10.0 equivalent) and triphosgene (35.9 mg, 0.121 mmol, 0.4 equivalent) in dichloromethane (5 mL) was stirred at 0°C for 30 minutes. 3-Dimethylamino-2,2-dimethyl-1-propanol (96.45 mg, 0.606 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-363 (42.5 mg, 16.57% yield) as a colorless oil.

LCMS (ESI) m/z: 846.71 (M+H)⁺.

1H NMR (400 MHz, Chloroform-d) δ 5.60 (s, 1H), 5.42 - 5.29 (m, 4H), 4.23 - 4.14 (m, 6H), 3.92 (s, 2H), 2.77 (t, J = 6.5 Hz, 2H), 2.61 - 2.54 (m, 2H), 2.53 - 2.41 (m, 5H), 2.30 (t, J = 7.6 Hz, 2H), 2.24 (s, 2H), 2.17 - 2.10 (m, 2H), 2.05 (m, 4H), 1.44 (d, J = 7.4 Hz, 2H), 1.39 - 1.25 (m, 40H), 0.95 (t, J = 7.1 Hz, 6H), 0.88 (m, 15H).

### Example 73: Preparation of 3-(((Z)-3-butylnon-2-enoyl)oxy)-2-(((((1-methylpiperidin-3-yl)methoxy)carbonyl)oxy)methyl)propyl (9Z,12Z)-octadeca-9,12-dienoate (CPL-372)

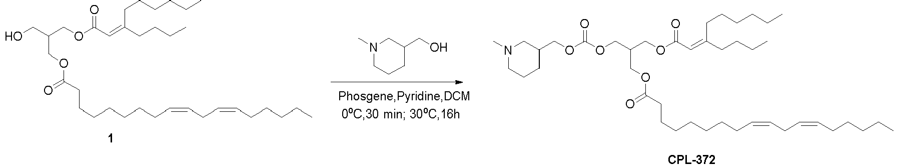

A mixture of 3-((E)-3-butylnon-2-enoate)-2-(hydroxymethyl)propyl-(9Z,12Z)-octadeca-9,12-dienoate (150 mg, 0.267 mmol, 1.0 equivalent), pyridine (212.2 mg, 2.67 mmol, 10.0 equivalent) and triphosgene (31.69 mg, 0.107 mmol, 0.4 equivalent) in dichloromethane (2 mL) was stirred at 0°C for 30 minutes. 1-Methylpiperidine-3-methanol (68.82 mg, 0.533 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-372 (40.26 mg, 21.01% yield) as a colorless oil.

LCMS (ESI) m/z: 718.55 (M+H)⁺.

1H NMR (400 MHz, Chloroform-d) δ 5.61 (s, 1H), 5.43 - 5.28 (m, 4H), 4.24 - 4.11 (m, 6H), 4.06 (m, 1H), 3.96 (m, 1H), 2.87 (d, J = 11.1 Hz, 1H), 2.77 (t, J = 6.6 Hz, 2H), 2.58 (t, J = 6.5 Hz, 1H), 2.44 (m, 1H), 2.30 (d, J = 6.9 Hz, 4H), 2.17 (s, 7H), 2.13 (d, J = 8.2 Hz, 2H), 2.05 (m, 5H), 1.72 (d, J = 11.6 Hz, 3H), 1.46 -1.40 (m, 3H), 1.38 - 1.25 (m, 24H), 0.93 - 0.86 (m, 9H).

### Example 74: Preparation of 3-((((1-methylpiperidin-3-yl)methoxy)carbonyl)oxy)-2-((((E)-3-pentylundec-2-enoyl)oxy)methyl)propyl (9Z,12Z)-octadeca-9,12-dienoate (CPL-377)

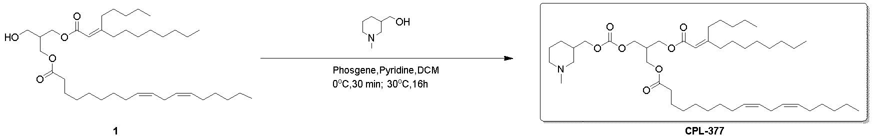

A mixture of 3-((E)-3-pentylundec-2-enoate)-2-(hydroxymethyl)propyl-(9Z,12Z)-octadeca-9,12-dienoate (200 mg, 0.33 mmol, 1.0 equivalent), pyridine (261 mg, 3.3 mmol, 10.0 equivalent) and triphosgene (40 mg, 0.13 mmol, 0.4 equivalent) in dichloromethane (3 mL) was stirred at 0°C for 30 minutes. A solution of 1-methylpiperidine-3-methanol (185 mg, 0.99 mmol, 3.0 equivalent) in dichloromethane (1 mL) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-377 (22 mg, 8% yield) as a colorless oil.

LC/MS (ESI) m/z: 760.6 (M+H)⁺.

1H NMR (400 MHz, CDCl3) δ 5.61 (s, 1H), 5.44 - 5.27 (m, 4H), 4.25 - 4.11 (m, 6H), 4.11- 4.03 (m, 1H), 3.99 - 3.89 (m, 1H), 2.88 - 2.71 (m, 4H), 2.61 - 2.55 (m, 2H), 2.48 - 2.40 (m, 1H), 2.35 - 2.30 (m, 2H), 2.26 (s, 3H), 2.18 - 2.10 (m, 2H), 2.09 - 1.97 (m, 4H), 1.77 - 1.66 (m, 4H), 1.45 - 1.28 (m, 35H), 1.05 - 0.99 (m, 2H), 0.94 - 0.85 (m, 9H).

### Example 75: Preparation of 3-((((1-methylpiperidin-3-yl)methoxy)carbonyl)oxy)-2-((((E)-3-pentyltridec-2-enoyl)oxy)methyl)propyl (9Z,12Z)-octadeca-9,12-dienoate (CPL-378)

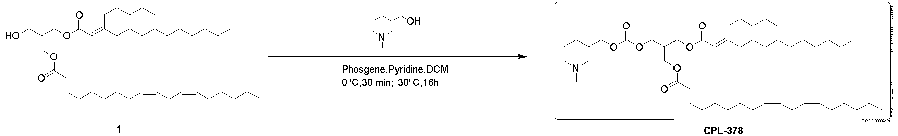

A mixture of 3-((E)-3-pentyltridec-2-enoate)-2-(hydroxymethyl)propyl-(9Z,12Z)-octadeca-9,12-dienoate (200 mg, 0.31 mmol, 1.0 equivalent), pyridine (250 mg, 3.1 mmol, 10.0 equivalent) and triphosgene (38 mg, 0.12 mmol, 0.4 equivalent) in dichloromethane (3 mL) was stirred at 0°C for 30 minutes. A solution of 1-methylpiperidine-3-methanol (125 mg, 0.95 mmol, 3.0 equivalent) in dichloromethane (1 mL) was added and stirred at room temperature for 16 hours. 10 mL of water was added and extracted three times with 10 mL of dichloromethane. The organic phase was washed three times with 20 mL of sodium chloride solution, filtered and dried over sodium sulfate. The mixture was concentrated and purified by preparative HPLC to obtain the target CPL-378 (81 mg, 32% yield) as a colorless oil.

LC/MS (ESI) m/z: 788.6 (M+H)⁺.

1H NMR (400 MHz, CDCl3) δ 5.60 (s, 1H), 5.44 - 5.28 (m, 4H), 4.28 - 4.11 (m, 6H), 4.10 - 3.89 (m, 2H), 2.88 - 2.69 (m, 4H), 2.64 - 2.54 (m, 2H), 2.48 - 2.42 (m, 1H), 2.36 - 2.29 (m, 2H), 2.27 (s, 3H), 2.18 - 2.10 (m, 2H), 2.09 - 1.96 (m, 5H), 1.96 - 1.85 (m, 1H), 1.77 - 1.66 (m, 4H), 1.45 - 1.22 (m, 37H), 1.08 - 0.93 (m, 2H), 0.92 - 0.83 (m, 9H).

### Example 76: Preparation of 3-(((E)-3-hexyltridec-2-enoyl)oxy)-2-(((((1-methylpiperidin-3-yl)methoxy)carbonyl)oxy)methyl)propyl (9Z,12Z)-octadeca-9,12-dienoate (CPL-381)

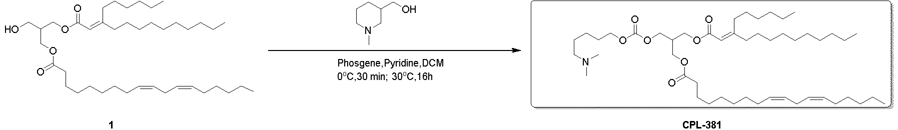

A mixture of 3-((E)-3-hexyltridec-2-enoate)-2-(hydroxymethyl)propyl-(9Z,12Z)-octadeca-9,12-dienoate (150 mg, 0.23 mmol, 1.0 equivalent), pyridine (184 mg, 2.3 mmol, 10.0 equivalent) and triphosgene (28 mg, 0.09 mmol, 0.4 equivalent) in dichloromethane (3 mL) was stirred at 0°C for 30 minutes. A solution of 1-methylpiperidine-3-methanol (92 mg, 0.69 mmol, 3.0 equivalent) in dichloromethane (1 mL) was added and stirred at room temperature for 16 hours. 10 mL of water was added and extracted three times with 10 mL of dichloromethane. The organic phase was washed three times with 20 mL of sodium chloride solution, filtered and dried over sodium sulfate. The mixture was concentrated and purified by preparative HPLC to obtain the target CPL-381 (52 mg, 28% yield) as a colorless oil.

LC/MS (ESI) m/z: 802.6 (M+H)⁺.

1H NMR (400 MHz, CDCl3) δ 5.60 (s, 1H), 5.44 - 5.28 (m, 4H), 4.27 - 4.19 (m, 2H), 4.19 - 4.11 (m, 4H), 4.09 - 4.01 (m, 1H), 4.01 - 3.92 (m, 1H), 2.88 - 2.70 (m, 4H), 2.62 - 2.53 (m, 2H), 2.51 - 2.39 (m, 1H), 2.38 - 2.28 (m, 2H), 2.28 (s, 3H), 2.16 - 2.11 (m, 2H), 2.08 - 1.86 (m, 6H), 1.80 - 1.66 (m, 4H), 1.46 - 1.26 (m, 38H), 1.07 - 0.85 (m, 12H).

### Example 77: Preparation of 3-(((2-(dimethylamino)ethoxy)carbonyl)oxy)-2-((((E)-3-hexyldec-2-enoyl)oxy)methyl)propyl (9Z,12Z)-octadeca-9,12-dienoate (CPL-383)

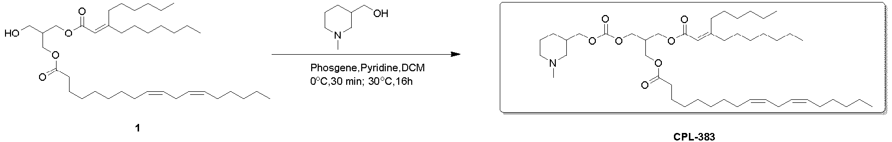

A mixture of 3-((E)-3-hexyldec-2-enoate)-2-(hydroxymethyl)propyl-(9Z,12Z)-octadeca-9,12-dienoate (200 mg, 0.33 mmol, 1.0 equivalent), pyridine (260.7 mg, 3.3 mmol, 10.0 equivalent) and triphosgene (40 mg, 0.13 mmol, 0.4 equivalent) in dichloromethane (2 mL) was stirred at 0°C for 30 minutes. 1-Methylpiperidine-3-methanol (129 mg, 1.04 mmol, 3.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-383 (25.83 mg, 6% yield) as a colorless oil.
LCMS (ESI) m/z:760.59(M+H)⁺.
1H NMR(400 MHz, CDCl3) δ 5.60 (s, 1H), 5.44 - 5.25 (m, 4H), 4.17-3.93 (m, 8H), 2.89 - 2.67 (m, 4H), 2.61 - 2.54 (m, 1H), 2.49 - 2.39 (m, 1H), 2.33 - 2.24 (m, 5H), 2.19 - 1.80 (m, 10H), 1.78 - 1.66 (m, 4H), 1.32 - 1.25 (m, 32H), 0.91-0.85 (m, 9H)

### Example 78: Preparation of 3-(((Z)-3-butylnon-2-enoyl)oxy)-2-((((3-(4-methylpiperazin-1-yl)propoxy)carbonyl)oxy)methyl)propyl (9Z,12Z)-octadeca-9,12-dienoate (CPL-394)

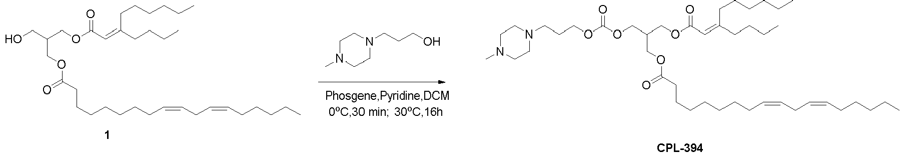

A mixture of 3-((E)-3-butylnon-2-enoate)-2-(hydroxymethyl)propyl-(9Z,12Z)-octadeca-9,12-dienoate (150 mg, 0.267 mmol, 1.0 equivalent), pyridine (212.2 mg, 2.67 mmol, 10.0 equivalent) and triphosgene (31.69 mg, 0.107 mmol, 0.4 equivalent) in dichloromethane (2 mL) was stirred at 0°C for 30 minutes. A solution of 1-(3-hydroxypropyl)-4-methylpiperazine (69.89 mg, 0.533 mmol, 2.0 equivalent) in dichloromethane (1 mL) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-394 (30.22 mg, 15.2% yield) as a colorless oil.

LCMS (ESI) m/z: 747.58 (M+H)⁺.

1H NMR (400 MHz, Chloroform-d) δ 5.61 (s, 1H), 5.42 - 5.28 (m, 4H), 4.25 - 4.11 (m, 8H), 2.77 (t, J = 6.3 Hz, 2H), 2.63 - 2.39 (m, 11H), 2.35 - 2.27 (m, 5H), 2.14 (t, J = 7.6 Hz, 2H), 2.05 (m, 4H), 1.87 (m, 2H), 1.48 - 1.40 (m, 4H), 1.40 - 1.22 (m, 26H), 0.89 (m, 9H).

### Example 79: Preparation of 3-(((3-(4-methylpiperazin-1-yl)propoxy)carbonyl)oxy)-2-((((E)-3-pentylnonan-2-enoyl)oxy)methyl)propyl (9Z,12Z)-octadeca-9,12-dienoate (CPL-398)

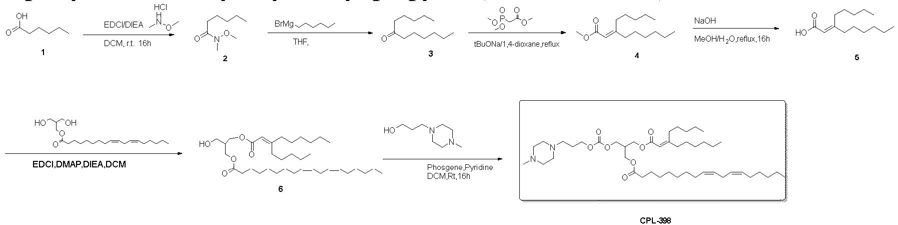

A mixture of 3-((E)-3-pentylnon-2-enoate)-2-(hydroxymethyl)propyl-(9Z,12Z)-octadeca-9,12-dienoate (200 mg, 0.3472 mmol, 1.0 equivalent), 1-(3-hydroxypropyl)-4-methylpiperazine (110 mg, 0.6944 mmol, 2.0 equivalent), pyridine (274 mg, 3.472 mmol, 10.0 equivalent) and triphosgene (41 mg, 0.1389 mmol, 0.4 equivalent) in dichloromethane (8 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-398 (25.79 mg, 9.8% yield) as a colorless oil.

LC/MS (ESI) m/z: 761.6 (M+H)⁺.

1HNMR(400 MHz, CDCl3) δ 5.54 (s, 1H), 5.33 - 5.22 (m, 4H), 4.15 - 4.07 (m, 8H), 2.70 (t,J = 6.6 Hz, 2H), 2.53 - 2.48 (m, 2H), 2.43 - 2.31 (m, 8H), 2.27 - 2.20 (m, 6H), 2.09 - 2.03 (m, 2H), 1.98 (q,J = 6.4 Hz, 4H), 1.79 (dd,J = 14.2, 6.8 Hz, 2H), 1.39 - 1.34 (m, 4H), 1.24 - 1.18 (m, 28H), 0.82 (dd,J = 6.9, 4.4 Hz, 9H).

### Example 80: Preparation of 3-(((E)-3-heptyltridec-2-enoyl)oxy)-2-((((3-(4-methylpiperazin-1-yl)propoxy)carbonyl)oxy)methyl)propyl (9Z,12Z)-octadeca-9,12- dienoate (CPL-407)

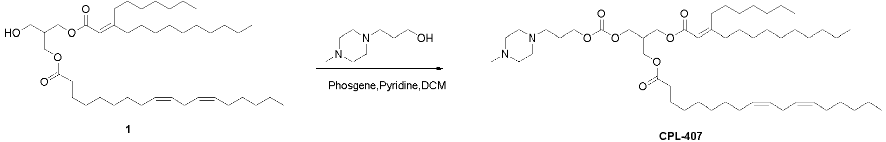

A mixture of 3-((E)-3-heptyltridec-2-enoate)-2-(hydroxymethyl)propyl-(9Z,12Z)-octadeca-9,12-dienoate (200 mg, 0.303 mmol, 1.0 equivalent), pyridine (239.67 mg, 3.03 mmol, 10.0 equivalent) and triphosgene (35.9 mg, 0.121 mmol, 0.4 equivalent) in dichloromethane (5 mL) was stirred at 0°C for 30 minutes. 1-(3-hydroxypropyl)-4-methylpiperazine (95.83 mg, 0.606 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-407 (21.72 mg, 8.48% yield) as a colorless oil.

LCMS (ESI) m/z: 845.69 (M+H)⁺.

1H NMR (400 MHz, Chloroform-d) δ 5.60 (s, 1H), 5.41 - 5.30 (m, 4H), 4.22 - 4.14 (m, 8H), 2.77 (t, J = 6.5 Hz, 2H), 2.57 (t, J = 7.8 Hz, 7H), 2.45 (m, 4H), 2.34 (d, J = 10.7 Hz, 3H), 2.29 (d, J = 7.6 Hz, 2H), 2.16 - 2.10 (m, 2H), 2.05 (m, 4H), 1.87 (m, 2H), 1.44 (d, J = 7.3 Hz, 4H), 1.36 - 1.24 (m, 40H), 0.91 - 0.86 (m, 9H).

### Example 81: Preparation of 3-(((3-(4-methylpiperazin-1-yl)propoxy)carbonyl)oxy)-2-((((Z)-3-octyltridec-2-enoyl)oxy)methyl)propyl (9Z,12Z)-octadeca-9,12-dienoate (CPL-409)

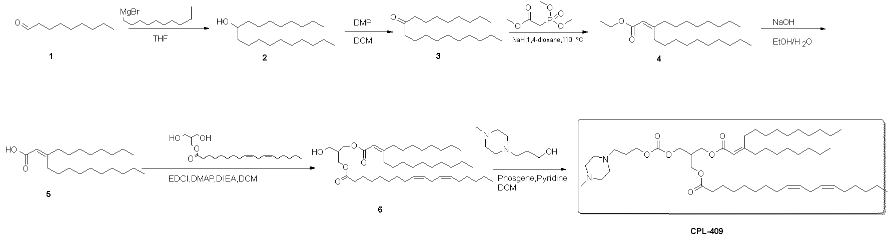

A mixture of 3-((E)-3-octyltridec-2-enoate)-2-(hydroxymethyl)propyl-(9Z,12Z)-octadeca-9,12-dienoate (190 mg, 0.2819 mmol, 1.0 equivalent), 1-(3-hydroxypropyl)-4-methylpiperazine (89 mg, 0.5638 mmol, 2.0 equivalent), pyridine (223 mg, 2.819 mmol, 10.0 equivalent) and triphosgene (33 mg, 0.1128 mmol, 0.4 equivalent) in dichloromethane (8 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-409 (23.57 mg, 10% yield) as a colorless oil.

LC/MS (ESI) m/z: 859.7 (M+H)⁺.

1H NMR (400 MHz, CDCl3) δ 5.53 (s, 1H), 5.34 - 5.22 (m, 4H), 4.18 - 4.03 (m, 8H), 2.70 (t, J = 6.5 Hz, 2H), 2.53 - 2.48 (m, 4H), 2.38 (dd, J = 14.3, 6.7 Hz, 4H), 2.28 - 2.21 (m, 5H), 2.10 - 2.03 (m, 2H), 1.98 (q, J = 6.8 Hz, 4H), 1.83 - 1.74 (m, 2H), 1.58 - 1.49 (m, 3H), 1.37 (d, J = 7.9 Hz, 4H), 1.25 - 1.17 (m, 42H), 0.83 - 0.79 (m, 9H).

### Example 82: Preparation of 3-(((Z)-3-butylnon-2-enoyl)oxy)-2-((((3-(piperidin-1-yl)propoxy)carbonyl)oxy)methyl)propyl (9Z,12Z)-octadeca-9,12-dienoate (CPL-416)

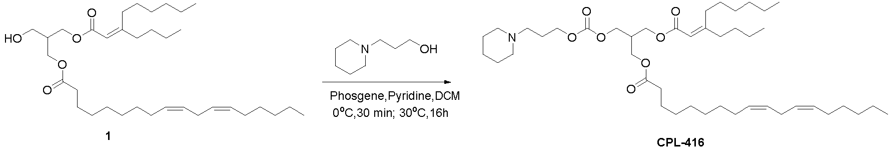

A mixture of 3-((E)-3-butylnon-2-enoate)-2-(hydroxymethyl)propyl-(9Z,12Z)-octadeca-9,12-dienoate (150 mg, 0.267 mmol, 1.0 equivalent), pyridine (212.12 mg, 2.67 mmol, 10.0 equivalent) and triphosgene (31.69 mg, 0.107 mmol, 0.4 equivalent) in dichloromethane (2 mL) was stirred at 0°C for 30 minutes. 1-Piperidinepropanol (76.28 mg, 0.533 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-416 (29.03 mg, 14.9% yield) as a colorless oil.

LCMS (ESI) m/z: 732.57 (M+H)⁺.

1H NMR (400 MHz, Chloroform-d) δ 5.61 (s, 1H), 5.35 (m, 4H), 4.24 - 4.11 (m, 8H), 2.77 (t, J = 6.5 Hz, 2H), 2.60 - 2.56 (m, 1H), 2.51 - 2.35 (m, 6H), 2.30 (t, J = 7.6 Hz, 2H), 2.14 (m, 2H), 2.05 (q, J = 6.9 Hz, 4H), 1.90 (t, J = 7.2 Hz, 2H), 1.58 - 1.19 (m, 36H), 0.89 (m, 9H).

### Example 83: Preparation of 3-(((E)-3-pentylundec-2-enoyl)oxy)-2-((((3-(piperidin-1-yl)propoxy)carbonyl)oxy)methyl)propyl (9Z,12Z)-octadeca-9,12-dienoate (CPL-421)

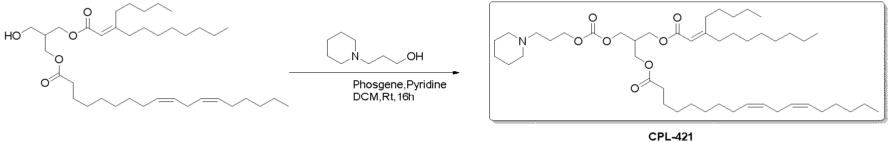

A mixture of 3-((E)-3-pentylundec-2-enoate)-2-(hydroxymethyl)propyl-(9Z,12Z)-octadeca-9,12-dienoate (200 mg, 0.3306 mmol, 1.0 equivalent), 1-piperidinylpropanol (142.06 mg, 0.9918 mmol, 3.0 equivalent), pyridine (261.51 mg, 3.31 mmol, 10.0 equivalent) and triphosgene (39.24 mg, 0.1322 mmol, 0.4 equivalent) in dichloromethane (5 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-421 (86.45 mg, 33.78% yield) as a colorless oil.

LC/MS (ESI) m/z: 774.6 (M+H)⁺.

1H NMR (400 MHz, CDCl3) δ 5.60 (s, 1H), 5.35 (qd, J = 8.4, 2.7 Hz, 4H), 4.21 - 4.14 (m, 8H), 2.76 (d, J = 6.8 Hz, 2H), 2.60 - 2.53 (m, 2H), 2.34 (t, J = 15.8 Hz, 6H), 2.13 - 2.03 (m, 6H), 1.88 (s, 2H), 1.54 - 1.27 (m, 42H), 0.91 - 0.87 (m, 10H).

### Example 84: Preparation of 3-(((E)-3-heptylundec-2-enoyl)oxy)-2-((((3-(piperidin-1-yl)propoxy)carbonyl)oxy)methyl)propyl (9Z,12Z)-octadeca-9,12-dienoate (CPL-428)

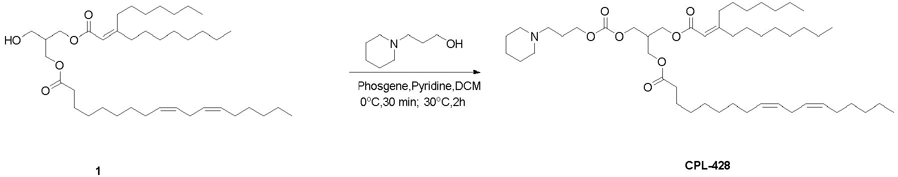

A mixture of 3-((E)-3-heptylundec-2-enoate)-2-(hydroxymethyl)propyl-(9Z,12Z)-octadeca-9,12-dienoate (200 mg, 0.32 mmol, 1.0 equivalent), pyridine (253.12 mg, 3.2 mmol, 10.0 equivalent) and triphosgene (35.60 mg, 0.12 mmol, 0.4 equivalent) in dichloromethane (3 mL) was stirred at 0°C for 30 minutes. 1-Piperidinepropanol (90.23 mg, 0.63 mmol, 2.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-428 (33.21 mg, 12.93% yield) as a colorless oil.

LCMS (ESI) m/z: 803.7 (M+H)⁺.

1H NMR (400 MHz, CDCl3) δ 5.60 (d, J = 5.8 Hz, 1H), 5.42 - 5.28 (m, 4H), 4.28 - 4.03 (m, 8H), 2.77 (t, J = 6.4 Hz, 2H), 2.63 - 2.54 (m, 2H), 2.54 - 2.34 (m, 6H), 2.34 - 2.19 (m, 3H), 2.17 - 2.09 (m, 2H), 2.07 - 2.01 (m, 4H), 1.84 (ddd, J = 62.8, 46.7, 40.0 Hz, 4H), 1.60 - 1.20 (m, 42H), 0.88 (dd, J = 6.9, 4.2 Hz, 9H).

### Example 85: Preparation of 3-((((1-ethylpiperidin-4-yl)methoxy)carbonyl)oxy)-2-((((E)-3-hexyldec-2-enoyl)oxy)methyl)propyl (9Z,12Z)-octadeca-9,12-dienoate (CPL-449)

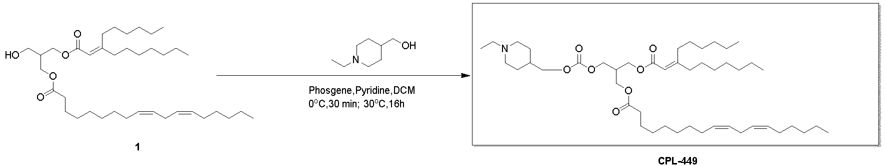

A mixture of 3-((E)-3-hexyldec-2-enoate)-2-(hydroxymethyl)propyl-(9Z,12Z)-octadeca-9,12-dienoate (200 mg, 0.33 mmol, 1.0 equivalent), pyridine (260.7 mg, 3.3 mmol, 10.0 equivalent) and triphosgene (40 mg, 0.13 mmol, 0.4 equivalent) in dichloromethane (3 mL) was stirred at 0°C for 30 minutes. 1-Piperidinepropanol (143.23 mg, 1.04 mmol, 3.0 equivalent) was added and stirred at room temperature for 16 hours. 30 mL of water was added and extracted three times with 20 mL of dichloromethane. The organic phase was washed three times with 30 mL of sodium chloride solution, filtered and dried over sodium sulfate. The mixture was concentrated and purified by preparative HPLC to obtain the target CPL-449 (50.72 mg, 39% yield) as a colorless oil.

LCMS (ESI) m/z: 774.18(M+H)⁺.

1 H NMR (400 MHz, CDCl3) δ 5.60 (s, 1H), 5.41 - 5.26 (m, 4H), 4.22-4.14 (m, 6H), 3.98 (d, J = 6.4 Hz, 2H), 2.96 (d, J = 14.0 Hz, 2H), 2.77 (t, J = 6.4 Hz, 2H), 2.61 - 2.54 (m, 1H), 2.47 - 2.37 (m, 3H), 2.29 (d, J = 7.7 Hz, 2H), 2.16 - 1.98 (m, 6H), 1.89 (t, J = 10.8 Hz, 2H), 1.75 (d, J = 13.2 Hz, 2H), 1.61 (s, 6H), 1.38 - 1.23 (m, 38H), 1.08 (t, J = 7.2 Hz, 4H), 0.91-0.83 (m, 9H).

### Example 86: Preparation of 3-(((1-ethylpiperidin-4-methoxy)carbonyl)oary)-2-((((Z)-3-octyltridec-2-enoyl))oxy)methyl)propyl (9Z,12Z)-octadeca-9,12-dienoate (CPL-453)

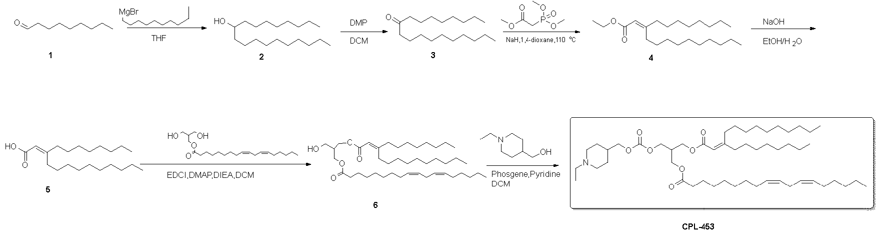

A mixture of 3-((E)-3-octyltridec-2-enoate)-2-(hydroxymethyl)propyl-(9Z,12Z)-octadeca-9,12-dienoate (200 mg, 0.2967 mmol, 1.0 equivalent), N-ethylpiperidine-1-methanol (85 mg, 0.5935 mmol, 3.0 equivalent), pyridine (234 mg, 2.967 mmol, 10.0 equivalent) and triphosgene (35 mg, 0.1187 mmol, 0.4 equivalent) in dichloromethane (8 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-453 (44.16 mg, 18% yield) as a colorless oil.

LC/MS (ESI) m/z: 844.7 (M+H)⁺.

1HNMR(400 MHz, CDCl3) δ 5.53 (s, 1H), 5.35 - 5.22 (m, 4H), 4.17 - 4.04 (m, 6H), 3.92 (d,J = 6.4 Hz, 2H), 2.70 (t,J = 6.5 Hz, 2H), 2.57 - 2.46 (m, 2H), 2.37 (dt,J = 11.9, 5.9 Hz, 2H), 2.22 (dd,J = 17.7, 10.1 Hz, 2H), 2.09 - 2.02 (m, 2H), 1.98 (dd,J = 13.6, 6.7 Hz, 4H), 1.65 (d,J = 30.6 Hz, 4H), 1.37 (d,J = 6.7 Hz, 4H), 1.32 - 1.17 (m, 46H), 1.05 (s, 3H), 0.84 - 0.80 (m, 9H).

### Example 87: Preparation of 3-((3-decyltridec-2-enoyl)oxy)-2-(((((1-ethylpiperidin-4-yl)methoxy)carbonyl)oxy)methyl)propyl (9Z,12Z)-octadeca-9,12-dienoate (CPL-454)

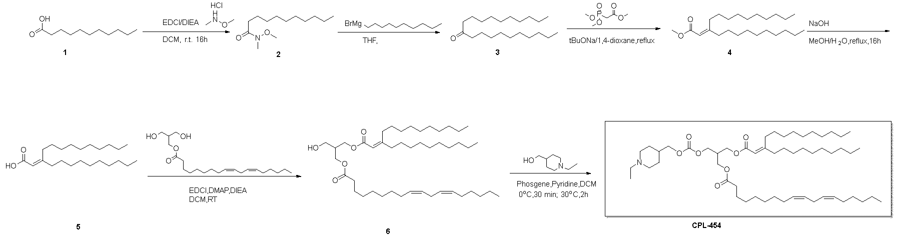

A mixture of 3-((E)-3-decyltridec-2-enoate)-2-(hydroxymethyl)propyl-(9Z,12Z)-octadeca-9,12-dienoate (200 mg, 0.2849 mmol, 1.0 equivalent), N-ethylpiperidine-1-methanol (81 mg, 0.5698 mmol, 2.0 equivalent), pyridine (225 mg, 2.849 mmol, 10.0 equivalent) and triphosgene (34 mg, 0.1114 mmol, 0.4 equivalent) in dichloromethane (6 mL) was stirred at room temperature for 16 hours. 40 mL of water was added and extracted three times with 20 mL of dichloromethane. The organic phase was washed three times with 40 mL of sodium chloride solution, filtered and dried over sodium sulfate. The mixture was concentrated and purified by preparative HPLC to obtain the target CPL-454 (35.65 mg, 14% yield) as a colorless oil.

LC/MS (ESI) m/z: 873.7 (M+H)⁺.

1HNMR(400 MHz, CDCl3) δ 5.53 (s, 1H), 5.35 - 5.21 (m, 4H), 4.19 - 4.04 (m, 6H), 3.92 (d,J = 6.5 Hz, 2H), 2.70 (t,J = 6.5 Hz, 2H), 2.56 - 2.46 (m, 2H), 2.41 - 2.33 (m, 2H), 2.22 (dd,J = 17.5, 9.9 Hz, 2H), 2.09 - 2.03 (m, 2H), 1.98 (q,J = 6.6 Hz, 4H), 1.66 (t,J = 19.1 Hz, 4H), 1.37 (dd,J = 12.1, 4.5 Hz, 4H), 1.31 - 1.17 (m, 50H), 1.03 (t,J = 6.9 Hz, 3H), 0.83 - 0.79 (m, 9H).

### Example 88: Preparation of 5-((5-(E)-3-butyltridec-2-enoyl)oxy)pentyl((3-hydroxypropyl)amino)pentyl (E)-3-propyl-2-tridecenoate (CPL-1092)

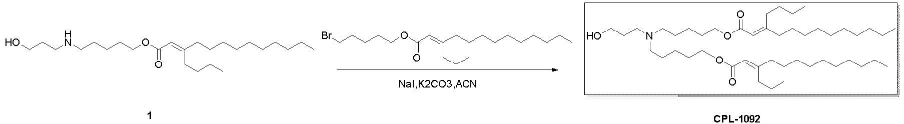

A mixture of compound (E)-5-bromopentyl-3-propyltridec-2-enoate (108 mg, 0.27 mmol, 1.1 equivalent), 5-(3-hydroxypropylamino)-pentyl-3-butyltridec-2-enoate (100 mg, 0.24 mmol, 1.0 equivalent), K₂CO₃ (67 mg, 0.49 mmol, 2.0 equivalent) and Nal (1.8 mg, 0.01 mmol, 0.05 equivalent) in ACN (10 mL) was stirred at 85°C for 16 hours. 40 mL of water was added and extracted three times with 20 mL of ethyl acetate. The organic phase was washed three times with 40 mL of sodium chloride solution, filtered and dried over sodium sulfate. The mixture was concentrated and purified by preparative HPLC to obtain the target CPL-1092 (75.02 mg, 42.06% yield) as a colorless oil.

LC/MS (ESI) m/z: 734.7 (M+H) +.

1H NMR (400 MHz, CDCl3) δ 5.55 (s, 2H), 4.02-3.99 (m, 4H), 3.74-3.71 (m, 2H), 2.52-2.39 (m, 10H), 2.10-2.05 (m, 4H), 1.63 - 1.56 (m, 6H), 1.45-1.23 (m, 46H), 0.87-0.81 (m, 12H).

### Example 89: Preparation of 10-(((5-(E)-3-butyltridec-2-enoyl)oxy)pentyl)((3-hydroxypropyl)amino)decyl (E)-3-propyl-2-tridecenoate (CPL-1093)

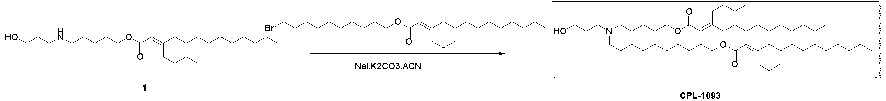

A mixture of compound (E)-10-bromodecyl-3-propyltridec-2-enoate (126 mg, 0.27 mmol, 1.1 equivalent), 5-(3-hydroxypropylamino)-pentyl-3-butyltridec-2-enoate (100 mg, 0.24 mmol, 1.0 equivalent), K₂CO₃ (67 mg, 0.49 mmol, 2.0 equivalent) and Nal (1.8 mg, 0.01 mmol, 0.05 equivalent) in ACN (10 mL) was stirred at 80°C overnight. 40 mL of water was added and extracted three times with 20 mL of ethyl acetate. The organic phase was washed three times with 40 mL of sodium chloride solution, filtered and dried over sodium sulfate. The mixture was concentrated and purified by preparative HPLC to obtain the target CPL-1093 (80.54 mg, 41.30% yield) as a colorless oil.

LC/MS (ESI) m/z: 805.7 (M+H) +.

1H NMR (400 MHz, CDCl3) δ 5.55 (d, J = 4.8 Hz, 2H), 4.02-3.98 (m, 4H), 3.74-3.72 (m, 2H), 2.64-2.41 (m, 10H), 2.10-1.97 (m, 4H), 1.65 - 1.25 (m, 62H), 0.85 - 0.80 (m, 12H).

### Test Examples

The properties of the cationic lipids as prepared in the above examples were tested according to the following methods.

### Preparation and characterization of lipid nanoparticles

Lipid nanoparticles (LNPs) were prepared using the cationic lipids disclosed in the present invention according to the following steps, taking mRNA as an encapsulated drug as an example.
1. The cationic lipid (50 mg/mL) prepared in the above examples of the present disclosure, cholesterol (20 mg/mL), DSPC (20 mg/mL) and DMG-PEG2000 (20 mg/mL) were dissolved in ethanol, and the solution was placed in a 55°C metal thermostatic bath as needed to aid dissolution, to obtain a lipid mixture with the following molar composition: 1.5% DMG-PEG2000, 50% cationic lipid, 38.5% cholesterol, and 10% DSPC.
2. The mRNA encoding Ffluc was diluted with 10 mM pH 4.0 citrate buffer to prepare a 0.2 mg/mL mRNA solution.
3. The lipid mixture prepared in step 1 was mixed with the mRNA solution prepared in step 2 using a syringe pump with a tee at a total flow rate of 40 mL/min.
4. The LNP mixture was collected after the three-way mixing and placed in a dialysis bag with a molecular weight cut-off of 100 KDa.
5. The LNP mixture was dialyzed overnight against 50 mM Tris-buffered saline, pH 7.4.
6. Sieving, and subsequent measurement of the sieved LNP to measure the PDI of the LNP by dynamic laser light scattering; to measure the mRNA encapsulation efficiency and concentration by Ribogreen assay; and to measure the pKa by TNS-α assay.

The results are shown in Table 1 below.

**Table 1 Performance Parameters Of Lipid Nanoparticles (LNPs) Made From Cationic Lipids Of The Present Disclosure**

| Example No. | Compound No. | Size (nm) | PDI | Encapsulation Rate |
|---|---|---|---|---|
| 1 | CPL-001 | 62.9 | 0.0594 | 96% |
| 2 | CPL-062 | 67.12 | 0.0828 | 98% |
| 3 | CPL-069 | 84.59 | 0.126 | 97% |
| 4 | CPL-092 | 98.6 | 0.21 | 97% |
| 5 | CPL-113 | 97.7 | 0.24 | 96% |
| 6 | CPL-133 | 80.25 | 0.0569 | 97% |
| 7 | CPL-174 | 112 | 0.18 | 97% |
| 8 | CPL-180 | 66.8 | 0.1334 | 86% |
| 9 | CPL-455 | 69.76 | 0.1065 | 85% |
| 10 | CPL-456 | 70.36 | 0.1225 | 85% |
| 11 | CPL-457 | 73 | 0.078 | 90% |
| 13 | CPL-009 | 98 | 0.205 | 92% |
| 14 | CPL-021 | 69.64 | 0.0729 | 95% |
| 15 | CPL-025 | 92.1 | 0.2 | 97% |
| 16 | CPL-026 | 71.34 | 0.2143 | 95% |
| 17 | CPL-031 | 92.39 | 0.2448 | 94% |
| 18 | CPL-032 | 90.11 | 0.1692 | 91% |
| 19 | CPL-039 | 97.61 | 0.2202 | 94% |
| 20 | CPL-043 | 77.48 | 0.1723 | 95% |
| 21 | CPL-044 | 78.4 | 0.15 | 98% |
| 22 | CPL-047 | 114.7 | 0.25 | 96% |
| 23 | CPL-048 | 110.1 | 0.2409 | 94% |
| 24 | CPL-049 | 101.6 | 0.1651 | 90% |
| 25 | CPL-052 | 90.76 | 0.1162 | 93% |
| 26 | CPL-061 | 94.04 | 0.1612 | 94% |
| 27 | CPL-065 | 83.7 | 0.1898 | 95% |
| 28 | CPL-066 | 87.2 | 0.19 | 98% |
| 29 | CPL-070 | 100.4 | 0.2173 | 95% |
| 30 | CPL-071 | 88.06 | 0.1733 | 92% |
| 31 | CPL-074 | 95.7 | 0.3691 | 99% |
| 32 | CPL-079 | 94.46 | 0.1557 | 94% |
| 33 | CPL-090 | 85.28 | 0.1615 | 94% |
| 34 | CPL-096 | 71.62 | 0.1973 | 95% |
| 35 | CPL-101 | 69.38 | 0.0791 | 95% |
| 36 | CPL-112 | 85.04 | 0.1173 | 95% |
| 37 | CPL-127 | 94.43 | 0.0486 | 92% |
| 38 | CPL-136 | 92.9 | 0.2 | 98% |
| 39 | CPL-148 | 90.31 | 0.1437 | 93% |
| 40 | CPL-152 | 72.18 | 0.05 | 95% |
| 41 | CPL-184 | 84.6 | 0.173 | 94% |
| 42 | CPL-199 | 86.92 | 0.1394 | 96% |
| 44 | CPL-208 | 96.3 | 0.2382 | 95% |
| 45 | CPL-210 | 52.37 | 0.2144 | 83% |
| 46 | CPL-218 | 114.4 | 0.1194 | 87% |
| 47 | CPL-221 | 82.01 | 0.0453 | 96% |
| 48 | CPL-222 | 72.01 | 0.0629 | 96% |
| 49 | CPL-223 | 99.55 | 0.102 | 98% |
| 50 | CPL-224 | 90.88 | 0.1162 | 94% |
| 51 | CPL-231 | 69.42 | 0.1118 | 95% |
| 53 | CPL-252 | 86.5 | 0.1369 | 98% |
| 54 | CPL-256 | 83.7 | 0.1009 | 95% |
| 55 | CPL-266 | 68.11 | 0.0481 | 97% |
| 56 | CPL-274 | 78.29 | 0.1812 | 97% |
| 57 | CPL-280 | 119.6 | 0.2607 | 97% |
| 58 | CPL-289 | 83 | 0.1371 | 97% |
| 59 | CPL-290 | 105.5 | 0.1615 | 98% |
| 60 | CPL-292 | 90.31 | 0.1816 | 97% |
| 62 | CPL-309 | 76.44 | 0.0937 | 93% |
| 63 | CPL-316 | 75.82 | 0.1387 | 93% |
| 64 | CPL-322 | 96.21 | 0.1302 | 94% |
| 65 | CPL-324 | 99.42 | 0.1961 | 95% |
| 66 | CPL-329 | 75.57 | 0.0596 | 97% |
| 67 | CPL-338 | 83.4 | 0.1084 | 99% |
| 68 | CPL-343 | 116.3 | 0.1584 | 85% |
| 69 | CPL-348 | 63.86 | 0.1929 | 91% |
| 70 | CPL-351 | 69.64 | 0.1225 | 82% |
| 73 | CPL-372 | 90.88 | 0.1182 | 98% |
| 74 | CPL-377 | 88.31 | 0.1248 | 94% |
| 75 | CPL-378 | 82.38 | 0.1099 | 96% |
| 76 | CPL-381 | 83.86 | 0.1174 | 91% |
| 77 | CPL-383 | 99.82 | 0.1733 | 97% |
| 78 | CPL-394 | 95.77 | 0.1069 | 97% |
| 79 | CPL-398 | 115.7 | 0.0778 | 97% |
| 80 | CPL-407 | 98.86 | 0.0562 | 97% |
| 81 | CPL-409 | 94.97 | 0.1474 | 96% |
| 82 | CPL-416 | 91.52 | 0.009 | 96% |
| 83 | CPL-421 | 82.69 | 0.1904 | 94% |
| 84 | CPL-428 | 73.57 | 0.1411 | 96% |
| 85 | CPL-449 | 73.57 | 0.0875 | 97% |
| 86 | CPL-453 | 81.2 | 0.0653 | 98% |
| 87 | CPL-454 | 83.51 | 0.1315 | 99% |
| 88 | CPL-1092 | 117.0 | 0.20 | 99% |
| 89 | CPL-1093 | 112.08 | 0.21 | 99% |

As can be seen from the results in the above table, the LNPs made from the cationic lipids of the present disclosure have excellent drug encapsulation rate and can effectively achieve drug encapsulation for targeted delivery in vivo.

### Animal Studies

The prepared encapsulation solution was applied to mice in the following manner, and its delivery efficiency in mice was tested. Among them, DLin-MC3-DMA (MC3) was a cationic liposome for delivering small nucleic acids that was approved by the FDA for marketing in 2018, which has high nucleic acid drug delivery efficiency and biosafety, and was usually used as the industry gold standard in the screening process of cationic lipids. In the evaluation of mice experiments, the ratio of nucleic acid delivery efficiency normalized to MC3 was greater than 0.1, which was considered to be a cationic lipid with high delivery efficiency.

### Intramuscular Administration

The LNP solution was diluted with 50 mM TBS, pH 7.4 to a concentration of 0.1 mg/mL mRNA.

2. The mice involved in this study were female Balb/C, weighing 18-22 g, 6-8 weeks old, with 5 mice in each group.

3. The dosing solution was kept at 4°C prior to injection.

4. 50 uL of LNP solution was injected into the right thigh of the mice.

5. The mice were injected intraperitoneally with 200 uL of 1.5 mg/mL D-luciferin solution 6 h after injection.

6. The in vivo luminescence images of the mice were obtained using the IVIS system (Aniview).

7. The luminescence intensity of each mice was measured using Aniview software.

8. MC3 lipid nanoparticles were used as a positive control. The in vivo efficacy of each cationic lipid tested was recorded as the fold change in luminescence intensity relative to MC3 (ratio normalized to MC3).

### Intravenous administration

1. The LNP solution was diluted with 50 mM TBS, pH 7.4 to a concentration of 0.1 mg/mL mRNA.
2. The mice involved in this study were female Balb/C, weighing 18-22 g, 6-8 weeks old, with 5 mice in each group.
3. The dosing solution was kept at 4°C prior to injection.
4. Each mice received 0.06 mg/kg via tail vein injection.
5. The mice were injected intraperitoneally with 200 uL of 1.5 mg/mL D-luciferin solution 6 h after injection.
6. The in vivo luminescence images of the mice were obtained using the IVIS system (Aniview).
7. The luminescence intensity of each mice was measured using Aniview software.
8. MC3 lipid nanoparticles were used as a positive control. The in vivo efficacy of each cationic lipid tested was recorded as the fold change in luminescence intensity relative to MC3 (ratio normalized to MC3).

The results are shown in Table 2 below.

**Table 2. In Vivo Delivery Efficacy Of Lipid Nanoparticles Prepared From Cationic Lipids Of The Present Disclosure**

| Example No. | Compound No. | In Vivo Efficacy Normalized To Mc3 In Mice (Intravenous Injection) | In Vivo Efficacy Normalized To Mc3 In Mice (Intramuscular Injection) |
|---|---|---|---|
| 1 | CPL-001-01 | 2.00 | - |
| 2 | CPL-062-01 | 2.21 | 1.06 |
| 3 | CPL-069-01 | 2.70 | 1.72 |
| 4 | CPL-092-01 | 8.96 | 2.20 |
| 5 | CPL-113-01 | 2.52 | 1.65 |
| 6 | CPL-133-01 | 3.54 | 6.61 |
| 7 | CPL-174-01 | 2.32 | 1.20 |
| 8 | CPL-180-01 | 9.31 | 0.72 |
| 9 | CPL-455-01 | - | - |
| 10 | CPL-456-01 | 7.36 | 5.51 |
| 11 | CPL-457-01 | 5.49 | 12.13 |
| 12 | CPL-002 | - | - |
| 13 | CPL-003 | 1.55 | - |
| 14 | CPL-021 | 0.75 | - |
| 15 | CPL-025 | 1.34 | - |
| 16 | CPL-026 | 2.51 | 0.92 |
| 17 | CPL-031 | 0.75 | - |
| 18 | CPL-032 | 1.18 | - |
| 19 | CPL-039 | 1.44 | - |
| 20 | CPL-043 | 1.58 | - |
| 21 | CPL-044 | 1.26 | - |
| 22 | CPL-047 | 1.59 | - |
| 23 | CPL-048 | 1.57 | - |
| 24 | CPL-049 | 1.88 | - |
| 25 | CPL-052 | 1.46 | - |
| 26 | CPL-061 | 1.06 | - |
| 27 | CPL-065 | 1.62 | - |
| 28 | CPL-066 | 1.95 | 4.04 |
| 29 | CPL-070 | 3.74 | 1.85 |
| 30 | CPL-071 | 2.05 | 7.81 |
| 31 | CPL-074 | 2.40 | 7.93 |
| 32 | CPL-079 | 4.95 | 1.30 |
| 33 | CPL-090 | 2.02 | - |
| 34 | CPL-096 | 2.06 | 2.59 |
| 35 | CPL-101 | 2.90 | 1.76 |
| 36 | CPL-112 | 2.14 | - |
| 37 | CPL-127 | 2.54 | 2.37 |
| 38 | CPL-136 | 2.81 | 1.61 |
| 39 | CPL-148 | 2.15 | 2.56 |
| 40 | CPL-152 | 2.03 | 2.62 |
| 41 | CPL-184 | 0.98 | - |
| 42 | CPL-199 | 0.12 | - |
| 43 | CPL-203 | 0.23 | - |
| 45 | CPL-210 | 0.14 | - |
| 46 | CPL-218 | 0.19 | - |
| 47 | CPL-221 | 0.44 | - |
| 48 | CPL-222 | 0.37 | - |
| 49 | CPL-223 | 0.41 | - |
| 50 | CPL-224 | 0.87 | - |
| 51 | CPL-231 | 0.55 | - |
| 52 | CPL-234 | 0.96 | - |
| 53 | CPL-252 | 0.34 | - |
| 54 | CPL-256 | 0.63 | - |
| 55 | CPL-266 | 0.72 | - |
| 56 | CPL-274 | 0.37 | - |
| 60 | CPL-292 | 0.27 | - |
| 65 | CPL-324 | 0.31 | - |
| 66 | CPL-329 | 0.33 | - |
| 67 | CPL-338 | 0.52 | - |
| 68 | CPL-343 | 0.44 | - |
| 73 | CPL-372 | 1.01 | - |
| 74 | CPL-377 | 0.45 | - |
| 75 | CPL-378 | 0.43 | - |
| 76 | CPL-381 | 0.81 | - |
| 77 | CPL-383 | 0.80 | - |
| 78 | CPL-394 | 0.13 | - |
| 80 | CPL-407 | 0.21 | - |
| 82 | CPL-416 | 0.43 | - |
| 83 | CPL-421 | 0.37 | - |
| 84 | CPL-428 | 0.34 | - |
| 85 | CPL-449 | 0.49 | - |
| 86 | CPL-453 | 0.82 | - |
| 87 | CPL-454 | 0.88 | - |
| 88 | CPL-1092 | 0.83 | - |
| 89 | CPL-1093 | 1.55 | - |

As can be seen from the results in the above table, the nucleic acid delivery efficiency of LNPs made of the cationic lipids of the present invention in mice was normalized to MC3 and was above 0.10, and could even reach up to 9.31. Therefore, the cationic lipids of the present invention achieved good or even excellent targeted delivery of nucleic acid drugs in vivo.

Although the nucleic acid delivery efficiency of cationic lipids in the present disclosure was tested using mRNA as an example, the nucleic acid drugs adapted by the cationic lipids of the present disclosure were not limited thereto, but can be used to effectively deliver various nucleic acid drugs including, but not limited to, DNA drugs, RNA drugs, or both.

It is to be understood that the present invention is not limited to the particular embodiments of the invention described above, as variations may be made thereto and still fall within the scope of the appended claims.

## Claims

1. A cationic lipid of Formula (I): 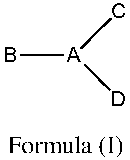 wherein,
A is a nitrilo;
B is C₁₋₆ alkyl, wherein the C₁₋₆ alkyl is optionally unsubstituted or substituted with 1-3 R^{d}; C is -L³-Y²-L⁴-CH=CR⁴R⁵, wherein
L³ and L⁴ are each independently selected from a chemical bond and a linear or branched C₁₋₁₂ alkylene, wherein each occurrence of the C₁₋₁₂ alkylene is optionally unsubstituted or substituted with 1 to 3 R^{e},
Y² is selected from the group consisting of -C(=X)-X-, -X-C(=X)-, -C(=X)-NH-, -NH-C(=X)-, -X-C(=X)-NH-, -NH-C(=X)-X-, -X-C(=X)-X-, -NH-C(=X)-NH-, and -X-X-, and
R⁴ and R⁵ are each independently selected from a linear or branched C₂₋₁₂ alkyl, wherein the C₂₋₁₂ alkyl is optionally unsubstituted or substituted with 1 to 3 R^{f};
D is -L⁵-Y³-L⁶-R⁶, wherein
L⁵ and L⁶ are each independently selected from a chemical bond and a linear or branched C₁₋₁₂ alkylene, wherein each occurrence of the C₁₋₁₂ alkylene is optionally unsubstituted or substituted with 1 to 3 R^{g},
Y³ is selected from the group consisting of -C(=X)-X-, -X-C(=X)-, -C(=X)-NH-, -NH-C(=X)-, -X-C(=X)-NH-, -NH-C(=X)-X-, -X-C(=X)-X-, -NH-C(=X)-NH-, and -X-X-, and
R⁶ is independently selected from a saturated or unsaturated, linear or branched C₆₋₁₈ hydrocarbon group or a steroid group, wherein the C₆₋₁₈ hydrocarbon group or the steroid group is optionally unsubstituted or substituted with 1-3 R^{h};
or, D is -L³-Y²-L⁴-CH=CR⁴R⁵;
each occurrence of X is independently O or S, and
each occurrence of R^{d}, R^{e}, R^{f}, R^{g} and R^{h} are each independently selected from the group consisting of C₁₋₃ alkyl, C₁₋₃ alkoxy, C₁₋₃ alkylamino, di(C₁₋₃ alkyl)amino, C₁₋₃ alkoxycarbonyl, C₁₋₃ alkylcarbonyloxy, C₁₋₃ alkylcarbonylamino, C₁₋₃ alkylaminocarbonyl, hydroxy, halogen, and cyano.

2. The cationic lipid of claim 1, wherein X is O.

3. The cationic lipid of claim 2, wherein
B is C₂₋₄ alkyl, which is unsubstituted or substituted with 1 to 3 substituents selected from the group consisting of hydroxy, halogen and cyano.

4. The cationic lipid of claim 3, wherein B is selected from the group consisting of methyl, ethyl, n-propyl, isopropyl, n-butyl, isobutyl and tert-butyl substituted with primary hydroxyl.

5. The cationic lipid of claim 1, wherein
C is -L³-Y²-L⁴-CH=CR⁴R⁵, wherein
R⁴ and R⁵ are the same or different and are each independently selected from linear C₂₋₁₂ alkyl.

6. The cationic lipid of claim 1, wherein
D is -L⁵-Y³-L⁶-R⁶, and
R⁶ is a linear or branched C₆₋₁₈ hydrocarbon group containing one or two carbon-carbon double bonds.

7. The cationic lipid of claim 6, wherein
R⁶ is a linear or branched C₆₋₁₈ hydrocarbon group containing two carbon-carbon double bonds.

8. The cationic lipid of claim 1, wherein
D is -L⁵-Y³-L⁶-R⁶, and
R⁶ is C₆₋₁₈ alkyl substituted with C₁₋₆ alkoxycarbonyl.

9. The cationic lipid of claim 1, wherein
D is -L³-Y²-L⁴-CH=CR⁴R⁵, wherein
R⁴ and R⁵ are the same or different and are each independently selected from linear C₂₋₁₂ alkyl.

10. The cationic lipid of any one of claims 1 to 9, wherein L³, L⁴, L⁵ and L⁶ are each independently selected from a chemical bond and C₁₋₆ alkylene, and the C₁₋₆ alkylene is optionally unsubstituted or substituted with 1 to 3 substituents independently selected from the group consisting of C₁₋₃ alkyl, C₁₋₃ alkoxy, hydroxyl and halogen.

11. The cationic lipid of claim 10, wherein L³, L⁴, L⁵ and L⁶ are each independently selected from a chemical bond and unsubstituted C₁₋₆ alkylene.

12. The cationic lipid of any one of claims 1 to 10, wherein ^{y2} and Y³ are each independently selected from the group consisting of -O-C(=O)-, -C(=O)-O- and -O-C(=O)-.

13. The cationic lipid of claim 1, having a structure selected from the group consisting of:

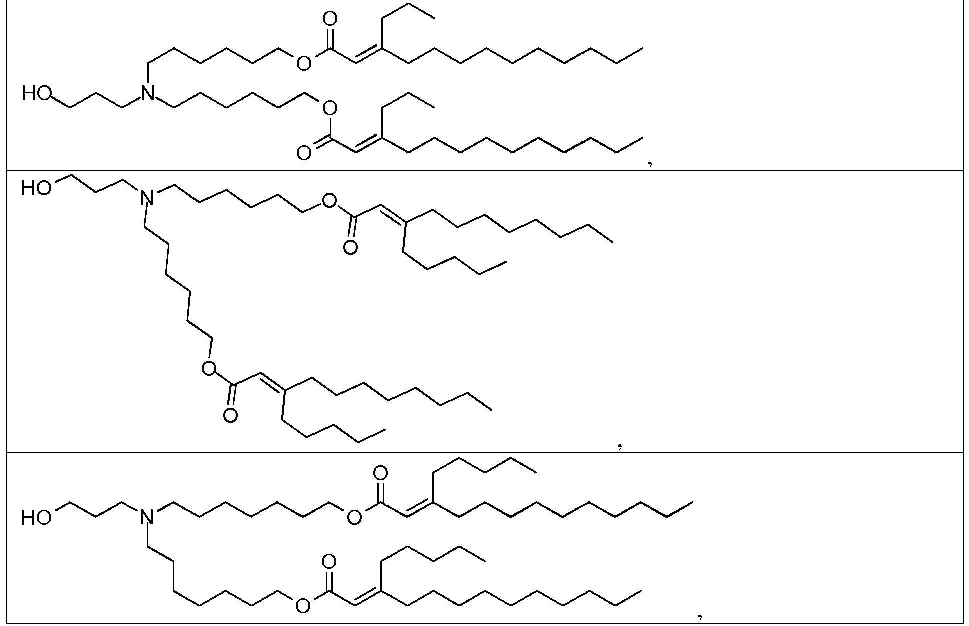

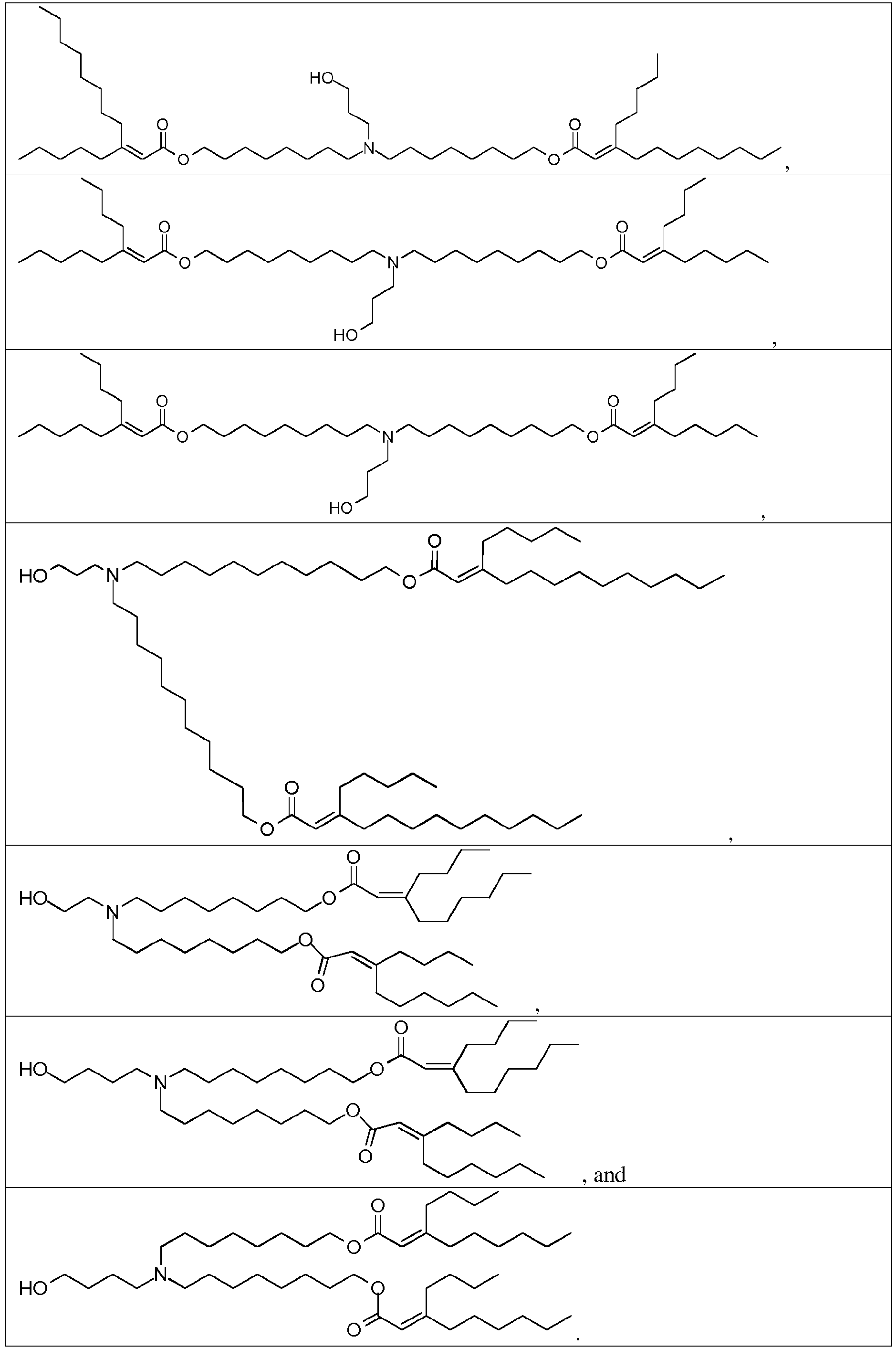

14. A liposome comprising the cationic lipid of any one of claims 1 to 13.

15. A lipid nanoparticle comprising the cationic lipid of any one of claims 1 to 13.

16. Use of the cationic lipid of any one of claims 1 to 13 for preparing liposomes.

17. Use of the cationic lipid of any one of claims 1 to 13 for preparing lipid nanoparticles.
